(19) Europäisches Patentamt
European Patent Office
Office européen des brevets

(11) EP 4 066 845 A1

(12) EUROPEAN PATENT APPLICATION

(43) Date of publication:
**05.10.2022 Bulletin 2022/40**

(21) Application number: **22162425.7**

(22) Date of filing: **13.05.2019**

(51) International Patent Classification (IPC):
***A61K 35/74*** $^{(2015.01)}$ ***A23L 33/135*** $^{(2016.01)}$
***A61P 1/00*** $^{(2006.01)}$ ***A61P 11/06*** $^{(2006.01)}$
***A61P 3/10*** $^{(2006.01)}$ ***A61P 17/06*** $^{(2006.01)}$
***A61P 19/02*** $^{(2006.01)}$ ***A61P 37/00*** $^{(2006.01)}$
***A61P 35/00*** $^{(2006.01)}$ ***A61P 43/00*** $^{(2006.01)}$

(52) Cooperative Patent Classification (CPC):
**A61K 35/74; A23L 33/135; A61P 1/00; A61P 3/10; A61P 11/06; A61P 17/06; A61P 19/02; A61P 35/00; A61P 37/00; A61P 43/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority:
**11.05.2018 EP 18171893**
**15.06.2018 EP 18178136**
**25.06.2018 GB 201810386**
**17.08.2018 GB 201813460**
**29.10.2018 GB 201817642**
**12.12.2018 GB 201820264**
**12.12.2018 GB 201820256**

(62) Document number(s) of the earlier application(s) in accordance with Art. 76 EPC:
**19725067.3 / 3 790 564**

(71) Applicant: **4D Pharma Research Limited**
**Aberdeen, Aberdeenshire AB25 2ZS (GB)**

(72) Inventors:
- **MULDER, Imke Elisabeth**
  **Aberdeenshire, AB25 2ZS (GB)**
- **AHMED, Suaad**
  **Aberdeenshire, AB25 2ZS (GB)**
- **ETTORRE, Anna**
  **Aberdeenshire, AB25 2ZS (GB)**
- **REID, Sarah Anne**
  **Aberdeenshire, AB25 2ZS (GB)**
- **DINAN, Ted**
  **Cork (IE)**
- **CRYAN, John**
  **Cork (IE)**

(74) Representative: **Carpmaels & Ransford LLP**
**One Southampton Row**
**London WC1B 5HA (GB)**

Remarks:
This application was filed on 16-03-2022 as a divisional application to the application mentioned under INID code 62.

# (54) COMPOSITIONS COMPRISING BACTERIAL STRAINS

(57) The invention provides a composition comprising a bacterial strain of the genus *Megasphaera*, for use in the treatment or prevention of an autoimmune or inflammatory disorder or cancer

EP 4 066 845 A1

SEQUENCE LISTING

<110> 4D PHARMA RESEARCH LIMITED

<120> COMPOSITIONS COMPRISING BACTERIAL STRAINS

<130> P073037WO

<141> 2019-05-13

<150> 18171893.3
<151> 2018-05-11

<150> 18178136
<151> 2018-06-15

<150> 1810386.1
<151> 2018-06-25

<150> 1813460.1
<151> 2018-08-17

<150> 1817642.0
<151> 2018-10-29

<150> 1820264.8
<151> 2018-12-12

<150> 1820256.4
<151> 2018-12-12

<160> 22

<170> SeqWin2010, version 1.0

<210> 1
<211> 1398
<212> DNA
<213> consensus 16S rRNA sequence for Megasphaera massiliensis strain MRx0029

<400> 1
```
tgagaagctt gcttcttatc gattctagtg gcaaacgggt gagtaacgcg taagcaacct  60
gcccttcaga tggggacaac agctggaaac ggctgctaat accgaatacg ttctttccgc  120
cgcatgacgg gaagaagaaa gggaggcctt cggctttcg ctggaggagg ggcttgcgtc  180
tgattagcta gttggagggg taacggccca ccaaggcgac gatcagtagc cggtctgaga  240
ggatgaacgg ccacattggg actgagacac ggcccagact cctacgggag gcagcagtgg  300
ggaatcttcc gcaatggacg aaagtctgac ggagcaacgc cgcgtgaacg atgacggcct  360
tcgggttgta aagttctgtt atatgggacg aacaggacat cggttaatac ccggtgtctt  420
tgacggtacc gtaagagaaa gccacggcta actacgtgcc agcagccgcg gtaatacgta  480
ggtggcaagc gttgtccgga attattgggc gtaaagggcg cgcaggcggc atcgcaagtc  540
ggtcttaaaa gtgcggggct taaccccgtg aggggaccga aactgtgaag ctcgagtgtc  600
ggagaggaaa gcggaattcc tagtgtagcg gtgaaatgcg tagatattag gaggaacacc  660
agtggcgaaa gcggctttct ggacgacaac tgacgctgag gcgcgaaagc caggggagca  720
aacgggatta gataccccgg tagtcctggc cgtaaacgat ggatactagg tgtaggaggt  780
atcgactcct tctgtgccgg agttaacgca ataagtatcc cgcctgggga gtacggccgc  840
aaggctgaaa ctcaaaggaa ttgacggggg cccgcacaag cggtggagta tgtggtttaa  900
ttcgacgcaa cgcgaagaac cttaccaagc cttgacattg attgctacgg aaagagattt  960
ccggttcttc ttcggaagac aagaaaacag gtggtgcacg gctgtcgtca gctcgtgtcg  1020
tgagatgttg ggttaagtcc cgcaacgagc gcaacccta tcttctgttg ccagcacctc  1080
gggtggggac tcagaagaga ctgccgcaga caatgcggag gaaggcgggg atgacgtcaa  1140
gtcatcatgc cccttatggc ttgggctaca cacgtactac aatggctctt aatagaggga  1200
agcgaaggag cgatccggag caaaccccaa aaacagagtc ccagttcgga ttgcaggctg  1260
```

caactcgcct gcatgaagca ggaatcgcta gtaatcgcag gtcagcatac tgcggtgaat 1320
acgttcccgg gccttgtaca caccgcccgt cacaccacga aagtcattca cacccgaagc 1380
cggtgaggca accgcaag 1398

<210> 2
<211> 21
<212> DNA
<213> GPR109a qPCR forward primer

<400> 2
atgttggcta tgaaccgcca g 21

<210> 3
<211> 19
<212> DNA
<213> GPR109a qPCR reverse primer

<400> 3
gctgctgtcc gattggaga 19

<210> 4
<211> 20
<212> DNA
<213> TPH1 forward primer

<400> 4
aaagagcgta caggtttttc 20

<210> 5
<211> 20
<212> DNA
<213> TPH1 reverse primer

<400> 5
gtctcacata ttgagtgcag 20

<210> 6
<211> 20
<212> DNA
<213> TPH2 forward primer

<400> 6
cactattgtg acgctgaatc 20

<210> 7
<211> 20
<212> DNA
<213> TPH2 reverse primer

<400> 7
agctcagaac catacatgag 20

<210> 8
<211> 20
<212> DNA
<213> Beta-actin forward primer

<400> 8
gatcaagatc attgctcctc 20

<210> 9
<211> 20
<212> DNA

<213> Beta-actin reverse primer

<400> 9
ttgtcaagaa agggtgtaac 20

<210> 10
<211> 19
<212> DNA
<213> SLC6A4 forward primer

<400> 10
aatctgccga ttttcaaag 19

<210> 11
<211> 21
<212> DNA
<213> SLC6A4 reverse primer

<400> 11
gtgttgtagt aggaagcaat g 21

<210> 12
<211> 20
<212> DNA
<213> Beta-actin forward primer

<400> 12
gatcaagatc attgctcctc 20

<210> 13
<211> 20
<212> DNA
<213> Beta-actin reverse primer

<400> 13
ttgtcaagaa agggtgtaac 20

<210> 14
<211> 1409
<212> DNA
<213> consensus 16S rRNA sequence for the Megasphaera strain deposited under accession number NCIMB 43385

<400> 14
ggctggttcc ttgcggttgc ctcaccggct tcgggtgtga atgactttcg tggtgtgacg 60
ggcggtgtgt acaaggcccg ggaacgtatt caccgcagta tgctgacctg cgattactag 120
cgattcctgc ttcatgcagg cgagttgcag cctgcaatcc gaactgggac tctgtttttg 180
gggtttgctc cggatcgctc cttcgcttcc ctctattaag agccattgta gtacgtgtgt 240
agcccaagcc ataaggggca tgatgacttg acgtcatccc cgccttcctc cgcattgtct 300
gcggcagtct cttctgagtc cccaccctta gtgctggcaa cagaagatag ggttgcgct 360
cgttgcggga cttaacccaa catctcacga cacgagctga cgacagccgt gcaccacctg 420
ttttcttgtc ttccgaagaa gaaccggaaa tctctttccg tagcaatcaa tgtcaaggct 480
tggtaaggtt cttcgcgttg cgtcgaatta aaccacatac tccaccgctt gtgcgggccc 540
ccgtcaattc ctttgagttt cagccttgcg gccgtactcc ccaggcggga tacttattgc 600
gttaactccg gcacagaagg agtcgatacc tcctacacct agtatccatc gtttacggcc 660
aggactaccg gggtatctaa tcccgtttgc tcccctggct ttcgcgcctc agcgtcagtt 720
gtcgtccaga aagccgcttt cgccactggt gttcctccta atatctacgc atttcaccgc 780
tacactagga attccgcttt cctctccgac actcgagctt cacagtttcg gtcccctcac 840
ggggttaagc cccgcacttt taagaccgac ttgcgatgcc gcctgcgcgc cctttacgcc 900
caataattcc ggacaacgct tgccacctac gtattaccgc ggctgctggc acgtagttag 960
ccgtggcttt ctcttacggt accgtcaggg ataacgggta ttgaccgcta tcctgttcgt 1020
cccatataac agaactttac aacccgaagg ccgtcatcgt tcacgcggcg ttgctccgtc 1080
agactttcgt ccattgcgga agattcccca ctgctgcctc ccgtaggagt ctgggccgtg 1140

tctcagtccc aatgtggccg ttcatcctct cagaccggct actgatcgtc gccttggtgg 1200
gccgttaccc ctccaactag ctaatcagac gcaagcccct cctccagcga aagcccgaag 1260
gcctcccttt cttcatcccg tcatgcggcg gaaagaacgt attcggtatt agcagccgtt 1320
tccagctgtt gtccccatct gaagggcagg ttgcttacgc gttactcacc cgtttgccac 1380
tcgaattgat aagaagcaag cttctcatc 1409

<210> 15
<211> 1415
<212> DNA
<213> consensus 16S rRNA sequence for the Megasphaera massiliensis strain deposited under accession number NCIMB 43388

<400> 15
ggctggttcc ttgcggttgc ctcaccggct tcgggtgtga atgactttcg tggtgtgacg 60
ggcggtgtgt acaaggcccg ggaacgtatt caccgcagta tgctgacctg cgattactag 120
cgattcctgc ttcatgcagg cgagttgcag cctgcaatcc gaactgggac tctgtttttg 180
gggtttgctc cggatcgctc cttcgcttcc ctctattaag agccattgta gtacgtgtgt 240
agcccaagcc ataaggggca tgatgacttg acgtcatccc cgccttcctc cgcattgtct 300
gcggcagtct cttctgagtc ccccacccgag gtgctggcaa cagaagatag gggttgcgct 360
cgttgcggga cttaacccaa catctcacga cacgagctga cgacagccgt gcaccacctg 420
ttttcttgtc ttccgaagaa gaaccggaaa tctctttccg tagcaatcaa tgtcaaggct 480
tggtaaggtt cttcgcgttg cgtcgaatta aaccacatac tccaccgctt gtgcgggccc 540
ccgtcaattc ctttgagttt cagccttgcg gccgtactcc ccaggcggga tacttattgc 600
gttaactccg gcacagaagg agtcgatacc tcctacacct agtatccatc gtttacggcc 660
aggactaccg gggtatctaa tcccgtttgc tcccctggct ttcgcgcctc agcgtcagtt 720
gtcgtccaga aagccgcttt cgccactggt gttcctccta atatctacgc atttcaccgc 780
tacactagga attccgcttt cctctccgac actcgagctt cacagtttcg gtcccctcac 840
ggggttaagc cccgcacttt taagaccgac ttgcgatgcc gcctgcgcgc cctttacgcc 900
caataattcc ggacaacgct tgccacctac gtattaccgc ggctgctggc acgtagttag 960
ccgtggcttt ctcttacggt accgtcaaag acaccgggta ttaaccgatg tcctgttcgt 1020
cccatataac agaactttac aacccgaagg ccgtcatcgt tcacgcggcg ttgctccgtc 1080
agactttcgt ccattgcgga agattcccca ctgctgcctc ccgtaggagt ctgggccgtg 1140
tctcagtccc aatgtggccg ttcatcctct cagaccggct actgatcgtc gccttggtgg 1200
gccgttaccc ctccaactag ctaatcagac gcaagcccct cctccagcga aagcccgaag 1260
gcctcccttt cttcttcccg tcatgcggcg gaaagaacgt attcggtatt agcagccgtt 1320
tccagctgtt gtccccatct gaagggcagg ttgcttacgc gttactcacc cgtttgccac 1380
tagaatcgat aagaagcaag cttctcatgt cttct 1415

<210> 16
<211> 1433
<212> DNA
<213> consensus 16S rRNA sequence for the Megasphaera massilliensis strain deposited under accession number NCIMB 43389

<400> 16
cgacggctgg ttccttgcgg ttgcctcacc ggcttcgggt gtgaatgact ttcgtggtgt 60
gacgggcggt gtgtacaagg cccgggaacg tattcaccgc agtatgctga cctgcgatta 120
ctagcgattc ctgcttcatg caggcgagtt gcagcctgca atccgaactg ggactctgtt 180
tttggggttt gctccggatc gctccttcgc ttccctctat taagagccat tgtagtacgt 240
gtgtagccca agccataagg ggcatgatga cttgacgtca tccccgcctt cctccgcatt 300
gtctgcggca gtctcttctg agtccccacc cgaggtgctg gcaacagaag ataggggttg 360
cgctcgttgc gggacttaac ccaacatctc acgacacgag ctgacgacag ccgtgcacca 420
cctgttttct tgtcttccga agaagaaccg gaaatctctt tccgtagcaa tcaatgtcaa 480
ggcttggtaa ggttcttcgc gttgcgtcga attaaaccac atactccacc gcttgtgcgg 540
gcccccgtca attcctttga gtttcagcct tgcggccgta ctccccaggc gggatactta 600
ttgcgttaac tccggcacag aaggagtcga tacctcctac acctagtatc catcgtttac 660
ggccaggact accggggtat ctaatcccgt ttgctcccct ggctttcgcg cctcagcgtc 720
agttgtcgtc cagaaagccg ctttcgccac tggtgttcct cctaatatct acgcatttca 780
ccgctacact aggaattccg ctttcctctc cgacactcga gcttcacagt ttcggtcccc 840
tcacggggtt aagccccgca cttttaagac cgacttgcga tgccgcctgc gcgcccttta 900
cgcccaataa ttccggacaa cgcttgccac ctacgtatta ccgcggctgc tggcacgtag 960
ttagccgtgg ctttctctta cggtaccgtc aaagacaccg ggtattaacc gatgccctgt 1020
tcgtcccata taacagaact ttacaacccg aaggccgtca tcgttcacgc ggcgttgctc 1080

cgtcagactt tcgtccattg cggaagattc cccactgctg cctcccgtag gagtctgggc 1140
cgtgtctcag tcccaatgtg gccgttcatc ctctcagacc ggctactgat cgtcgccttg 1200
gtgggccgtt accccctccaa ccagctaatc agacgcaagc ccctcctcca gcgaaagccc 1260
gaaggcctcc ctttcttctt cccgtcatgc ggcggaaaga acgtattcgg tattagcagc 1320
cgtttccagc tgttgtcccc atctgaaggg caggttgctt acgcgttact cacccgtttg 1380
ccactagaat cgataagaag caagcttctc atgtcttctc gttcgacttg cat 1433

<210> 17
<211> 1431
<212> DNA
<213> consensus 16S rRNA sequence for the Megasphaera strain deposited under accession number NCIMB 43386

<400> 17
cgacggctgg ttccttgcgg ttgcctcacc ggcttcgggt gtgaatgact ttcgtggtgt 60
gacgggcggt gtgtacaagg cccgggaacg tattcaccgc agtatgctga cctgcgatta 120
ctagcgattc ctgcttcatg caggcgagtt gcagcctgca atccgaactg ggactctgtt 180
tttggggttt gctccggatc gctccttcgc ttccctctat taagagccat tgtagtacgt 240
gtgtagccca agccataagg ggcatgatga cttgacgtca tccccgcctt cctccgcatt 300
gtctgcggca gtctcttctg agtccccacc cttagtgctg gcaacagaag ataggggttg 360
cgctcgttgc gggacttaac ccaacatctc acgacacgag ctgacgacag ccgtgcacca 420
cctgttttct tgtcttccga agaagaaccg gaaatctctt tccgtagcaa tcaatgtcaa 480
ggcttggtaa ggttcttcgc gttgcgtcga attaaaccac atactccacc gcttgtgcgg 540
gccccccgtca attcctttga gtttcagcct tgcggccgta ctccccaggc gggatactta 600
ttgcgttaac tccggcacag aaggagtcga tacctcctac acctagtatc catcgtttac 660
ggccaggact accgggggtat ctaatcccgt ttgctcccct ggctttcgcg cctcagcgtc 720
agttgtcgtc cagaaagccg ctttcgccac tggtgttcct cctaatatct acgcatttca 780
ccgctacact aggaattccg ctttcctctc cgacactcga gcttcacagt ttcggtcccc 840
tcacggggtt aagccccgca cttttaagac cgacttgcga tgccgcctgc gcgcccttta 900
cgcccaataa ttccggacaa cgcttgccac ctacgtatta ccgcggctgc tggcacgtag 960
ttagccgtgg ctttctctta cggtaccgtc agggataacg ggtattgacc gctatcctgt 1020
tcgtcccata taacagaact ttacaacccg aaggccgtca tcgttcacgc ggcgttgctc 1080
cgtcagactt tcgtccattg cggaagattc cccactgctg cctcccgtag gagtctgggc 1140
cgtgtctcag tcccaatgtg gccgttcatc ctctcagacc ggctactgat cgtcgccttg 1200
gtgggccgtt acccctccaa ctagctaatc agacgcaagc ccctcctcca gcgaaagccc 1260
gaaggcctcc ctttcttcat cccgtcatgc ggcggaaaga acgtattcgg tattagcagc 1320
cgtttccagc tgttgtcccc atctgaaggg caggttgctt acgcgttact cacccgtttg 1380
ccactcgaat tgataagaag caagcttctc atctcttctc gttcgactgc a 1431

<210> 18
<211> 1434
<212> DNA
<213> consensus 16S rRNA sequence for the Megasphaera strain deposited under accession number NCIMB 43387

<400> 18
tcgaacggct ggttccttgc ggttgcctca ccggcttcgg gtgtgaatga ctttcgtggt 60
gtgacgggcg gtgtgtacaa ggcccgggaa cgtattcacc gcagtatgct gacctgcgat 120
tactagcgat tcctgcttca tgcaggcgag ttgcagcctg caatccgaac tgggactctg 180
tttttggggt ttgctccgga tcgctccttc gcttccctct attaagagcc attgtagtac 240
gtgtgtagcc caagccataa ggggcatgat gacttgacgt catccccgcc ttcctccgca 300
ttgtctgcgg cagtctcttc tgagtcccca cccttagtgc tggcaacaga agataggggt 360
tgcgctcgtt gcgggactta acccaacatc tcacgacacg agctgacgac agccgtgcac 420
cacctgtttt cttgtcttcc gaagaagaac cggaaatctc tttccgtagc aatcaatgtc 480
aaggcttggt aaggttcttc gcgttgcgtc gaattaaacc acatactcca ccgcttgtgc 540
gggcccccgt caattccttt gagtttcagc cttgcggccg tactccccag gcgggatact 600
tattgcgtta actccggcac agaaggagtc gatacctcct acacctagta tccatcgttt 660
acggccagga ctaccggggt atctaatccc gtttgctccc ctggctttcg cgcctcagcg 720
tcagttgtcg tccagaaagc cgctttcgcc actggtgttc ctcctaatat ctacgcattt 780
caccgctaca ctaggaattc cgctttcctc tccgacactc gagcttcaca gtttcggtcc 840
cctcacgggg ttaagccccg cacttttaag accgacttgc gatgccgcct gcgcgccctt 900
tacgcccaat aattccggac aacgcttgcc acctacgtat taccgcggct gctggcacgt 960
agttagccgt ggctttctct tacggtaccg tcagggataa cgggtattga ccgctatcct 1020

```
gttcgtccca tataacagaa ctttacaacc cgaaggccgt catcgttcac gcggcgttgc  1080
tccgtcagac tttcgtccat tgcggaagat tccccactgc tgcctcccgt aggagtctgg  1140
gccgtgtctc agtcccaatg tggccgttca tcctctcaga ccggctactg atcgtcgcct  1200
tggtgggccg ttacccctcc aactagctaa tcagacgcaa gcccctcctc cagcgaaagc  1260
ccgaaggcct ccctttcttc atcccgtcat gcggcggaaa gaacgtattc ggtattagca  1320
gccgtttcca gctgttgtcc ccatctgaag ggcaggttgc ttacgcgtta ctcacccgtt  1380
tgccactcga attgataaga agcaagcttc tcatctcttc tcgttcgact tgca        1434

<210>   19
<211>   20
<212>   DNA
<213>   NSE forward primer

<400>   19
ccctgtatcg taagaacggt                                              20

<210>   20
<211>   20
<212>   DNA
<213>   NSE reverse primer

<400>   20
gccaccattg atcacgttga                                              20

<210>   21
<211>   21
<212>   DNA
<213>   GAPDH forward primer

<400>   21
ggtatcgtgg aaggactcat g                                            21

<210>   22
<211>   21
<212>   DNA
<213>   GAPDH reverse primer

<400>   22
atgccagtga gcttcccgtt c                                            21
```

## Description

### TECHNICAL FIELD

This invention is in the field of compositions comprising bacterial strains isolated from the mammalian digestive tract and the use of such compositions in the treatment of disease.

### BACKGROUND TO THE INVENTION

The human intestine is thought to be sterile *in utero,* but it is exposed to a large variety of maternal and environmental microbes immediately after birth. Thereafter, a dynamic period of microbial colonization and succession occurs, which is influenced by factors such as delivery mode, environment, diet and host genotype, all of which impact upon the composition of the gut microbiota, particularly during early life. Subsequently, the microbiota stabilizes and becomes adult-like [1]. The human gut microbiota contains more than 500-1000 different phylotypes belonging essentially to two major bacterial divisions, the Bacteroidetes and the Firmicutes [2]. The successful symbiotic relationships arising from bacterial colonization of the human gut have yielded a wide variety of metabolic, structural, protective and other beneficial functions. The enhanced metabolic activities of the colonized gut ensure that otherwise indigestible dietary components are degraded with release of by-products providing an important nutrient source for the host. Similarly, the immunological importance of the gut microbiota is well-recognized and is exemplified in germfree animals which have an impaired immune system that is functionally reconstituted following the introduction of commensal bacteria [3-5].

Dramatic changes in microbiota composition have been documented in gastrointestinal disorders such as inflammatory bowel disease (IBD). For example, the levels of *Clostridium* cluster XIVa bacteria are reduced in IBD patients whilst numbers of *E. coli* are increased, suggesting a shift in the balance of symbionts and pathobionts within the gut [6-9]. Interestingly, this microbial dysbiosis is also associated with imbalances in T effector cell populations.

In recognition of the potential positive effect that certain bacterial strains may have on the animal gut, various strains have been proposed for use in the treatment of various diseases (see, for example, [10-13]). Also, certain strains, including mostly *Lactobacillus* and *Bifidobacterium* strains, have been proposed for use in treating various inflammatory and autoimmune diseases that are not directly linked to the intestines (see [14] and [15] for reviews). However, the relationship between different diseases and different bacterial strains, and the precise effects of particular bacterial strains on the gut and at a systemic level and on any particular types of diseases, are poorly characterised.

There is a requirement in the art for new methods of treating diseases. There is also a requirement for the potential effects of gut bacteria to be characterised so that new therapies using gut bacteria can be developed.

Ahmed et al (submitted to Frontiers Cellular Neuroscience) considers *in vitro* characterisation of gut microbiota-derived bacterial strains.

### SUMMARY OF THE INVENTION

The inventors have developed new compositions comprising a bacterial strain of the genus *Megasphaera* that can be used in treating and preventing autoimmune and inflammatory diseases and cancer, in particular autoimmune and inflammatory diseases and cancer that are mediated by histone deacetylase (HDAC) activity. The inventors have identified that bacterial strains from the genus *Megasphaera* can be effective for reducing histone deacetylase activity. Histone deacetylase activity has been shown to mediate pathological symptoms in an array of autoimmune or inflammatory diseases and conditions including, but not limited to, Graft-versus-host disease (GVHD) and inflammatory bowel diseases, such as ulcerative colitis and Crohn's disease. As described in the examples, administration of compositions comprising *Megasphaera massiliensis* reduce the activity of histone deacetylase in models of disease. The inventors have also identified that treatment with *Megasphaera massiliensis* can reduce the activation of proinflammatory molecules, such as NFκB and IL-6, by LPS. The inventors have identified that treatment with *Megasphaera massiliensis* can reduce lipid peroxidation *in vitro,* which can help to reduce cell death and apoptosis. The inventors have also identified that *Megasphaera massiliensis* can produce indole that can attenuate inflammation and oxidative stress.

HDAC activity is also associated with pathological mechanisms in a range of cancers. Inhibition of HDAC activity may therefore be therapeutically beneficial in the treatment cancer. As such, the compositions of the invention may have pleiotropic benefits in the treatment or prevention of cancers, in particular cancers mediated at least in part by HDAC activity. In some embodiments, the compositions of the invention are for use in the treatment or prevention of cancer, such as prostate cancer, colorectal cancer, breast cancer, lung cancer, liver cancer or gastric cancer, in particular wherein the cancer is mediated by increased HDAC activity.

The inventors have identified that treatment with bacterial strains from the genus *Megasphaera* can reduce the activity of HDAC, which can provide clinical benefits in the treatment of diseases mediated by HDAC activity. In some embodiments, the compositions of the invention have been found to be particularly beneficial in reducing Class I HDAC activity. In certain embodiments, the compositions of the invention may reduce HDAC1, HDAC2 or HDAC3 activity. Class I HDACs are ubiquitously expressed and most commonly reside in the nucleus. Class I HDACs deacetylate histone lysine residues to restore positive charge to the histone, thereby increasing electrostatic binding between histones and DNA. HDAC activity therefore increases chromatin compaction, causing downregulation of the expression of genes at the underlying DNA sequence. In certain embodiments, the compositions of the invention can therefore be used to regulate target gene expression. HDACs also have additional regulatory effects by modifying non-histone protein targets. The inhibition of the acetylation of non-histone protein targets may be beneficial in the treatment or prevention of other aspects of disease not directly related to the control of gene expression by chromatin morphology.

The inventors have also shown that a composition comprising a bacterial strain of the genus *Megasphaera* upregulates the expression of Serotonin Transporter (SERT) gene SLC6A4. SERT is expressed by epithelial cells lining the intestines and removes serotonin from the interstitial space. It has been reported in some literature references [16] that serotonin may have a pro-inflammatory effect in certain scenarios, for example in the gastrointestinal tract. Therefore, the compositions of the invention may be useful in treating inflammatory disorders, and in particular inflammatory bowel diseases, such as Crohn's disease or ulcerative colitis. In certain embodiments, the compositions of the invention are for use in treating inflammatory bowel diseases by increasing serotonin transport in the gastrointestinal tract, for example by upregulating expression of Serotonin Transporter (SERT) gene SLC6A4.

In particular embodiments, the invention provides a composition comprising a bacterial strain of the genus *Megasphaera,* for use in a method of treating or preventing a disease or condition selected from the group consisting of: an inflammatory or autoimmune disease, such as asthma, arthritis, psoriasis, diabetes, allograft rejection, graft-versus-host disease, or an inflammatory bowel disease, such as Crohn's disease or ulcerative colitis; or cancer, such as prostate cancer, colorectal cancer, breast cancer, lung cancer, liver cancer or gastric cancer. The effect shown for the bacterial strains from the genus *Megasphaera* on HDAC activity may provide therapeutic benefits for diseases and conditions mediated by HDAC activity, such as those listed above. In certain embodiments, the compositions of the invention may provide therapeutic benefits in the treatment of diseases or conditions with increased HDAC expression. In certain embodiments, the compositions of the invention may provide therapeutic benefits in the treatment of diseases or conditions with increased HDAC activity.

In some embodiments, the invention provides a composition comprising a bacterial strain of the species *Megasphaera massiliensis,* for use in a method of treating or preventing inflammatory or autoimmune diseases mediated by HDAC activity. In some embodiments, the compositions of the invention may be useful in the treatment or prevention of symptoms of inflammatory or autoimmune diseases mediated by HDAC activity. The inventors have identified that the strains of the invention inhibit HDAC activity. Histone acetylation and deacetylation are important epigenetic regulators of gene expression. Histone acetylation imbalance has been implicated in the pathogenesis of inflammatory or autoimmune diseases such as such as asthma, arthritis, psoriasis, diabetes, allograft rejection, graft-versus-host disease, or an inflammatory bowel disease, such as Crohn's disease or ulcerative colitis.

In some embodiments the invention provides a composition comprising a bacterial strain of the genus *Megasphaera* for use in a method of treating or preventing an inflammatory bowel disease mediated by HDAC activity. Inhibition of HDAC activity has been shown to suppress the production of proinflammatory cytokines in the gastrointestinal tract. Thus, the compositions of the invention may be useful in the treatment of inflammatory diseases. In particular, the compositions of the invention may be useful in the treatment or prevention of conditions associated with increased colonic proinflammatory cytokine pathogenesis. In some embodiments, the compositions of the invention are for use in the treatment or prevention of inflammatory bowel disease. In some embodiments, the compositions of the invention are for use in the treatment or prevention of ulcerative colitis. In some embodiments, the compositions of the invention are for use in the treatment or prevention of Crohn's disease. In certain embodiments, the invention provides a composition comprising a bacterial strain of the genus *Megasphaera* for use in the treatment or prevention of inflammatory disease. In preferred embodiments, the invention provides a composition comprising a bacterial strain of the genus *Megasphaera,* preferably the species *Megasphaera massiliensis,* for use in the treatment or prevention of colitis.

In some embodiments, the compositions of the invention are for use in the treatment or prevention of cancer. Dysregulation of acetylation pathways in cancer have been implicated in cancer cell survival and tumour immune evasion. For example, HDAC mediated deacetylation of p53 reduces the stability and half-life of p53. Acetylated p53 binds and regulates the expression of cell cycle regulatory and pro-apoptotic genes with greater efficacy, reducing cancer cell growth and promoting apoptosis. Deacetylation of p53 may therefore inhibit apoptosis in cancer cells, increasing cancer cell survival. In certain embodiments, the compositions of the invention are for use in the treatment or prevention of cancers. In some embodiments, the compositions of the invention are for use in the treatment of cancers with non-mutated p53. In some embodiments, the compositions of the invention are for use in a method of increasing apoptosis in cancer cells. In some embodiments, the compositions of the invention are for use in a method of decreasing tumour immune evasion. In some embodiments, the compositions of the invention are for use in the treatment or prevention of cancers with increased HDAC-activity. In some embodiments, the compositions are for use as pro-apoptotic medicaments, for example for use in the treatment or prevention of cancers. In certain embodiments, the invention provides a composition comprising a bacterial strain of the genus *Megasphaera,* preferably the species *Megasphaera massiliensis,* for use in the treatment or prevention of cancer.

In further preferred embodiments, the invention provides a composition comprising a bacterial strain of the genus *Megasphaera,* for use in a method of treating or preventing cancer, such as breast, lung or liver cancer. In certain embodiments, the composition is for use in a method of reducing tumour size or preventing tumour growth in the treatment of cancer. In certain embodiments, the invention provides a composition comprising a bacterial strain of the genus *Megasphaera,* for use in the treatment of cancer.

In certain embodiments, the compositions of the invention are for use in a method of reducing histone deacetylase activity in the treatment or prevention of a disease or condition mediated by histone deacetylase activity.

In certain embodiments, the composition is for use in a patient with elevated histone deacetylase activity. In certain embodiments, the composition is for use in a patient with elevated Class I HDAC activity. The effect on histone deacetylase activity shown for *Megasphaera massiliensis* strains may be particularly beneficial for such patients.

In certain embodiments of the invention, the bacterial strain in the composition is of *Megasphaera massiliensis.* In certain embodiments of the invention, the bacterial strain in the composition is of *Megasphaera massiliensis.* Closely related strains may also be used, such as bacterial strains that have a 16s rRNA gene sequence that is at least 95%, 96%, 97%, 98%, 99%, 99.5% or 99.9% identical to SEQ ID NO: 1. Preferably, the bacterial strain for use in the invention has the 16s rRNA gene sequence represented by SEQ ID NO: 1.

In certain embodiments, the composition of the invention is for oral administration. Oral administration of the strains of the invention can be effective for treating diseases and conditions mediated by HDAC activity. In certain embodiments, oral administration of the strains of the invention can be effective for treating diseases and conditions mediated by Class I HDAC activity Also, oral administration is convenient for patients and practitioners and allows delivery to and / or partial or total colonisation of the intestine.

In certain embodiments, the composition of the invention comprises one or more pharmaceutically acceptable excipients or carriers.

In certain embodiments, the composition of the invention comprises a bacterial strain that has been lyophilised. Lyophilisation is an effective and convenient technique for preparing stable compositions that allow delivery of bacteria.

In certain embodiments, the invention provides a food product comprising the composition as described above.

Additionally, the invention provides a method of treating or preventing a disease or condition mediated by HDAC activity, comprising administering a composition comprising a bacterial strain of the genus *Megasphaera.*

In developing the above invention, the inventors have identified and characterised a bacterial strain that is particularly useful for therapy. The *Megasphaera massiliensis* strain of the invention is shown to be effective for treating the diseases described herein, such as inflammatory and autoimmune diseases such as GVHD and colitis. Therefore, in another aspect, the invention provides a cell of the *Megasphaera massiliensis* strain deposited under accession number NCIMB 42787, or a derivative thereof. The invention also provides compositions comprising such cells, or biologically pure cultures of such cells. The invention also provides a cell of the *Megasphaera massiliensis* strain deposited under accession number NCIMB 42787, or a derivative thereof, for use in therapy, in particular for the diseases described herein.

Further numbered embodiments of the invention are provided below:

1. A composition comprising a bacterial strain of the genus *Megasphaera,* for use in the treatment or prevention of a disease or condition selected from the list consisting of: an inflammatory or autoimmune disease, such as asthma, arthritis, psoriasis, diabetes, allograft rejection, graft-versus-host disease, or an inflammatory bowel disease, such as Crohn's disease or ulcerative colitis; or cancer, such as prostate cancer, colorectal cancer, breast cancer, lung cancer, liver cancer or gastric cancer.
2. The composition according to any preceding embodiment, for use in the treatment or prevention of a disease or condition mediated by histone deacetylase (HDAC) activity.
3. The composition of embodiment 1, for use in the treatment or prevention of a disease or condition mediated by Class I HDAC activity.
4. The composition according to any preceding embodiment, for use in a method of inhibiting Class I HDAC activity in a condition mediated by Class I HDAC activity.
5. The composition according to any preceding embodiment, for use in a method of selectively inhibiting Class I HDAC activity in the treatment of a condition mediated by Class I HDAC activity.
6. The composition according to any preceding embodiment, wherein the composition is for use in selectively inhibiting HDAC1, HDAC2 or HDAC3 in a disease or condition mediated by HDAC1, HDAC2 or HDAC3 activity.
7. The composition according to any preceding embodiment, wherein the composition is for use in the treatment or prevention of a disease or condition in which inhibiting HDAC activity is beneficial.
8. The composition according to any preceding embodiment, for use in a patient with elevated HDAC activity.
9. The composition according to any preceding embodiment, for use in the treatment or prevention of an inflammatory or autoimmune or disease.

10. The composition according to any preceding embodiment, for use in the treatment or prevention of inflammatory bowel disease.

11. The composition according to any preceding embodiment, for use in the treatment or prevention of ulcerative colitis.

12. The composition according to any preceding embodiment, for use in the treatment or prevention of Crohn's disease.

13. The composition according to any preceding embodiment, for use in the treatment or prevention of cancer.

14. The composition for use according to embodiment 13, wherein the cancer is selected from the list consisting of prostate cancer, colorectal cancer, breast cancer, lung cancer, liver cancer or gastric cancer.

15. The composition of any preceding embodiment, wherein the bacterial strain has a 16S rRNA sequence that is at least 95%, 96%, 97%, 98%, 99%, 99.5% or 99.9% identical to the 16S rRNA sequence of a bacterial strain of the genus *Megasphaera.*

16. The composition of any preceding embodiment, wherein the bacterial strain has a 16s rRNA gene sequence that is at least 95%, 96%, 97%, 98%, 99%, 99.5% or 99.9% identical to any one of SEQ ID NOs: 14, 15, 16, 17 or 18 or wherein the bacterial strain has a 16s rRNA gene sequence represented by any one of SEQ ID NOs:14, 15, 16, 17 or 18.

17. The composition of any preceding embodiment, wherein the bacterial strain is of *Megasphaera massiliensis.*

18. The composition of any preceding embodiment, wherein the bacterial strain has a 16s rRNA gene sequence that is at least 95%, 96%, 97%, 98%, 99%, 99.5% or 99.9% identical to SEQ ID NO:1.

19. The composition of any preceding embodiment, wherein the bacterial strain has a 16s rRNA gene sequence represented by SEQ ID NO:1.

20. The composition of any preceding embodiment, wherein the composition is for oral administration.

21. The composition of any preceding embodiment, wherein the composition comprises one or more pharmaceutically acceptable excipients or carriers.

22. The composition of any preceding embodiment, wherein the bacterial strain is lyophilised.

23. The composition according to any preceding embodiment, for use as an anti-inflammatory medicament.

24. The composition according to any preceding embodiment, for use as a histone deacetylase inhibiting medicament.

25. The composition according to any preceding embodiment, for use as a Class I histone deacetylase inhibiting medicament.

26. A food product comprising the composition of any preceding embodiment, for the use of any preceding embodiment.

27. A method of treating or preventing a disease or condition mediated by histone deacetylase activity, comprising administering a composition comprising a bacterial strain of the species *Megasphaera massiliensis* to a patient in need thereof.

28. A cell of the *Megasphaera massiliensis* strain deposited under accession number NCIMB 42787, or a derivative thereof.

29. A cell of the *Megasphaera massiliensis* strain deposited under accession number NCIMB 42787, or a derivative thereof, for use in therapy, preferably for use in the treatment or prevention of a disease or condition as defined in one of embodiments 1-14.

30. A bacterial strain for use in therapy, wherein the bacterial strain has a 16S rRNA sequence that is at least 95%, 96%, 97%, 98%, 99%, 99.5% or 99.9% identical to any one of SEQ ID NOs: 14, 15, 16, 17 or 18.

31. A bacterial strain having the 16S rRNA sequence represented by any one of SEQ ID NOs: 14, 15, 16, 17 or 18 for use in therapy.

## BRIEF DESCRIPTION OF DRAWINGS

**Figure 1** Whole-cell histone deacetylase activity and cell lysate histone deacetylase activity (Figure 1A), acid-induced changes in histone deacetylase activity, levels of metabolite production in MRx0029

**Figure 2** Inhibition of Class I HDACs (Figure 2A); inhibition of HDAC1 (Figure 2B); inhibition of HDAC2 (Figure 2C); inhibition of HDAC3 (Figure 2D)

**Figure 3:** Levels of IL-6 secretion

**Figure 4:** Inhibition of LPS induced NFκB promoter activation

**Figure 5:** Change in antioxidant capacity

**Figure 6:** Change in lipid oxidation

**Figure 7:** Level of Indole production

**Figure 8** shows the levels of neurotransmitter metabolites in the brain following administration of MRx0029.

**Figure 9** shows the fold-change in expression of tryptophan hydroxylase 1 and 2 in SH-SY5Y neuroblastoma cells. Cells were incubated with 10% MRx0029 supernatant for 24 h.

**Figure 10** shows the fold-change in expression of tryptophan hydroxylase 1 and 2 in SH-SY5Y neuroblastoma cells. Cells were incubated with 5% MRx0029 supernatant for 72 h.

**Figure 11** shows the fold-change in expression of SLC6A4 in SH-SY5Y neuroblastoma cells. Cells were incubated with 10% MRx0029 supernatant for 24 h.

**Figure 12** shows the fold-change in expression of SLC6A4 in SH-SY5Y neuroblastoma cells. Cells were incubated with 5% MRx0029 supernatant for 72 h.

**Figure 13** shows the fold-change in expression of tryptophan hydroxylase 1 (TPH1) in differentiated Caco2 cells. Cells were incubated with 10% MRx0029 bacterial cell-free supernatant for 24h.

**Figure 14** shows the fold-change in expression of SLC6A4 in differentiated Caco2 cells. Cells were incubated with 10% MRx0029 bacterial cell-free supernatant for 24h.

**Figure 15:** Metabolite analysis for *Megasphaera massiliensis* strain NCIMB 42787.

**Figure 16:** Valeric acid production in the supernatant for MRx0029 and reference *Megasphaera massiliensis* strains.

**Figure 17:** Organic acid production and consumption by MRx0029 and reference *Megasphaera massiliensis* strains.

**Figure 18:** GPR109a RNA expression in differentiated Caco-2 cells (A) without, and (B) with phorbolmyristate treatment in addition to MRx0029. "YCFA" = YCFA+

**Figure 19:** Suppression of NSE/Enolase 2 by MRx0029. "YCFA" = YCFA+

**Figure 20:** Organic acid production and consumption by NCIMB 42787, NCIMB 43385, NCIMB 43388 and NCIMB 43389.

**Figure 21:** Downregulation of IL-6 secretion in LPS stimulated U373 cells by NCIMB 42787 and other deposited strains (n=3).

**Figure 22:** Suppression of activation of the NFκB-AP1 promoter in stimulated HEK-TLR4 cells by NCIMB 42787 and other deposited strains (n=3).

**Figure 23:** Suppression of Enolase 2 by NCIMB 42787, NCIMB 43385, NCIMB 43388, NCIMB 43389, NCIMB 43386and NCIMB 43387.

**Figure 24:** Modulation of cytokine levels and NFκB-AP1 promoter by NCIMB 42787

**Figure 25:** Review of antioxidant capacity of NCIMB 42787.

**Figure 26:** NCIMB 42787 produces butyric, valeric and hexanoic acid.

**Figure 27:** Anti-inflammatory activity of metabolites produced by NCIMB 42787.

**Figure 28:** Analysis of role of metabolites in anti-inflammatory activity of NCIMB 42787.

**Figure 29:** NCIMB 42787 modulates the production of TNFα by stimulated splenocytes isolated from BALB/c mice.

**Figure 30:** *Megasphaera* reference strain NCIMB 43385 triggers an increase in the expression of the glucocorticoid receptor.

## DISCLOSURE OF THE INVENTION

### *Bacterial strains*

The compositions of the invention comprise a bacterial strain of the genus *Megasphaera*. The examples demonstrate that bacteria of this genus are useful for treating or preventing cancer and inflammatory and autoimmune diseases and conditions mediated by HDAC activity. The preferred bacterial strains are of the species *Megasphaera massiliensis*.

Examples of *Megasphaera* species for use in the invention include *Megasphaera elsdenii, Megasphaera cerevisiae, Megasphaera massiliensis, Megasphaera indica, Megasphaera paucivorans, Megasphaera sueciensis* and *Megasphaera micronuciformis*. A further example of a *Megasphaera* species for use in the invention is *Megasphaera hexanoica*. The *Megasphaera* are obligately anaerobic, lactate-fermenting, gastrointestinal microbe of ruminant and non-ruminant mammals, including humans.

The type strain *of M. massiliensis* is NP3 (=CSUR P245=DSM 26228)[17]. The GenBank accession number for the 16S rRNA gene sequences *of M. massiliensis* strain NP3 is JX424772.1.

The *Megasphaera massiliensis* bacterium tested in the Examples is referred to herein as strain MRx0029. A 16S rRNA sequence for the MRx0029 strain that was tested is provided in SEQ ID NO: 1.

Strain MRx0029 was deposited with the international depositary authority NCIMB, Ltd. (Ferguson Building, Aberdeen, AB21 9YA, Scotland) by 4D Pharma Research Ltd. (Life Sciences Innovation Building, Cornhill Road, Aberdeen, AB25 2ZS, Scotland) on 13th July 2017 as *"Megasphaera massiliensis* MRx0029" and was assigned accession number NCIMB 42787.

Bacterial strains closely related to the strain tested in the examples are also expected to be effective for treating or preventing inflammatory or autoimmune diseases. In certain embodiments, the bacterial strain for use in the invention has a 16S rRNA sequence that is at least 95%, 96%, 97%, 98%, 99%, 99.5% or 99.9% identical to the 16S rRNA sequence of a bacterial strain of *Megasphaera massiliensis*. Preferably, the bacterial strain for use in the invention has a 16S rRNA sequence that is at least 95%, 96%, 97%, 98%, 99%, 99.5% or 99.9% identical to SEQ ID NO:1. Preferably, the bacterial strain for use in the invention has the 16S rRNA sequence represented by SEQ ID NO: 1.

Bacterial strains that are biotypes of strains MRx0029 are also expected to be effective for treating or preventing inflammatory or autoimmune disorders. A biotype is a closely related strain that has the same or very similar physiological and biochemical characteristics.

Strains that are biotypes of strains MRx0029 and that are suitable for use in the invention may be identified by sequencing other nucleotide sequences for strains MRx0029. For example, substantially the whole genome may be sequenced and a biotype strain for use in the invention may have at least 95%, 96%, 97%, 98%, 99%, 99.5% or 99.9% sequence identity across at least 80% of its whole genome (e.g. across at least 85%, 90%, 95% or 99%, or across its whole genome). Other suitable sequences for use in identifying biotype strains may include hsp60 or repetitive sequences such as BOX, ERIC, $(GTG)_5$, or REP or [18]. Biotype strains may have sequences with at least 95%, 96%, 97%, 98%, 99%, 99.5% or 99.9% sequence identity to the corresponding sequence of the strains MRx0029.

Alternatively, strains that are biotypes of strains MRx0029 and that are suitable for use in the invention may be identified by using strains MRx0029 and restriction fragment analysis and/or PCR analysis, for example by using fluorescent amplified fragment length polymorphism (FAFLP) and repetitive DNA element (rep)-PCR fingerprinting, or protein profiling, or partial 16S or 23S rDNA sequencing. In preferred embodiments, such techniques may be used to identify other *Megasphaera massiliensis* strains.

In certain embodiments, strains that are biotypes of strains MRx0029 and that are suitable for use in the invention are strains that provide the same pattern as strains MRx0029 when analysed by amplified ribosomal DNA restriction analysis (ARDRA), for example when using Sau3AI restriction enzyme (for exemplary methods and guidance see, for example,[19]). Alternatively, biotype strains are identified as strains that have the same carbohydrate fermentation patterns as strains MRx0029.

Other *Megasphaera* strains that are useful in the compositions and methods of the invention, such as biotypes of strains MRx0029, may be identified using any appropriate method or strategy, including the assays described in the examples. For instance, strains for use in the invention may be identified by adding to cell lysate or whole cells and testing for total or Class I HDAC activity. In particular, bacterial strains that have similar growth patterns, metabolic type and/or surface antigens to strains MRx0029 may be useful in the invention. A useful strain will have comparable immune modulatory activity to strains MRx0029. In particular, a biotype strain will elicit comparable effects on the HDAC activity as shown in the Examples, which may be identified by using the culturing and administration protocols described in the Examples.

In some embodiments, bacterial strains useful in the invention may be identified by routinely profiling the production and consumption of metabolites by a bacterial strain. The inventors have found that the bacterial strain used in the Examples produces butyrate, valeric acid and hexanoic acid and consumes acetate and propionate (see Figures 15-17). The *Megasphaera massiliensis* strains Ref 1, Ref 2 and Ref 3 were also found to consume and produce these metabolites (see Figures 15-17). Therefore, in some embodiments, the bacterial strain of the invention produces one or more of the metabolites butyrate, valeric acid and hexanoic acid. In some embodiments, the bacterial strain of the invention consumes one or both of acetate and propionate. In preferred embodiments, the bacterial strain of the invention produces butyrate, valeric acid and hexanoic acid and consumes acetate and propionate.

A particularly preferred strain of the invention is the *Megasphaera massiliensis* MRx0029 strain. This is the exemplary strain tested in the examples and shown to be effective for treating disease. Therefore, the invention provides a cell, such as an isolated cell, of the *Megasphaera massiliensis* strain MRx0029, or a derivative thereof. The invention also provides a composition comprising a cell of the *Megasphaera massiliensis* strain MRx0029, or a derivative thereof. The invention also provides a biologically pure culture of the *Megasphaera massiliensis* strain MRx0029. The invention also provides a cell of the *Megasphaera massiliensis* strain MRx0029, or a derivative thereof, for use in therapy, in particular for the diseases described herein.

A particularly preferred strain of the invention is the *Megasphaera massiliensis* strain deposited under accession number NCIMB 42787. This is the exemplary MRx0029 strain tested in the examples and shown to be effective for treating disease. Therefore, the invention provides a cell, such as an isolated cell, of the *Megasphaera massiliensis* strain deposited under accession number NCIMB 42787, or a derivative thereof. The invention also provides a composition comprising a cell of the *Megasphaera massiliensis* strain deposited under accession number NCIMB 42787, or a derivative thereof. The invention also provides a biologically pure culture of the *Megasphaera massiliensis* strain deposited under accession number NCIMB 42787. The invention also provides a cell of the *Megasphaera massiliensis* strain deposited under accession number NCIMB 42787, or a derivative thereof, for use in therapy, in particular for the diseases described herein.

A derivative of the strain of the invention may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable therapeutic activity to the MRx0029 strain. In particular, a derivative strain will elicit comparable effects on HDAC activity shown in the Examples, which may be identified by using the culturing and administration protocols described in the Examples. A derivative of the MRx0029 strain will generally be a biotype of the MRx0029 strain.

References to cells of the *Megasphaera massiliensis* MRx0029 strain encompass any cells that have the same safety and therapeutic efficacy characteristics as the strain MRx0029, and such cells are encompassed by the invention.

In preferred embodiments, the bacterial strains in the compositions of the invention are viable and capable of partially or totally colonising the intestine.

The inventors have found that *Megasphaera massiliensis* strains reduce the activation of inflammatory cytokines such as IL-6. Chronic inflammation induced by IL-6 can ultimately lead to cell death. Therefore, the bacterial strains of the invention are particularly useful in the treatment or prevention of inflammatory or autoimmune disorders. In some embodiments, the bacterial strains are useful in the treatment of inflammatory or autoimmune disorders characterised by the enhanced activation of IL-6.

In preferred embodiments, the invention provides a composition comprising the strain deposited at NCIMB under accession number NCIMB 42787, or a derivative or biotype thereof, preferably for use in the treatment or prevention of inflammatory or autoimmune diseases.

In preferred embodiments, the bacterial strains in the compositions of the invention are viable and capable of partially or totally colonising the intestine.

In certain embodiments, the composition of the invention does not comprise a cell of the *Megasphaera massiliensis* strain 42787.In some embodiments, the bacterial strain in the compositions of the invention is a bacterial strain of the genus *Megasphaera,* wherein the bacterial strain is not the strain deposited under accession number NCIMB 42787.

In some embodiments, the bacterial strain in the compositions of the invention is a bacterial strain of the species *Megasphaera massiliensis,* wherein the bacterial strain is not the strain deposited under accession number NCIMB 42787.

The Examples further demonstrate other bacterial strains that are useful for treating or preventing cancer and inflammatory and autoimmune diseases and conditions mediated by HDAC activity. These bacterial strains were deposited with the international depositary authority NCIMB, Ltd. (Ferguson Building, Aberdeen, AB21 9YA, Scotland) by 4D Pharma Research Ltd. (Life Sciences Innovation Building, Cornhill Road, Aberdeen, AB25 2ZS, Scotland) on 6th May 2019 as *Megasphaera massiliensis* (under accession numbers NCIMB 43388 and NCIMB 43389) and *Megasphaera spp.* (accession numbers NCIMB 43385, NCIMB 43386 and NCIMB 43387). Accordingly, in an alternative embodiment, the compositions of the invention comprise one or more of these bacterial strains, or biotypes or derivatives thereof. For the avoidance of doubt, Ref 1 referred to above is the strain deposited under accession number NCIMB 43385, Ref 2 referred to above is the strain deposited under accession number NCIMB 43388, and Ref 3 referred to above is the strain deposited under accession number NCIMB 43389.

Bacterial strains closely related to the strains tested in the Examples are also expected to be effective for treating or preventing cancer and inflammatory and autoimmune diseases and conditions mediated by HDAC activity.

In certain embodiments, the bacterial strain for use in the invention is the *Megasphaera massiliensis* strain deposited under accession number NCIMB 43388. In certain embodiments, the invention provides a cell of the strain deposited under accession number NCIMB 43388, or a derivative thereof, for use in therapy. In certain embodiments, the invention provides a cell of the strain deposited under accession number NCIMB 43388, or derivative thereof for use in treating or preventing cancer and inflammatory and autoimmune diseases and conditions mediated by HDAC activity. In certain embodiments, the invention provides a cell of the strain deposited under accession number NCIMB 43388, for use in any one of the diseases described herein.

In preferred embodiments, the invention provides a composition comprising the strain deposited at NCIMB under accession number NCIMB 43388, or a derivative or biotype thereof, preferably for use in the treatment or prevention of inflammatory or autoimmune diseases.

In certain embodiments, the composition of the invention does not comprise a cell of the *Megasphaera* strain deposited under accession number NCIMB 43388. In some embodiments, the bacterial strain in the compositions of the invention is a bacterial strain of the genus *Megasphaera,* wherein the bacterial strain is not the strain deposited under accession number NCIMB 43388. In some embodiments, the bacterial strain in the compositions of the invention is a bacterial strain of the species *Megasphaera massiliensis,* wherein the bacterial strain is not the strain deposited under accession number NCIMB 43388.

Accordingly, in certain embodiments, the bacterial strain for use in the invention is the *Megasphaera massiliensis* strain deposited under accession number NCIMB 43389. In certain embodiments, the invention provides a cell of the strain deposited under accession number NCIMB 43389, or a derivative thereof, for use in therapy. In certain embodiments, the invention provides a cell of the strain deposited under accession number NCIMB 43389, or derivative thereof for use in treating or preventing cancer and inflammatory and autoimmune diseases and conditions mediated by HDAC activity. In certain embodiments, the invention provides a cell of the strain deposited under accession number NCIMB 43389, for use in any one of the diseases described herein.

In preferred embodiments, the invention provides a composition comprising the strain deposited at NCIMB under accession number NCIMB 43389, or a derivative or biotype thereof, preferably for use in the treatment or prevention of inflammatory or autoimmune diseases.

In certain embodiments, the composition of the invention does not comprise a cell of the *Megasphaera massiliensis* strain deposited under accession number NCIMB 43389. In some embodiments, the bacterial strain in the compositions of the invention is a bacterial strain of the genus *Megasphaera,* wherein the bacterial strain is not the strain deposited under accession number NCIMB 43389. In some embodiments, the bacterial strain in the compositions of the invention is a bacterial strain of the species *Megasphaera massiliensis,* wherein the bacterial strain is not the strain deposited under accession number NCIMB 43389.

In certain embodiments, the bacterial strain for use in the invention has a 16S rRNA sequence that is at least 95%, 96%, 97%, 98%, 99%, 99.5% or 99.9% identical to SEQ ID NO: 15. In certain embodiments, the bacterial strain for use in the invention has the 16S rRNA sequence represented by SEQ ID NO:15. In certain embodiments, the invention provides a bacterial strain having a 16S rRNA sequence that is at least 95%, 96%, 97%, 98%, 99%, 99.5% or 99.9% identical to SEQ ID NO:15 for use in therapy. In certain embodiments, the invention provides a bacterial strain having the 16S rRNA sequence represented by SEQ ID NO:15 for use in therapy.

In certain embodiments, the bacterial strain for use in the invention has a 16S rRNA sequence that is at least 95%, 96%, 97%, 98%, 99%, 99.5% or 99.9% identical to SEQ ID NO:16. In certain embodiments, the bacterial strain for use in the invention has the 16S rRNA sequence represented by SEQ ID NO: 16. In certain embodiments, the invention provides a bacterial strain having a 16S rRNA sequence that is at least 95%, 96%, 97%, 98%, 99%, 99.5% or 99.9% identical to SEQ ID NO:16 for use in therapy. In certain embodiments, the invention provides a bacterial strain having the 16S rRNA sequence represented by SEQ ID NO:16 for use in therapy.

In certain embodiments, the bacterial strain for use in the invention has a 16S rRNA sequence that is at least 95%, 96%, 97%, 98%, 99%, 99.5% or 99.9% identical to the 16S rRNA sequence of a bacterial strain of the genus *Megasphaera.* In certain embodiments, the bacterial strain for use in the invention is of the genus *Megasphaera.*

In certain embodiments, the bacterial strain for use in the invention is the *Megasphaera* strain deposited under accession number NCIMB 43385. In certain embodiments, the invention provides a cell of the strain deposited under accession number NCIMB 43385, or a derivative thereof, for use in therapy. In certain embodiments, the invention provides a cell of the strain deposited under accession number NCIMB 43385, or derivative thereof for use in treating or preventing cancer and inflammatory and autoimmune diseases and conditions mediated by HDAC activity. In certain embodiments, the invention provides a cell of the strain deposited under accession number NCIMB 43385, for use in any one of the diseases described herein.

In preferred embodiments, the invention provides a composition comprising the strain deposited at NCIMB under accession number NCIMB 43385, or a derivative or biotype thereof, preferably for use in the treatment or prevention of inflammatory or autoimmune diseases.

In certain embodiments, the composition of the invention does not comprise a cell of the *Megasphaera* strain deposited under accession number NCIMB 43385. In some embodiments, the bacterial strain in the compositions of the invention is a bacterial strain of the genus *Megasphaera,* wherein the bacterial strain is not the strain deposited under accession number NCIMB 43385. In some embodiments, the bacterial strain in the compositions of the invention is a bacterial strain of the species *Megasphaera massiliensis,* wherein the bacterial strain is not the strain deposited under accession number NCIMB 43385.

In certain embodiments, the bacterial strain for use in the invention is the *Megasphaera* strain deposited under accession number NCIMB 43386. In certain embodiments, the invention provides a cell of the strain deposited under accession number NCIMB 43386, or a derivative thereof, for use in therapy. In certain embodiments, the invention provides a cell of the strain deposited under accession number NCIMB 43386, or derivative thereof for use in treating or preventing cancer and inflammatory and autoimmune diseases and conditions mediated by HDAC activity. In certain embodiments, the invention provides a cell of the strain deposited under accession number NCIMB 43386, for use in any one of the diseases described herein.

In preferred embodiments, the invention provides a composition comprising the strain deposited at NCIMB under accession number NCIMB 43386, or a derivative or biotype thereof, preferably for use in the treatment or prevention of inflammatory or autoimmune diseases.

In certain embodiments, the composition of the invention does not comprise a cell of the *Megasphaera* strain deposited under accession number NCIMB 43386. In some embodiments, the bacterial strain in the compositions of the invention is a bacterial strain of the genus *Megasphaera,* wherein the bacterial strain is not the strain deposited under accession number NCIMB 43386. In some embodiments, the bacterial strain in the compositions of the invention is a bacterial strain of the species *Megasphaera massiliensis,* wherein the bacterial strain is not the strain deposited under accession number NCIMB 43386.

In certain embodiments, the bacterial strain for use in the invention is the *Megasphaera* strain deposited under accession number NCIMB 43387. In certain embodiments, the invention provides a cell of the strain deposited under accession number NCIMB 43387, or a derivative thereof, for use in therapy. In certain embodiments, the invention provides a cell of the strain deposited under accession number NCIMB 43387, or derivative thereof for use in treating or preventing cancer and inflammatory and autoimmune diseases and conditions mediated by HDAC activity. In certain embodiments, the invention provides a cell of the strain deposited under accession number NCIMB 43387, for use in any one of the diseases described herein.

In preferred embodiments, the invention provides a composition comprising the strain deposited at NCIMB under accession number NCIMB 43387, or a derivative or biotype thereof, preferably for use in the treatment or prevention of inflammatory or autoimmune diseases.

In certain embodiments, the composition of the invention does not comprise a cell of the *Megasphaera* strain deposited under accession number NCIMB 43387. In some embodiments, the bacterial strain in the compositions of the invention is a bacterial strain of the genus *Megasphaera,* wherein the bacterial strain is not the strain deposited under accession number NCIMB 43387. In some embodiments, the bacterial strain in the compositions of the invention is a bacterial strain of the species *Megasphaera massiliensis,* wherein the bacterial strain is not the strain deposited under accession number NCIMB 43387.

In certain embodiments, the bacterial strain for use in the invention has a 16S rRNA sequence that is at least 95%, 96%, 97%, 98%, 99%, 99.5% or 99.9% identical to SEQ ID NO:14. In certain embodiments, the bacterial strain for use in the invention has a 16S rRNA sequence that is at least 95%, 96%, 97%, 98%, 99%, 99.5% or 99.9% identical to SEQ ID NO:17. In certain embodiments, the bacterial strain for use in the invention has a 16S rRNA sequence that is at least 95%, 96%, 97%, 98%, 99%, 99.5% or 99.9% identical to SEQ ID NO:18. In certain embodiments, the bacterial strain for use in the invention has a 16S rRNA sequence that is at least 95%, 96%, 97%, 98%, 99%, 99.5% or 99.9% identical to SEQ ID NOs:14, 17 or 18. In certain embodiments, the invention provides a bacterial strain having a 16S rRNA sequence that is at least 95%, 96%, 97%, 98%, 99%, 99.5% or 99.9% identical to SEQ ID NOs:14, 17 or 18 for use in therapy.

In certain embodiments, the bacterial strain for use in the invention has the 16S rRNA sequence represented by SEQ ID NO:14. In certain embodiments, the bacterial strain for use in the invention has the 16S rRNA sequence represented by SEQ ID NO:17. In certain embodiments, the bacterial strain for use in the invention has the 16S rRNA sequence represented by SEQ ID NO:18. In certain embodiments, the bacterial strain for use in the invention has the 16S rRNA sequence represented by SEQ ID NOs: 14, 17 or 18. In certain embodiments, the invention provides a bacterial strain having the 16S rRNA sequence represented by SEQ ID NOs: 14, 17 or 18 for use in therapy.

Bacterial strains that are biotypes of one or more of the strains are also expected to be effective for treating or preventing cancer and inflammatory and autoimmune diseases and conditions mediated by HDAC activity. A biotype is a closely related strain that has the same or very similar physiological and biochemical characteristics.

In certain embodiments, the invention provides the bacterial strains deposited under accession numbers NCIMB 43385, NCIMB 43386, NCIMB 43387, NCIMB 43388 and/or NCIMB 43389, or biotypes thereof, for use in therapy.

Strains that are biotypes of one or more of the strains deposited under accession numbers NCIMB 43385, NCIMB 43386, NCIMB 43387, NCIMB 43388 and/or NCIMB 43389 and that are suitable for use in the invention may be identified by sequencing other nucleotide sequences for one or more of the strains deposited under accession numbers NCIMB 43385, NCIMB 43386, NCIMB 43387, NCIMB 43388 and/or NCIMB 43389. For example, substantially the whole genome may be sequenced and a biotype strain for use in the invention may have at least 85%, 90%, 95%, 96%, 97%, 98%, 99%, 99.5% or 99.9% sequence identity across at least 80% of its whole genome (e.g. across at least 85%, 90%, 95% or 99%, or across its whole genome). Other suitable sequences for use in identifying biotype strains may include hsp60 or repetitive sequences such as BOX, ERIC, $(GTG)_5$, or REP. Biotype strains may have sequences with at least 85%, 90%, 95%, 96%, 97%, 98%, 99%, 99.5% or 99.9% sequence identity to the corresponding sequence of one or more of the strains deposited under accession numbers NCIMB 43385, NCIMB 43386, NCIMB 43387, NCIMB 43388 and/or NCIMB 43389.

Alternatively, strains that are biotypes of one or more of the strains deposited under accession numbers NCIMB 43385, NCIMB 43386, NCIMB 43387, NCIMB 43388 and/or NCIMB 43389 and that are suitable for use in the invention may be identified by using one or more of the strains deposited under accession numbers NCIMB 43385, NCIMB 43386, NCIMB 43387, NCIMB 43388 and/or NCIMB 43389 and restriction fragment analysis and/or PCR analysis, for example by using fluorescent amplified fragment length polymorphism (FAFLP) and repetitive DNA element (rep)-PCR fingerprinting, or protein profiling, or partial 16S or 23S rDNA sequencing. In preferred embodiments, such techniques may be used to identify other *Megasphaera massiliensis* strains.

In certain embodiments, strains that are biotypes of one or more of the strains deposited under accession numbers NCIMB 43385, NCIMB 43386, NCIMB 43387, NCIMB 43388 and/or NCIMB 43389 and that are suitable for use in the invention are strains that provide the same pattern as one or more of the strains deposited under accession numbers NCIMB 43385, NCIMB 43386, NCIMB 43387, NCIMB 43388 and/or NCIMB 43389 when analysed by amplified ribosomal DNA restriction analysis (ARDRA), for example when using Sau3AI restriction enzyme. Alternatively, biotype strains are identified as strains that have the same carbohydrate fermentation patterns as one or more of the strains deposited under accession numbers NCIMB 43385, NCIMB 43386, NCIMB 43387, NCIMB 43388 and/or NCIMB 43389.

Other strains that are useful in the compositions and methods of the invention, such as biotypes of one or more of the strains deposited under accession numbers NCIMB 43385, NCIMB 43386, NCIMB 43387, NCIMB 43388 and/or NCIMB 43389, may be identified using any appropriate method or strategy, including the assays described in the Examples. For instance, strains for use in the invention may be identified by adding to cell lysate or whole cells and testing for total or Class I HDAC activity. In particular, bacterial strains that have similar growth patterns, metabolic type and/or surface antigens to one or more of the strains deposited under accession numbers NCIMB 43385, NCIMB 43386, NCIMB 43387, NCIMB 43388 and/or NCIMB 43389 may be useful in the invention. A useful strain will have comparable immune modulatory activity to one or more of the strains deposited under accession numbers NCIMB 43385, NCIMB 43386 NCIMB 43387, NCIMB 43388 and/or NCIMB 43389. In particular, a biotype strain will elicit comparable effects on the HDAC activity as shown in the Examples, which may be identified by using the culturing and administration protocols described in the Examples.

In certain embodiments, preferred strains of the invention are strains deposited under accession numbers NCIMB 43385, NCIMB 43386, NCIMB 43387, NCIMB 43388 and/or NCIMB 43389. These are exemplary strains tested in the Examples and shown to be effective for treating disease. Therefore, the invention provides a cell, such as an isolated cell, of one or more of the strains deposited under accession numbers NCIMB 43385, NCIMB 43386, NCIMB 43387, NCIMB 43388 and/or NCIMB 43389, or a derivative thereof. The invention also provides a composition comprising a cell of one of more of the strains deposited under accession numbers NCIMB 43385, NCIMB 43386, NCIMB 43387, NCIMB 43388 and/or NCIMB 43389, or a derivative thereof. The invention also provides a biologically pure culture of one or more of the strains deposited under accession numbers NCIMB 43385, NCIMB 43386, NCIMB 43387, NCIMB 43388 and/or NCIMB 43389. The invention also provides a cell of one or more of the strains deposited under accession numbers NCIMB 43385, NCIMB 43386, NCIMB 43387, NCIMB 43388 and/or NCIMB 43389, or a derivative thereof, for use in therapy, in particular for the diseases described herein.

A derivative of the strain of the invention may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable therapeutic activity to one or more of the strains deposited under accession numbers NCIMB 43385, NCIMB 43386, NCIMB 43387, NCIMB 43388 and/or NCIMB 43389. In particular, a derivative strain will elicit comparable effects on HDAC activity shown in the Examples, which may be identified by using the culturing and administration protocols described in the Examples. A derivative of one or more of the strains deposited under accession numbers NCIMB 43385, NCIMB 43386, NCIMB 43387, NCIMB 43388 and/or NCIMB 43389 will generally be a biotype of one or more of the strains deposited under accession numbers NCIMB 43385, NCIMB 43386, NCIMB 43387, NCIMB 43388 and/or NCIMB 43389, respectively.

The inventors have found that the bacterial strains used in the Examples produce 2-methyl-propanoic acid and 3-methyl-propanoic acid and consumes formic acid (see Figure 20). The strains deposited under accession numbers NCIMB 43385, NCIMB 43388 and NCIMB 43389 were also found to produce 2-methyl-propanoic acid and 3-methyl-propanoic acid. In addition, the strains deposited under accession numbers NCIMB 43385 and NCIMB 43388 were also found to consume formic acid. Therefore, in some embodiments, the bacterial strain of the invention produces one or more of the metabolites 2-methyl-propanoic acid and 3-methyl-propanoic acid. In some embodiments, the bacterial strain of the invention consumes formic acid. In some embodiments, the bacterial strain of the invention produces 2-methyl-propanoic acid and 3-methyl-propanoic acid and consumes formic acid. In preferred embodiments, the bacterial strain of the invention produces butyrate, valeric acid, hexanoic acid, 2-methyl-propanoic acid and 3-methyl-propanoic acid, and consumes acetate, propionate and formic acid.

In certain embodiments, the production of butyrate inhibits IL-6 secretion. Accordingly, in certain embodiments, the compositions of the invention may reduce inflammation in light of the reduction in IL-6 upon the production of butyrate.

In certain embodiments, the bacterial strains of the invention produce valeric acid. In certain embodiments, the bacterial strains of the invention are *Megasphaera* strains which produce valeric acid. Valeric acid inhibits HDAC activity and is therefore useful in the treatment of autoimmune and inflammatory disorders. Therefore, in certain embodiments, the compositions of the invention inhibit HDAC activity via increasing the production of valeric acid. In certain embodiments, the compositions of the present invention are therapeutically beneficial in inflammatory, autoimmune and/or disorders disclosed herein in light of the increase in valeric acid production and the inhibition of HDAC activity by valeric acid.

In certain embodiments, the compositions of the invention do not comprise *Megasphaera elsdenii.* In certain embodiments, the bacterial strain useful in the compositions and methods of the invention is not *Megasphaera elsdenii.*

*Therapeutic uses*

As demonstrated in the examples, the bacterial compositions of the invention are effective for reducing the HDAC activity. In particular, treatment with compositions of the invention achieves a reduction in Class 1 HDAC activity. In particular, treatment with the compositions of the invention achieves a reduction in HDAC1, 2 and 3 activity. In particular, treatment with the compositions of the invention achieves a reduction in HDAC1 and 2 activity. Therefore, the compositions of the invention may be useful for treating or preventing diseases or conditions mediated by HDAC activity. A condition may be a symptom of a disease. In particular, the compositions of the invention may be useful for reducing or preventing diseases or conditions mediated by elevated levels of HDAC activity. In particular, the compositions of the invention may be useful for reducing or preventing diseases or conditions mediated by elevated levels of Class I HDAC activity. In particular, the compositions of the invention may be useful for reducing or preventing diseases or conditions mediated by elevated levels of HDAC1, 2 or 3 activity.

Histone deacetylases are a class of enzymes that remove acetyl groups from protein targets. The most abundant HDAC target are histones, but HDACs are known to deacetylate lysine residues of non-histone protein targets to temporally regulate protein activity. As such, HDACs are sometimes referred to as lysine deacetylases. There are currently 13 known HDACs which are categorised into four main classes class I (HDACs 1, 2, 3 and 8), class IIa (HDACs 4,5,7 and 9) and class IIb (HDACs 6 and 10), Class III (sirt1-sirt7) and class IV (HDAC 11) [7]. Each class generally has a different tissue expression pattern and subcellular localisation.

Protein acetylation/deacetylation is generally used a mechanism of post-translational control of protein activity Histone acetylation/deacetylation is a well-established mechanism of transcriptional regulation. Genetic regulation is caused by histone deacetylase-mediated cleavage of an acetyl group from a ε-N-acetyl of a lysine amino acid in a histone tail. Removal of the acetyl group restores positive charge to the histone tail, leading to more favourable binding to the negative charged phosphodiester DNA backbone. Improved binding leads to tighter chromosome compaction and an overall reduction in gene expression at the site of histone deacetylation.

Histone deacetylase activity has been implicated in a wide array of diseases and conditions. Inhibition of histone deacetylase activity can be used to alleviate or ameliorate these diseases or conditions. Pan-inhibitors of histone deacetylases may be useful in the treatment or prevention of HDAC-mediated diseases. Isoform specific HDAC inhibitors may be useful in the treatment or prevention of diseases mediated by specific HDAC isoform activity.

Inhibition of HDAC activity is an established treatment modality and a number of HDAC inhibitors are approved medicines, including: Vorinostat (CTCL), Romidepsin (CTCL), Chidamide (PTCL), Panobinostat (multiple myeloma), Belinostat (T cell lymphoma), and many are in clinical trials, including: Panobinostat (CTCL), valproic acid (cervical cancer and ovarian cancer, spinal muscular atrophy), Mocetinostat (follicular lymphoma, Hodgkin lymphoma and acute myeloid leukemia), Abexinostat (sarcoma), Entinostat (Hodgkin lymphoma, lung cancer and breast cancer), SB939 (Recurrent or Metastatic Prostate Cancer), Resminostat (Hodgkin lymphoma), Givinostat (refractory leukemias and myelomas), HBI-800 (Advanced Solid Tumors Including Melanoma, Renal Cell Carcinoma (RCC), and Non-Small Cell Lung Cancer (NSCLC)) , Kevetrin (ovarian cancer), CUDC-101, AR-42 (relapsed or treatment-resistant multiple myelom, chronic lymphocytic leukemia or lymphoma), CHR-2845, CHR-3996, 4SC-202 (advanced haematological indications), CG200745 (solid tumours), ACY-1215 (multiple myeloma), ME-344 (solid refractory tumours), sulforaphane, and Trichostatin (anti-inflammatory).

Examples of diseases or conditions mediated by HDAC activity include inflammatory or autoimmune disease, such as asthma, arthritis, psoriasis, diabetes, allograft rejection, graft-versus-host disease, or an inflammatory bowel disease, such as Crohn's disease or ulcerative colitis, and cancer, such as prostate cancer, colorectal cancer, breast cancer, lung cancer, liver cancer or gastric cancer. In certain embodiments the compositions of the invention are used to treat or prevent one of these diseases or conditions. In certain embodiments, the compositions of the invention are used to treat or prevent one of these diseases or conditions mediated by HDAC activity. In certain embodiments, the compositions of the invention are used to treat or prevent one of these diseases or conditions mediated by Class I HDAC activity. In certain embodiments, the compositions of the invention are used to treat or prevent one of these diseases or conditions mediated by HDAC1, 2 or 3 activity.

In certain embodiments, the compositions of the invention are for use in therapy. In certain embodiments, the compositions of the invention are for use in the treatment of prevention of a disease or condition mediated by HDAC activity. In certain embodiments, the compositions of the invention are for use in a method of reducing HDAC activity in the treatment or prevention of a disease or condition mediated by HDAC activity. In some embodiments, the compositions of the invention are for use in treating or preventing a disease or condition mediated by Class I HDAC activity. In certain embodiments, the compositions of the invention are for use in a method of inhibiting Class I HDAC activity. In certain embodiments, the compositions of the invention are for use in a method of selectively inhibiting Class I HDAC activity in the treatment or prevention of a disease mediated by Class I HDAC activity. The inventors have identified that certain compositions of the invention selectively inhibit Class I HDACs. As used herein "selective" refers to compositions that have the greatest inhibitory effect on Class I HDACs, for example, in comparison to their inhibitory effect of HDACs from other classes. Selective inhibition of HDACs is advantageous for the treatment of diseases that require long-term administration of a therapeutic agent, for example where a disease or condition needs to be treated throughout the lifetime of a patient. In certain embodiments, the compositions of the invention that are Class I HDAC selective inhibitors are for use in the palliative treatment or prevention of a disease or condition mediated by Class I HDAC activity. Selective inhibitors are advantageous over pan-inhibitors known in the art by reducing side effects associated with the unwanted inhibition of other classes of HDACs. In certain embodiments, the compositions of the invention are HDAC2 selective inhibitors. In certain embodiments, the compositions of the invention are for use in a method of selectively reducing HDAC1, 2 or 3 activity. In certain embodiments, the compositions of the invention are for use in the treatment or prevention of a disease mediated by HDAC1, 2 or 3 activity.

In certain embodiments, the composition of the invention is for use in
GPR109a

In certain embodiments, the composition of the invention is not for use in treating cancer. In certain embodiments, the composition of the invention is for use in treating a disease or disorder that is not cancer.

*Inflammatory and autoimmune disorders*

The examples demonstrate that the compositions of the invention have HDAC inhibitory activity. HDAC activity is central to the pathology of many inflammatory and autoimmune disorders, and HDAC inhibitors have shown efficacy in the treatment of many inflammatory and autoimmune disorders, as discussed below in relation to specific conditions (see also [20]). Therefore, the compositions of the invention may be useful for treating inflammatory and autoimmune disorders, in particular inflammatory and autoimmune disorders mediated by histone deacetylase (HDAC) activity.

In certain embodiments, the compositions of the invention are for use in a method of treating or preventing an inflammatory or autoimmune disorder. In certain embodiments, the compositions of the invention are for use in treating or preventing an inflammatory or autoimmune disease, wherein said treatment or prevention is achieved by reducing or preventing HDAC activation. In certain embodiments, the compositions of the invention are for use in treating a patient with an inflammatory or autoimmune disease, wherein the patient has elevated HDAC levels or activity. In certain embodiments, the patient may have been diagnosed with a chronic inflammatory or autoimmune disease or condition, or the composition of the invention may be for use in preventing an inflammatory or autoimmune disease or condition developing into a chronic inflammatory or autoimmune disease or condition. In certain embodiments, the disease or condition may not be responsive to treatment with TNF-$\alpha$ inhibitors.

HDAC may be associated with chronic inflammatory and autoimmune diseases, so the compositions of the invention may be particularly useful for treating or preventing chronic diseases or conditions as listed above. In certain embodiments, the compositions are for use in patients with chronic disease. In certain embodiments, the compositions are for use in preventing the development of chronic disease.

The compositions of the invention may be useful for treating diseases and conditions mediated by HDAC and for addressing HDAC activation, so the compositions of the invention may be particularly useful for treating or preventing chronic disease, treating or preventing disease in patients that have not responded to other therapies (such as treatment with TNF-α inhibitors), and/or treating or preventing the tissue damage and symptoms associated with HDAC.

In certain preferred embodiments, the compositions of the invention modulate pro-inflammatory cytokine production and inflammation. In certain embodiments, the compositions of the invention modulate the inflammatory state. In certain embodiments, the compositions of the invention decrease IL-6 production and secretion. In certain embodiments, the compositions of the invention decrease the activation of the NFκB promoter. In certain embodiments, the compositions of the invention are able to modulate the activation of IL-6 production by the potent pro-inflammatory endotoxin lipopolysaccharide (LPS).

In certain embodiments, the compositions of the invention are for use in treating an inflammatory or autoimmune disorder that does not affect or involve the nervous system. In certain embodiments, the compositions are for use in treating an inflammatory or autoimmune disorder that affects the gastrointestinal tract, musculoskeletal system, respiratory system or skin. In certain embodiments, the compositions of the invention are for treating a disease that is not a neurodegenerative disorder. In certain embodiments, the compositions of the invention are for treating a disease that is not Parkinson's disease, including progressive supranuclear palsy, progressive supranuclear palsy, Steele-Richardson-Olszewski syndrome, normal pressure hydrocephalus, vascular or arteriosclerotic parkinsonism and drug-induced parkinsonism; Alzheimer's disease, including Benson's syndrome; Huntington's disease; amyotrophic lateral sclerosis; Lou Gehrig's disease; motor neurone disease; prion disease; spinocerebellar ataxia; spinal muscular atrophy; dementia, including Lewy body, vascular and frontotemporal dementia; primary progressive aphasia; mild cognitive impairment; HIV-related cognitive impairment or corticobasal degeneration. In certain embodiments, the compositions of the invention are for use in treating a subject with healthy nervous system. In certain embodiments, the compositions of the invention are for treating a disease that is not multiple sclerosis. In certain embodiments, the compositions of the invention are for treating a disease that is not a brain injury. In certain embodiments, the compositions of the invention are for treating a disease that is not a neurodegenerative disorder and is not multiple sclerosis and is not a brain injury.

The examples also demonstrate that the compositions of the invention are effective for upregulating GPR109a, which is known to suppress inflammation [62]. In certain embodiments, the compositions of the invention are for use in upregulating GPR109a in the treatment of an inflammatory or autoimmune disease.

The examples also demonstrate that the compositions of the invention are effective for suppressing enolase, which is known to have pro-inflammatory activities [69]. In certain embodiments, the compositions of the invention are for use in suppressing enolase in the treatment of an inflammatory or autoimmune disease.

- **Inflammatory bowel disease**

The examples demonstrate that the compositions of the invention have HDAC inhibitory activity, and so they may be useful in the treatment of inflammatory bowel disease. Overexpression of different HDAC isoforms have been implicated in a variety of disease pathologies, including colitis. Additionally, valproic acid has been associated with class I HDAC inhibition and amelioration of colitis in a DSS-colitis murine model [21]. This study suggested a role for HDAC class I inhibitors in IFN-γ, IL-10, IL-1β and TNF-α suppression, assigning functionality to HDAC inhibition and efficacy in colitis. Therefore, the examples indicate that the compositions of the invention may be useful for treating inflammatory bowel diseases.

In certain embodiments, the compositions of the invention are for use in treating or preventing inflammatory bowel disease. In certain embodiments, the compositions of the invention are for use in treating or preventing inflammatory bowel disease, wherein said treatment or prevention is achieved by reducing or preventing HDAC activation. In certain embodiments, the compositions of the invention are for use in treating a patient with inflammatory bowel disease, wherein the patient has elevated HDAC levels or activity.

Inflammatory bowel disease (IBD) is a complex disease that can be caused by multiple environmental and genetic factors. Factors contributing to the onset of IBD include diet, microbiota, intestinal permeability, and genetic susceptibility to increased inflammatory response to gut infection. Symptoms of inflammatory bowel disease include abdominal pain, vomiting, diarrhoea, rectal bleeding, severe internal cramps/muscle spasms in the pelvic region, weight loss and anaemia. In certain embodiments, the compositions are for use in reducing one or more symptoms associated with IBD. In certain embodiments, the compositions of the invention are for use in preventing one or more symptoms of IBD.

IBD may accompany other diseases or conditions, such as arthritis, pyoderma gangrenosum, primary sclerosing cholangitis, non-thyroidal illness syndrome, deep vein thrombosis, bronchiolitis obliterans organizing pneumonia. In certain embodiments, the compositions of the invention are for use in the treatment or prevention of one or more diseases or conditions that accompany IBD.

Inflammatory bowel disease is generally diagnosed by biopsy or colonoscopy. Measurements of faecal calprotectin is useful for the preliminary diagnosis of IBD. Other laboratory test for the diagnosis of IBD include, complete blood count, erythrocyte sedimentation rate, comprehensive metabolic panel, faecal occult blood test or C-reactive protein test. Typically a combination of laboratory testing and biopsy/colonoscopy will be used to confirm diagnosis of IBD. In certain embodiments, the of the invention are for use in a subject diagnosed with IBD.

In certain embodiments, the inflammatory bowel disease is ulcerative colitis. Ulcerative colitis is an autoimmune inflammatory bowel disease characterised by infiltrating T cells. HDAC inhibitors have previously been shown to ameliorate colitis in a DSS-colitis murine model [21]. Furthermore the inventors have shown that compositions of the invention reduce leukocyte infiltration in the ileum of animals with colitis. Therefore, the compositions of the invention may be for use in the treatment or prevention of ulcerative colitis. In some embodiments, the compositions of the invention may be for use in the treatment of ulcerative colitis by reducing leukocyte infiltration in the ileum of a subject with ulcerative colitis.

UC is usually restricted to the rectum and colon but sometimes involves the ileum. The disease is classified depending on the extent of involvement of the gastrointestinal tract. Classifications of ulcerative colitis include distal colitis, such as proctitis, proctosigmoiditis and left-sided colitis, or extensive colitis, such as pancolitis. In certain embodiments, the compositions are for use in the treatment of distal colitis. In certain embodiments, the compositions are for use in the treatment of proctitis. In certain embodiments, the compositions are for use in the treatment of proctosigmoiditis. In certain embodiments, the compositions are for use in the treatment of left-sided colitis. In certain embodiments, the compositions are for use in the treatment of extensive colitis. In certain embodiments, the compositions are for use in the treatment of pancolitis. In certain embodiments, the compositions are for use in the prevention of ulcerative colitis in a subject at risk of developing ulcerative colitis.

Ulcerative colitis is diagnosed by a combination of laboratory testing and surgery, such as endoscopy/colonoscopy and biopsy. Exemplary laboratory test that aid ulcerative colitis diagnosis include complete blood count, complete metabolic panel, liver function tests, urinalysis, stool culture, erythrocyte sedimentation rate and C-reactive protein measurement.

The severity of symptoms of ulcerative colitis can be determined using the Simple Clinical Colitis Activity Index (SCCAI) [22]. SCCAI can also be used as a means to assess efficacy of therapies designed to treat or prevent ulcerative colitis. SCCAI poses the following series of questions designed to determine the severity of ulcerative colitis symptoms: frequency of bowel movements (by day); frequency of bowel movements (by night); urgency of defecation; blood in stool; general well-being; extra-colonic features (for example, arthritis, uveitis, or other conditions that accompany UC). Each answer is provided on a sliding scale generating a score of between 0 and 19. A score of above 5 is usually indicative of the presence of ulcerative colitis.

In some embodiments, the composition is for use in a subject who has been diagnosed with ulcerative colitis. In some embodiments, the compositions are for use in alleviating or ameliorating one or more symptoms of ulcerative colitis. For example, the compositions may improve the score of one or more answers to the SCCAI. In certain embodiments, the compositions of the invention may be for use in reducing the frequency of bowel movements. In certain embodiments, the compositions of the invention may be for use in reducing urgency of defecation. In certain embodiments, the compositions of the invention may be for use in reducing blood in stool. In certain embodiments, the compositions of the invention may be for use in reducing extra-colonic features. The alleviation or amelioration of these symptoms may be determined by an improvement in the corresponding SCCAI score pre- and post-administration of a composition of the invention.

Additional symptoms of ulcerative colitis include diarrhoea, rectal bleeding, weight loss and anaemia, abdominal pain, abdominal cramping with bowel movements. In some embodiments, the compositions of the invention are for use in the treatment or prevention of one or more additional symptoms of ulcerative colitis.

In some instances, ulcerative colitis is accompanied by one or more extra-colonic features. Extra-colonic features are conditions or diseases that accompany ulcerative colitis and manifest outside the colon. Examples of extra-colonic features of ulcerative colitis include: aphthous ulcers, iritis, uveitis, episcleritis, seronegative arthritis, ankylosing spondylitis, sacroiliitis, erythema nodosum, pyoderma grangrenosum, deep venous thrombosis and pulmonary embolism, autoimmune haemolytic anaemia, clubbing, primary sclerosing cholangitis. In some embodiments, the compositions of the invention are for use in treating or preventions one or more extra-colonic features of ulcerative colitis.

Ulcerative colitis may be treated with a number of therapeutics agents, such as 5-aminosalicylic acids, such as sulfasalazine and mesalazine, corticosteroids, such as prednisone, immunosuppressive agents, such as azathioprine, biologics, such as infliximab, adalimumab, and golimumab, vedolizumab and etrolizumab, nicotine, or iron. In certain embodiments, the compositions of the invention are for in the treatment or prevention of ulcerative colitis in combination with an additional therapeutic agent, wherein the additional therapeutic agent is for the treatment or prevention of ulcerative colitis.

In certain embodiments the inflammatory bowel disease is Crohn's disease. Studies have shown that several HDACs are upregulated in the inflammatory muscosa of patients with Crohn's disease. Therefore, inhibition of HDAC activity may be useful in the treatment of Crohn's disease. In certain embodiments, the compositions of the invention are for use in the treatment or prevention of Crohn's disease.

Crohn's disease is a complex disease with an array of probable causes, including genetic risk factors, diet, other lifestyle factors, such as smoking and alcohol consumption, and microbiome composition. Crohn's disease can manifest anywhere along the gastrointestinal tract.

Gastrointestinal symptoms of Crohn's disease range from mild to severe and include abdominal pain, diarrhoea, faecal blood, ileitis, increased bowel movements, increased flatulence, intestinal stenosis, vomiting, and perianal discomfort. The compositions of the invention may be for use in the treatment of prevention of one or more gastrointestinal symptoms of Crohn's disease.

Systemic symptoms of Crohn's disease include growth defects, such as the inability to maintain growth during puberty, decreased appetite, fever and weight loss. Extra-intestinal features of Crohn's disease include uveitis, photobia, episcleritis, gall stones, seronegative spondyloarthropathy, arthritis, enthesitis, erythema nodosum, pyoderma gangrenosum, deep venous thrombosis, pulmonary embolism, autoimmune haemolytic anaemia, clubbing and osteoporosis. Extra-intestinal features are additional conditions associated with Crohn's disease that manifest outside the GI tract. Subjects with Crohn's disease also exhibit increased susceptibility to neurological complications such as seizures, strokes, myopathy, peripheral neuropathy, headache and depression. In certain embodiments, the compositions of the invention are for use in the treatment or prevention of one or more systemic symptoms of Crohn' disease. In certain embodiments, the compositions of the invention are for use in the treatment or prevention of one or more extra-intestinal features of Crohn's disease.

The diagnosis of Crohn's disease usually involves carrying out multiple tests and surgical procedures, such as gastroscopy and/or colonoscopy and biopsy, typically of the ileum, radiologic tests, complete blood counts, C-reactive protein tests and erythrocyte sedimentation rates. In certain embodiments, the compositions of the invention are for use in subjects diagnosed with Crohn's disease. In some embodiments, compositions of the invention are for use in treating a subject who has been diagnosed with Crohn's disease.

Crohn's disease is classified depending on the extent of the region of the GI tract affected [23]. A disease of both the ileum and colon is classified as Ileocolic Crohn's. In some embodiments, the compositions are for use in the treatment or prevention of Ileocolic Crohn's. In some embodiments, the compositions are for use in a subject diagnosed with Ileocolic Crohn's/ Crohn's ileitis is classified if only the ileum is affected. Crohn's colitis is classified if only the colon is affected. In certain embodiments, the compositions are for use in the treatment or prevention of Crohn's ileitis. In some embodiments, the compositions are for use in a subject diagnosed with Crohn's ileitis. In certain embodiments, the compositions are for use in the treatment or prevention of Crohn's colitis. In some embodiments, the compositions are for use in a subject diagnosed with Crohn's colitis.

Crohn's disease may be treated with a number of therapeutic agents, such as corticosteroids, such as prednisone, immunosuppressive agents, such as azathioprine, or biologics, such as infliximab, adalimumab, and golimumab, vedolizumab and etrolizumab. In certain embodiments, the compositions of the invention are for use in the treatment or prevention of Crohn's disease in combination with an additional therapeutic agent. In certain embodiments, the additional therapeutic agent is for use in the treatment or prevention of Crohn's disease.

The examples also demonstrate that the compositions of the invention are effective for upregulating GPR109a, which is known to suppress colonic inflammation [62]. In certain embodiments, the compositions of the invention are for use in upregulating GPR109a in the treatment of inflammatory bowel disease.

- **Arthritis**

Arthritis is a disease characterised by chronic joint inflammation. Rheumatoid arthritis is a chronic autoimmune disorder that typically results in swollen and painful joints. HDAC inhibition has been proposed to treat rheumatoid arthritis by a variety of mechanisms, including influencing cytokine production, inhibiting T-cell differentiation, suppressing proliferation of synovial fibroblasts and reducing bone loss by influencing osteoclasts and osteoblasts (Vojinov et al., 2011, Mol Med, 17 (5-6) 397-403). HDAC inhibition has been shown to have a strong anti-inflammatory effect in several animal models of arthritis (Joosten et al., 2011, Mol Med, 17 (5-6), 391-396). Therefore, the compositions of the invention may be useful for treating or preventing arthritis in a subject.

In preferred embodiments, the compositions of the invention are for use in treating or preventing rheumatoid arthritis (RA). In certain embodiments, the compositions of the invention are for use in treating or preventing rheumatoid arthritis, wherein said treatment or prevention is achieved by reducing or preventing HDAC activation. In certain embodiments, the compositions of the invention are for use in treating a patient with rheumatoid arthritis, wherein the patient has elevated HDAC levels or activity.

In certain embodiments, treatment with the compositions of the invention results in a reduction in the swelling of joints. In certain embodiments, the compositions of the invention are for use in patients with swollen joints or patients identified as at risk of having swollen joints. In certain embodiments, the compositions of the invention are for use in a method of reducing joint swelling in RA.

In certain embodiments, treatment with the compositions of the invention results in a reduction in cartilage damage or bone damage. In certain embodiments, the compositions of the invention are for use in reducing or preventing cartilage or bone damage in the treatment of RA. In certain embodiments, the compositions are for use in treating patient with severe RA that are at risk of cartilage or bone damage.

In certain embodiments, the compositions of the invention are for use in preventing bone erosion or cartilage damage in the treatment of RA. In certain embodiments, the compositions are for use in treating patients that exhibit bone erosion or cartilage damage or patients identified as at risk of bone erosion or cartilage damage.

The compositions of the invention may be useful for modulating a patient's immune system, so in certain embodiments the compositions of the invention are for use in preventing RA in a patient that has been identified as at risk of RA, or that has been diagnosed with early-stage RA. The compositions of the invention may be useful for preventing the development of RA.

The compositions of the invention may be useful for managing or alleviating RA. The compositions of the invention may be particularly useful for reducing symptoms associated with joint swelling or bone destruction. Treatment or prevention of RA may refer to, for example, an alleviation of the severity of symptoms or a reduction in the frequency of exacerbations or the range of triggers that are a problem for the patient.

- **Asthma**

Asthma is a chronic inflammatory respiratory disease. HDAC inhibitors have been shown to have anti-inflammatory effects that relieve airway inflammation, airway remodelling and airway hypersensitivity in a mouse model of chronic asthma (Ren et al., 2016, Inflamm Res, 65, 995-1008). Therefore, the compositions of the invention may be useful for treating or preventing asthma in a subject.

In preferred embodiments, the compositions of the invention are for use in treating or preventing asthma. In certain embodiments, the compositions of the invention are for use in treating or preventing asthma, wherein said treatment or prevention is achieved by reducing or preventing HDAC activation. In certain embodiments, the compositions of the invention are for use in treating a patient with asthma, wherein the patient has elevated HDAC levels or activity.

In certain embodiments, the asthma is eosinophilic or allergic asthma. Eosinophilic and allergic asthma are characterised by increased numbers of eosinophils in peripheral blood and in airway secretions and is associated pathologically with thickening of the basement membrane zone and pharmacologically by corticosteroid responsiveness [24]. Compositions that reduce or inhibit eosinophil recruitment or activation may be useful for treating or preventing eosinophilic and allergic asthma. Eosinophilic and allergic asthma are also characterised by a cascade of inflammatory events mediated by T helper type 2 lymphocyte (Th2) processes. Compositions that reduce or inhibit T helper type 2 lymphocyte (Th2) processes may therefore be useful for treating or preventing eosinophilic and allergic asthma.

In additional embodiments, the compositions of the invention are for use in treating or preventing neutrophilic asthma (or non-eosinophilic asthma). High neutrophil numbers are associated with severe asthma that may be insensitive to corticosteroid treatment. Compositions that reduce or inhibit neutrophil recruitment or activation may be useful for treating or preventing neutrophilic asthma.

Eosinophilic asthma (also referred to as Th2-high asthma) and neutrophilic asthma (also referred to as Th2-low or non-Th2 asthma) have different underlying pathophysiological mechanisms and present different clinical features. For example, Th2-high asthma generally presents early onset and exhibits seasonal variations of symptoms, whereas Th2-low asthma has a much later onset, typically around the age of 40 or later. Th2-high asthma is also characterised by increased immunoglobulin E (IgE) blood levels, whereas this feature is absent in Th2-low asthma. Th2 high asthma is also characterised by high sputum levels of eosinophils. By contrast, Th2-low asthma may be characterised by elevated levels of sputum neutrophils. In certain embodiments, the compositions of the invention are for use in treating Th2-low or non-Th2 asthma. In certain embodiments, the compositions of the invention are for use in treating Th2-high asthma.

Eosinophilic and neutrophilic asthma are not mutually exclusive conditions and treatments that help address either the eosinophil and neutrophil responses may be useful for treating asthma in general.

In certain embodiments, the compositions of the invention are for use in methods reducing an eosinophilic inflammatory response in the treatment or prevention of asthma, or for use in methods of reducing a neutrophilic inflammatory response in the treatment or prevention of asthma. As noted above, high levels of eosinophils in asthma is associated pathologically with thickening of the basement membrane zone, so reducing eosinophilic inflammatory response in the treatment or prevention of asthma may be able to specifically address this feature of the disease. Also, elevated neutrophils, either in combination with elevated eosinophils or in their absence, is associated with severe asthma and chronic airway narrowing. Therefore, reducing the neutrophilic inflammatory response may be particularly useful for addressing severe asthma.

In certain embodiments, the compositions reduce peribronchiolar infiltration in allergic asthma, or are for use in reducing peribronchiolar infiltration in the treatment of allergic asthma. In certain embodiments, the compositions reduce peribronchiolar and/or perivascular infiltration in neutrophilic asthma, or are for use in reducing peribronchiolar and/or perivascular infiltration in the treatment of allergic neutrophilic asthma.

In certain embodiments, treatment with compositions of the invention provides a reduction or prevents an elevation in TNF-$\alpha$ levels.

In certain embodiments, the compositions of the invention are for use in a method of treating asthma that results in a reduction of the eosinophilic and/or neutrophilic inflammatory response. In certain embodiments, the patient to be treated has, or has previously been identified as having, elevated neutrophil or eosinophil levels, for example as identified through blood sampling or sputum analysis.

The compositions of the invention may be useful for preventing the development of asthma in a new-born when administered to the new-born, or to a pregnant woman. The compositions may be useful for preventing the development of asthma in children. The compositions of the invention may be useful for treating or preventing adult-onset asthma. The compositions of the invention may be useful for managing or alleviating asthma. The compositions of the invention may be particularly useful for reducing symptoms associated with asthma that is aggravated by allergens, such as house dust mites.

Treatment or prevention of asthma may refer to, for example, an alleviation of the severity of symptoms or a reduction in the frequency of exacerbations or the range of triggers that are a problem for the patient.

- Psoriasis

Psoriasis is a chronic inflammatory skin disease. Overexpression of HDAC1 has been reported for in skin biopsies from psoriatic pateints (Tovar-Castillo et al., 2007, Int J Dermatol, 46, 239-46) and a HDAC inhibitor has been shown to block the conversion of Foxp3+ Tregs into Foxp3-ROR$\gamma$t+ IL-17/Tregs (a shift associated with psoriasis disease progression) (Bovenschen et al., 2011, J Invest Dermatol, 131, 1853-60). Therefore, the compositions of the invention may be useful for treating or preventing psoriasis in a subject.

In preferred embodiments, the compositions of the invention are for use in treating or preventing psoriasis. In certain embodiments, the compositions of the invention are for use in treating or preventing psoriasis, wherein said treatment or prevention is achieved by reducing or preventing HDAC activation. In certain embodiments, the compositions of the invention are for use in treating a patient with psoriasis, wherein the patient has elevated HDAC levels or activity.

- **Systemic lupus erythematosus**

Systemic lupus erythematosus (SLE) is an autoimmune disease. HDAC inhibition is believed to be a promising therapeutic approach for treating SLE based on studies on cell cultures and mouse models of SLE (Reilly et al., 2011, Mol Med, 17 (5-6), 417-425). Therefore, the compositions of the invention may be useful for treating or preventing systemic lupus erythematosus in a subject.

In preferred embodiments, the compositions of the invention are for use in treating or preventing SLE. In certain embodiments, the compositions of the invention are for use in treating or preventing SLE, wherein said treatment or prevention is achieved by reducing or preventing HDAC activation. In certain embodiments, the compositions of the invention are for use in treating a patient with SLE, wherein the patient has elevated HDAC levels or activity.

- **Allograft rejection**

Allograft rejection occurs when transplanted tissues are rejected by the recipient's immune system. Studies on murine cardiac transplants have shown that HDAC inhibition increases intra-graft histone 3 acetylation and is associated with increased intra-graft levels of Foxp3 protein (a forkhead transcription family member involved in controlling immune responses), maintenance of tissue architecture and a lack of the stigmata of chronic rejection relative to controls (Wang et al., Immunol Cell Biol, 1-8). Therefore, the compositions of the invention may be useful for treating or preventing allograft rejection in a subject.

In preferred embodiments, the compositions of the invention are for use in treating or preventing allograft rejection. In certain embodiments, the compositions of the invention are for use in treating or preventing allograft rejection, wherein said treatment or prevention is achieved by reducing or preventing HDAC activation. In certain embodiments, the compositions of the invention are for use in treating a patient with allograft rejection, wherein the patient has elevated HDAC levels or activity.

- **Diabetes**

Diabetes mellitus is a group of diseases in which low levels of insulin and/or peripheral insulin resistance lead to hyperglycermia. HDAC inhibition has been proposed to treat diabetes by a variety of mechanisms, including de-repression of *Pdxl* (Park et al., 2008, J Clin Invest, 118, 2316-24), enhancing expression of transcription factor *Ngn3* to increase the pool of endocrine progenitor cells (Haumaitre et al., 2008, Mol Cell Biol, 28, 6373-83) and enhancing insulin expression (Molsey et al., 2003, J Biol Chem, 278, 19660-6) amongst others. HDAC inhibition is also a promising treatment for late diabetic complications such as diabetic nephropathy and retinal ischemia (Christensen et al., 2011, Mol Med, 17 (5-6), 370-390). Therefore, the compositions of the invention may be useful for treating or preventing diabetes in a subject.

In preferred embodiments, the compositions of the invention are for use in treating or preventing diabetes. In preferred embodiments, the compositions of the invention are for use in treating or preventing type I diabetes. In preferred embodiments, the compositions of the invention are for use in treating or preventing type II diabetes. In certain embodiments, the compositions of the invention are for use in treating or preventing diabetes, wherein said treatment or prevention is achieved by reducing or preventing HDAC activation. In certain embodiments, the compositions of the invention are for use in treating a patient with diabetes, wherein the patient has elevated HDAC levels or activity.

- **Graft-versus-host Disease (GVHD)**

The compositions of the invention may be for use in the treatment or prevention of Graft-versus-host disease (GVHD). GVHD is a medical complication following transplantation of allogeneic tissue into a subject. GVHD commonly occurs following stem cell or bone marrow transplantation or solid organ transplantation, particularly where the genetic background of the graft (i.e. the donor) and the host (i.e. the recipient) are distinct.

The pathophysiology of GVHD comprises three distinct phases. Firstly, host antigen presenting cells (APCs), such as dendritic cells (DCs) are activated following recognition of the transplanted tissue as a foreign substance. APC activation precedes the recruitment and activation of effector immune cells, such as conventional cytotoxic T cells, which leads to destruction or rejection of the foreign tissue.

HDAC inhibition has been shown to mediate potent pleiotropic anti-inflammatory effects useful in the treatment or prevention of GVHD. HDAC inhibition may inhibit at multiple points of the GVHD pathophysiological cascade. For example, HDAC inhibition prevents antigen presenting cell and dendritic cell activation against allogeneic tissues *in vivo* by enhancing the expression of indoleamine 2,3-dioxygenase in a STAT-3 dependent manner [25]. HDAC inhibition of STAT-1 activity has also been shown to be beneficial in the treatment or prevention of GVHD [26]. In certain embodiments, the composition of the invention may be for use in the treatment or prevention of GVHD by inhibiting APC activation.

HDAC inhibition has also been shown to expand Treg cell populations and activity in vivo [27]. HDAC inhibition-mediated upregulation of Treg cell activity has been shown to supress conventional cytotoxic T cell activity, which may be useful in the treatment or prevention of GVHD by supressing the 2nd phase of the GVHD pathophysiological cascade. In certain embodiments, the compositions of the invention are for use in the treatment or prevention of GVHD by reducing conventional cytotoxic T cell activity. In certain embodiments, the compositions of the invention may be for use in reducing conventional cytotoxic T cell activity. In certain embodiments, the composition of the invention may be for use in the treatment or prevention of GVHD by upregulating Treg cell activity.

Donor NK cells have been shown to reduce GVHD by eliminating host APCs. HDAC inhibition has been shown to increase NK cell activity. Therefore, the compositions of the invention may be for use to increase NK cell activity, which may be useful in the treatment or prevention of GVHD by increasing the elimination of APCs. In certain embodiments, the compositions of the invention may be for use in the treatment or prevention of GVHD by enhancing the elimination of host APCs. In certain embodiments, the compositions of the invention may be for use in the treatment or prevention of GVHD by enhancing NK cell activity. In certain embodiments, the compositions of the invention may be for use in the treatment or prevention of GVHD by enhancing NK cell activity-mediated elimination of host APCs.

In certain embodiments, the compositions of the invention may be administered after the host has received the transplant. In certain embodiments, the compositions of the invention may be administered to the host before the subject has received the transplant. Administration of the compositions of the invention before the transplant has been received may be useful in priming the immune system of the subject to not elicit an inflammatory or autoimmune response against the transplanted tissue. In certain embodiments, the compositions of the invention may be used for preventing or preventing the onset of GVHD. In certain embodiments, the composition of the invention may be for use in the treatment or prevention of GVHD prophylactically. In certain embodiments, the compositions of the invention may be used in the prophylaxis of GVHD. In certain embodiments, the compositions of the invention may be for use in a method of preventing transplant tissue rejection in a subject.

In certain embodiments, the compositions of the invention may be useful for treating, delaying, preventing, or preventing the onset of acute GVHD. Symptoms of acute GVHD typically manifest within the first 100 days of transplantation. Delaying, treatment or prevention of acute GVHD may be particularly beneficial to aid the recovery of subjects in the immediate aftermath of transplant surgery. In certain embodiments, the compositions may treat, delay the onset of, prevent or prevent the onset of acute GVHD by inhibiting HDAC activity. In certain embodiments, the compositions may treat, delay the onset of, prevent, or prevent the onset of acute GVHD by upregulating Treg cell activity. The compositions may treat, delay the onset of, prevent or prevent the onset of acute GVHD by inhibiting conventional cytotoxic T cell activity. The compositions of the invention may treat, delay the onset of, prevent or prevent the onset of acute GVHD by enhancing NK cell activity. The compositions of the invention may treat, delay the onset of, prevent or prevent the onset of acute GVHD by inhibiting APC activation.

In certain embodiments, the compositions of the invention may treat, delay the onset of, prevent, or prevent the onset of acute GVHD when administered to a subject within 100 days following transplantation. In certain embodiments, the compositions of the invention may treat, delay the onset of, prevent, or prevent the onset of acute GVHD when administered to a subject prophylactically, for example, when the composition is administered to the subject before the transplant. In certain embodiments, the compositions of the invention may treat, delay the onset of, prevent, or prevent the onset of persistent, late-onset or recurrent acute GVHD, such as acute GVHD that occurs or recurs more than 100 days after transplantation.

In certain embodiments, the composition of the invention may treat, delay the onset of, prevent, or prevent the onset one or more symptoms of acute GVHD selected from the list consisting of macropaular skin rash, nausea, anorexia, diarrhea, severe abdominal pain, ileus and cholestatic hyperbilirubinemia.

In certain embodiments, the compositions of the invention may be useful for treating, delaying the onset of, preventing, or preventing the onset of chronic GVHD. Chronic GVHD is a complex, multisystem disorder that can involve any organ and is typically characterised by fibrosis. Chronic GVHD may evolve from acute GVHD, or may emerge after a period of quiescence following acute GVHD, or may emerge de novo. Symptoms of chronic GVHD may emerge at any time following transplantation. In certain embodiments, the compositions may be useful for treating, preventing, preventing the onset of, or delaying the onset of chronic GVHD by inhibiting HDAC activity. The compositions may treat, delay the onset of, prevent, or prevent the onset of chronic GVHD by upregulating Treg cell activity. The compositions may treat, delay the onset of, prevent, or prevent the onset of chronic GVHD by inhibiting conventional cytotoxic T cell activity. The compositions of the invention may treat, delay the onset of, prevent, or prevent the onset of chronic GVHD by enhancing NK cell activity. The compositions of the invention may treat, delay the onset of, prevent, or prevent the onset of chronic GVHD by inhibiting APC DC activation.

In certain embodiments, the compositions of the invention are for administration to a patient that has recently undergone a stem cell, bone marrow or solid organ transplant. In certain embodiments, the compositions of the invention are for administration to a patient is in need of a stem cell, bone marrow or solid organ transplant.

In certain embodiments, the composition of the invention may treat, delay the onset of, prevent, or prevent the onset of one or more symptoms of chronic GVHD selected from the list consisting of: dyspigmentation, new-onset alopecia, poikiloderma,lichen planuslike eruptions or sclerotic features, nail dystrophy or loss, xerostomia, mouth ulcers (such as aphthous stomatitis), lichen-type features in the mouth (such as lichen sclerosis), keratoconjunctivitis sicca, sicca syndrome, cicatricial conjunctivitis, fascititis, myostitis, joint stiffness, vaginal sclerosis, ulcerations, anorexia, weight loss, oesophageal web, jaundice, transaminitis, pleural effusions, bronchiolitis obliterans, nephrotic syndrome, pericarditis, thrombocytopenia, anemia, and neutropenia.

In certain embodiments, the compositions of the invention may be for use in combination with one or more pharmacological agents for the treatment or prevention of GVHD. In certain embodiments, the one or more pharmacological agents are for the pharmacological prevention or treatment of GVHD. In certain embodiments, the compositions of the invention are for use in the treatment or prevention of GVHD in a subject who is receiving, has received, or is about to receive, one or more of said pharmacological agents. In certain embodiments, the one or more pharmacological agents are selected from the list consisting of: suberoylanilide, vorisnostat, ITF2357 cyclosporine, ciclosporin, sirolimus, pentostatin, rituximab, imatinib, mycophenolate mofetil, tacrolimus, prednisone, methotrexate, remestemcel-L and Prochymal, wherein the pharmacological agent is administered in a therapeutically effective amount for the treatment or prevention of GVHD. In some embodiments, the compositions of the invention are for use in the treatment of GVHD in a subject who has received, is receiving, or is about to receive extracorporeal photophoreses.

### *Cancer*

HDAC function and expression is perturbed in a variety of cancers and often leads to poor prognosis. HDAC function in cancer is associated with the aberrant expression or function of genes that promote cellular proliferation and tumorigenic phenotypes. In certain cancers HDACs primarily regulate the onset of cancer and are described as oncogenes. In other cancers onco-fusion proteins recruit Class I HDACs to repress the expression of genes that regulate cellular differentiation or cell cycle control, leading to cellular transformation. The knockdown or inhibition of HDAC expression has been shown to have multiple anti-cancer effects, such as cell cycle arrest and inhibition of proliferation, apoptosis, differentiation and senescence and disruption of angiogenesis. Therefore, the compositions of the invention may be useful in the treatment of cancers mediated by HDAC activity, by inhibiting HDAC activity.

In certain embodiments, the compositions of the invention are for use in treating or preventing cancer. In certain embodiments, the composition of the invention are for use in treating or preventing cancers mediated by HDAC activity. In certain embodiments, the compositions of the invention are for use in treating or preventing colorectal cancer.

In certain embodiments, treatment with the compositions of the invention results in a reduction in tumour size or a reduction in tumour growth. In certain embodiments, the compositions of the invention are for use in reducing tumour size or reducing tumour growth. The compositions of the invention may be effective for reducing tumour size or growth. In certain embodiments, the compositions of the invention are for use in patients with solid tumours. In certain embodiments, the compositions of the invention are for use in reducing or preventing angiogenesis in the treatment of cancer. Genes regulated by HDACs have central roles in angiogenesis. In certain embodiments, the compositions of the invention are for use in preventing metastasis.

In certain embodiments, the compositions of the invention are for use in treating or preventing gastric cancer. HDAC2 has been shown to play a functional role in the development of gastric cancers and colorectal tumorigenesis [28,29]. In mice models of colorectal cancer, inhibition of HDAC2 resulted in a reduced rates of tumour development. In certain embodiments, the compositions of the invention that selectively inhibit HDAC2 are for use in treating or preventing colorectal cancer, in particular colorectal cancer mediated by HDAC2 activity.

In certain embodiments, the compositions of the invention are for use in treating or preventing breast cancer. The compositions of the invention may be effective for treating breast cancer, and HDACs have been shown to be upregulated in breast cancer [30]. In certain embodiments, the compositions of the invention are for use in reducing tumour size, reducing tumour growth, or reducing angiogenesis in the treatment of breast cancer.

In certain embodiments, the compositions of the invention are for use in treating or preventing prostate cancer. The compositions of the invention may be effective for treating prostate cancer, as HDAC activity play a major role in the development of prostate cancer [31]. In certain embodiments, the compositions of the invention are for use in reducing tumour size, reducing tumour growth, or reducing angiogenesis in the treatment of prostate cancer. In certain embodiments, the cancer is hormone refractory prostate cancer.

In certain embodiments, the compositions of the invention are for use in treating or preventing lung cancer. The compositions of the invention may be effective for treating lung cancer, and HDACs have been shown to be upregulated in lung cancer [32]. In certain embodiments, the compositions of the invention are for use in reducing tumour size, reducing tumour growth, or reducing angiogenesis in the treatment of lung cancer. In preferred embodiments the cancer is lung carcinoma. In preferred embodiments, the compositions are for use in the treatment of lung cancer with high levels of expression of HDAC2. Certain lung cancer tissues have be shown to abundantly express HDAC2. Inactivation of HDAC2 represses lung cancer cell growth. High levels of HDAC2 activity has been shown to repress p53 activity [33]. Active p53 arrests cell division and ultimately leads to the onset of apoptosis. In certain embodiments, compositions of the invention that inhibit HDAC2 are for use in the treatment of lung cancers with high levels of HDAC2 activity.

In certain embodiments, the compositions of the invention are for use in treating or preventing liver cancer. The compositions of the invention may be effective for treating liver cancer, and HDACs have been shown to be upregulated in liver cancer [34]. In certain embodiments, the compositions of the invention are for use in reducing tumour size, reducing tumour growth, or reducing angiogenesis in the treatment of liver cancer. In preferred embodiments the cancer is hepatoma (hepatocellular carcinoma).

In certain embodiments, the cancer is a low-grade or early-stage tumour

In certain embodiments, the compositions of the invention are for use in treating or preventing carcinoma. The compositions of the invention may be particularly effective for treating carcinoma. In certain embodiments, the compositions of the invention are for use in treating or preventing nonimmunogenic cancer. The compositions of the invention may be effective for treating nonimmunogenic cancers.

In further embodiments, the compositions of the invention are for use in treating or preventing acute lymphoblastic leukemia (ALL), acute myeloid leukemia, adrenocortical carcinoma, basal-cell carcinoma, bile duct cancer, bladder cancer, bone tumor, osteosarcoma/malignant fibrous histiocytoma, brainstem glioma, brain tumor, cerebellar astrocytoma, cerebral astrocytoma/malignant glioma, ependymoma, medulloblastoma, supratentorial primitive neuroectodermal tumors, breast cancer, bronchial adenomas/carcinoids, Burkitt's lymphoma, carcinoid tumor, cervical cancer, chronic lymphocytic leukemia, chronic myelogenous leukemia, chronic myeloproliferative disorders, colon cancer, cutaneous T-cell lymphoma, endometrial cancer, ependymoma, esophageal cancer, Ewing's sarcoma, intraocular melanoma, retinoblastoma, gallbladder cancer, gastric cancer, gastrointestinal carcinoid tumor, gastrointestinal stromal tumor (GIST), germ cell tumor, glioma, childhood visual pathway and hypothalamic, Hodgkin lymphoma, melanoma, islet cell carcinoma, Kaposi sarcoma, renal cell cancer, laryngeal cancer, leukaemias, lymphomas, mesothelioma, neuroblastoma, non-Hodgkin lymphoma, oropharyngeal cancer, osteosarcoma, ovarian cancer, pancreatic cancer, parathyroid cancer, pharyngeal cancer, pituitary adenoma, plasma cell neoplasia, prostate cancer, renal cell carcinoma, retinoblastoma, sarcoma, testicular cancer, thyroid cancer, or uterine cancer.

The compositions of the invention may be particularly effective when used in combination with further therapeutic agents. The HDAC inhibitory effects of the compositions of the invention may be effective when combined with more direct anti-cancer agents. Therefore, in certain embodiments, the invention provides a composition comprising a bacterial strain of the genus *Megasphaera* and an anticancer agent. In preferred embodiments the anticancer agent is an immune checkpoint inhibitor, a targeted antibody immunotherapy, a CAR-T cell therapy, an oncolytic virus, or a cytostatic drug. In preferred embodiments, the composition comprises an anti-cancer agent selected from the group consisting of: Yervoy (ipilimumab, BMS); Keytruda (pembrolizumab, Merck); Opdivo (nivolumab, BMS); MEDI4736 (AZ/MedImmune); MPDL3280A (Roche/Genentech); Tremelimumab (AZ/MedImmune); CT-011 (pidilizumab, CureTech); BMS-986015 (lirilumab, BMS); MEDI0680 (AZ/MedImmune); MSB-0010718C (Merck); PF-05082566 (Pfizer); MEDI6469 (AZ/MedImmune); BMS-986016 (BMS); BMS-663513 (urelumab, BMS); IMP321 (Prima Biomed); LAG525 (Novartis); ARGX-110 (arGEN-X); PF-05082466 (Pfizer); CDX-1127 (varlilumab; CellDex Therapeutics); TRX-518 (GITR Inc.); MK-4166 (Merck); JTX-2011 (Jounce Therapeutics); ARGX-115 (arGEN-X); NLG-9189 (indoximod, NewLink Genetics); INCB024360 (Incyte); IPH2201 (Innate Immotherapeutics/AZ); NLG-919 (NewLink Genetics); anti-VISTA (JnJ); Epacadostat (INCB24360, Incyte); F001287 (Flexus/BMS); CP 870893 (University of Pennsylvania); MGA271 (Macrogenix); Emactuzumab (Roche/Genentech); Galunisertib (Eli Lilly); Ulocuplumab (BMS); BKT140/BL8040 (Biokine Therapeutics); Bavituximab (Peregrine Pharmaceuticals); CC 90002 (Celgene); 852A (Pfizer); VTX-2337 (VentiRx Pharmaceuticals); IMO-2055 (Hybridon, Idera Pharmaceuticals); LY2157299 (Eli Lilly); EW-7197 (Ewha Women's University, Korea); Vemurafenib (Plexxikon); Dabrafenib (Genentech/GSK); BMS-777607 (BMS); BLZ945 (Memorial Sloan-Kettering Cancer Centre); Unituxin (dinutuximab, United Therapeutics Corporation); Blincyto (blinatumomab, Amgen); Cyramza (ramucirumab, Eli Lilly); Gazyva (obinutuzumab, Roche/Biogen); Kadcyla (adotrastuzumab emtansine, Roche/Genentech); Perjeta (pertuzumab, Roche/Genentech); Adcetris (brentuximab vedotin, Takeda/Millennium); Arzerra (ofatumumab, GSK); Vectibix (panitumumab, Amgen); Avastin (bevacizumab, Roche/Genentech); Erbitux (cetuximab, BMS/Merck); Bexxar (tositumomab-I131, GSK); Zevalin (ibritumomab tiuxetan, Biogen); Campath (alemtuzumab, Bayer); Mylotarg (gemtuzumab ozogamicin, Pfizer); Herceptin (trastuzumab, Roche/Genentech); Rituxan (rituximab, Genentech/Biogen); volociximab (Abbvie); Enavatuzumab (Abbvie); ABT-414 (Abbvie); Elotuzumab (Abbvie/BMS); ALX-0141 (Ablynx); Ozaralizumab (Ablynx); Actimab-C (Actinium); Actimab-P (Actinium); Milatuzumab-dox (Actinium); Emab-SN-38 (Actinium); Naptumonmab estafenatox (Active Biotech); AFM13 (Affimed); AFM11 (Affimed); AGS-16C3F (Agensys); AGS-16M8F (Agensys); AGS-22ME (Agensys); AGS-15ME (Agensys); GS-67E (Agensys); ALXN6000 (samalizumab, Alexion); ALT-836 (Altor Bioscience); ALT-801 (Altor Bioscience); ALT-803 (Altor Bioscience); AMG780 (Amgen); AMG 228 (Amgen); AMG820 (Amgen); AMG172 (Amgen); AMG595 (Amgen); AMG110 (Amgen); AMG232 (adecatumumab, Amgen); AMG211 (Amgen/MedImmune); BAY20-10112 (Amgen/Bayer); Rilotumumab (Amgen); Denosumab (Amgen); AMP-514 (Amgen); MEDI575 (AZ/MedImmune); MEDI3617 (AZ/MedImmune); MEDI6383 (AZ/MedImmune); MEDI551 (AZ/MedImmune); Moxetumomab pasudotox (AZ/MedImmune); MEDI565 (AZ/MedImmune); MEDI0639 (AZ/MedImmune); MEDI0680 (AZ/MedImmune); MEDI562 (AZ/MedImmune); AV-380 (AVEO); AV203 (AVEO); AV299 (AVEO); BAY79-4620 (Bayer); Anetumab ravtansine (Bayer); vantictumab (Bayer); BAY94-9343 (Bayer); Sibrotuzumab (Boehringer Ingleheim); BI-836845 (Boehringer Ingleheim); B-701 (BioClin); BIIB015 (Biogen); Obinutuzumab (Biogen/Genentech); BI-505 (Bioinvent); BI-1206 (Bioinvent); TB-403 (Bioinvent); BT-062 (Biotest) BIL-010t (Biosceptre); MDX-1203 (BMS); MDX-1204 (BMS); Necitumumab (BMS); CAN-4 (Cantargia AB); CDX-011 (Celldex); CDX1401 (Celldex); CDX301 (Celldex); U3-1565 (Daiichi Sankyo); patritumab (Daiichi Sankyo); tigatuzumab (Daiichi Sankyo); nimotuzumab (Daiichi Sankyo); DS-8895 (Daiichi Sankyo); DS-8873 (Daiichi Sankyo); DS-5573 (Daiichi Sankyo); MORab-004 (Eisai); MORab-009 (Eisai); MORab-003 (Eisai); MORab-066 (Eisai); LY3012207 (Eli Lilly); LY2875358 (Eli Lilly); LY2812176 (Eli Lilly); LY3012217(Eli Lilly); LY2495655 (Eli Lilly); LY3012212 (Eli Lilly); LY3012211 (Eli Lilly); LY3009806 (Eli Lilly); cixutumumab (Eli Lilly); Flanvotumab (Eli Lilly); IMC-TR1 (Eli Lilly); Ramucirumab (Eli Lilly); Tabalumab (Eli Lilly); Zanolimumab (Emergent Biosolution); FG-3019 (FibroGen); FPA008 (Five Prime Therapeutics); FP-1039 (Five Prime Therapeutics); FPA144 (Five Prime Therapeutics); catumaxomab (Fresenius Biotech); IMAB362 (Ganymed); IMAB027 (Ganymed); HuMax-CD74 (Genmab); HuMax-TFADC (Genmab); GS-5745 (Gilead); GS-6624 (Gilead); OMP-21M18 (demcizumab, GSK); mapatumumab (GSK); IMGN289 (ImmunoGen); IMGN901 (ImmunoGen); IMGN853 (ImmunoGen); IMGN529 (ImmunoGen); IMMU-130 (Immunomedics); milatuzumab-dox (Immunomedics); IMMU-115 (Immunomedics); IMMU-132 (Immunomedics); IMMU-106 (Immunomedics); IMMU-102 (Immunomedics); Epratuzumab (Immunomedics); Clivatuzumab (Immunomedics); IPH41 (Innate Immunotherapeutics); Daratumumab (Janssen/Genmab); CNTO-95 (Intetumumab, Janssen); CNTO-328 (siltuximab, Janssen); KB004 (KaloBios); mogamulizumab (Kyowa Hakko Kirrin); KW-2871 (ecromeximab, Life Science); Sonepcizumab (Lpath); Margetuximab (Macrogenics); Enoblituzumab (Macrogenics); MGD006 (Macrogenics); MGF007 (Macrogenics); MK-0646 (dalotuzumab, Merck); MK-3475 (Merck); Sym004 (Symphogen/Merck Serono); DI17E6 (Merck Serono); MOR208 (Morphosys); MOR202 (Morphosys); Xmab5574 (Morphosys); BPC-1C (ensituximab, Precision Biologics); TAS266 (Novartis); LFA102 (Novartis); BHQ880 (Novartis/Morphosys); QGE031 (Novartis); HCD122 (lucatumumab, Novartis); LJM716 (Novartis); AT355 (Novartis); OMP-21M18 (Demcizumab, OncoMed); OMP52M51 (Oncomed/GSK); OMP-59R5 (Oncomed/GSK); vantictumab (Oncomed/Bayer); CMC-544 (inotuzumab ozogamicin, Pfizer); PF-03446962 (Pfizer); PF-04856884 (Pfizer); PSMA-ADC (Progenics); REGN1400 (Regeneron); REGN910 (nesvacumab, Regeneron/Sanofi); REGN421 (enoticumab, Regeneron/Sanofi); RG7221, RG7356, RG7155, RG7444, RG7116, RG7458, RG7598, RG7599, RG7600, RG7636, RG7450, RG7593, RG7596, DCDS3410A, RG7414 (parsatuzumab), RG7160 (imgatuzumab), RG7159 (obintuzumab), RG7686, RG3638 (onartuzumab), RG7597 (Roche/Genentech); SAR307746 (Sanofi); SAR566658 (Sanofi); SAR650984 (Sanofi); SAR153192 (Sanofi); SAR3419 (Sanofi); SAR256212 (Sanofi), SGN-LIV1A (lintuzumab, Seattle Genetics); SGN-CD33A (Seattle Genetics); SGN-75 (vorsetuzumab mafodotin, Seattle Genetics); SGN-19A (Seattle Genetics) SGN-CD70A (Seattle Genetics); SEA-CD40 (Seattle Genetics); ibritumomab tiuxetan (Spectrum); MLN0264 (Takeda); ganitumab (Takeda/Amgen); CEP-37250 (Teva); TB-403 (Thrombogenic); VB4-845 (Viventia); Xmab2512 (Xencor); Xmab5574 (Xencor); nimotuzumab (YM Biosciences); Carlumab (Janssen); NY-ESO TCR (Adaptimmune); MAGE-A-10 TCR (Adaptimmune); CTL019 (Novartis); JCAR015 (Juno Therapeutics); KTE-C19 CAR (Kite Pharma); UCART19 (Cellectis); BPX-401 (Bellicum Pharmaceuticals); BPX-601 (Bellicum Pharmaceuticals); ATTCK20 (Unum Therapeutics); CAR-NKG2D (Celyad); Onyx-015 (Onyx Pharmaceuticals); H101 (Shanghai Sunwaybio); DNX-2401 (DNAtrix); VCN-01 (VCN Biosciences); Colo-Adl (PsiOxus Therapeutics); ProstAtak (Advantagene); Oncos-102 (Oncos Therapeutics); CG0070 (Cold Genesys); Pexa-vac (JX-594, Jennerex Biotherapeutics); GL-ONC1 (Genelux); T-VEC (Amgen); G207 (Medigene); HF10 (Takara Bio); SEPREHVIR (HSV1716, Virttu Biologics); OrienX010 (OrienGene Biotechnology); Reolysin (Oncolytics Biotech); SVV-001 (Neotropix); Cacatak (CVA21, Viralytics); Alimta (Eli Lilly), cisplatin, oxaliplatin, irinotecan, folinic acid, methotrexate, cyclophosphamide, 5-fluorouracil, Zykadia (Novartis), Tafinlar (GSK), Xalkori (Pfizer), Iressa (AZ), Gilotrif (Boehringer Ingelheim), Tarceva (Astellas Pharma), Halaven (Eisai Pharma), Veliparib (Abbvie), AZD9291 (AZ), Alectinib (Chugai), LDK378 (Novartis), Genetespib (Synta Pharma), Tergenpumatucel-L (NewLink Genetics), GV1001 (Kael-GemVax), Tivantinib (ArQule); Cytoxan (BMS); Oncovin (Eli Lilly); Adriamycin (Pfizer); Gemzar (Eli Lilly); Xeloda (Roche); Ixempra (BMS); Abraxane (Celgene); Trelstar (Debiopharm); Taxotere (Sanofi); Nexavar (Bayer); IMMU-132 (Immunomedics); E7449 (Eisai); Thermodox (Celsion); Cometriq (Exellxis); Lonsurf (Taiho Pharmaceuticals); Camptosar (Pfizer); UFT (Taiho Pharmaceuticals); and TS-1 (Taiho Pharmaceuticals).

In certain embodiments, the composition of the invention is for use in a method of inducing GPR109a gene expression in the treatment or prevention of cancer.

In certain embodiments, the composition of the invention is for use in treating colorectal cancer, such as colorectal adenocarcinoma. The Caco-2 cell line used in the examples is a colorectal adenocarcinoma cell line and the compositions of the invention were shown to have a useful effect on such cells.

In certain embodiments, the compositions are for use in treating or preventing metastatic melanoma, small cell lung cancer or adenosqamous lung carcinoma. The effect on NSE shown in the examples suggests that the compositions of the invention may be particular effective against these cancers.

In certain embodiments, the compositions of the invention display intrinsic antioxidant capacity. Indeed, antioxidant capacity is useful for the treatment or prevention of cancer, in particular by the avoidance of those types of free radical damaged associated with cancer development. In certain embodiments, the compositions of the invention treat or prevent cancer via the antioxidant capacity.

*Modes of **administration***

Preferably, the compositions of the invention are to be administered to the gastrointestinal tract in order to enable delivery to and / or partial or total colonisation of the intestine with the bacterial strain of the invention. Generally, the compositions of the invention are administered orally, but they may be administered rectally, intranasally, or via buccal or sublingual routes.

In certain embodiments, the compositions of the invention may be administered as a foam, as a spray or a gel.

In certain embodiments, the compositions of the invention may be administered as a suppository, such as a rectal suppository, for example in the form of a theobroma oil (cocoa butter), synthetic hard fat (e.g. suppocire, witepsol), glycero-gelatin, polyethylene glycol, or soap glycerin composition.

In certain embodiments, the composition of the invention is administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to the stomach, jejunum and other suitable access ports.

The compositions of the invention may be administered once, or they may be administered sequentially as part of a treatment regimen. In certain embodiments, the compositions of the invention are to be administered daily.

In certain embodiments of the invention, treatment according to the invention is accompanied by assessment of the patient's gut microbiota. Treatment may be repeated if delivery of and / or partial or total colonisation with the strain of the invention is not achieved such that efficacy is not observed, or treatment may be ceased if delivery and / or partial or total colonisation is successful and efficacy is observed.

In certain embodiments, the composition of the invention may be administered to a pregnant animal, for example a mammal such as a human in order to prevent an inflammatory or autoimmune disease developing in her child *in utero* and / or after it is born.

The compositions of the invention may be administered to a patient that has been diagnosed with a disease or condition mediated histone deacetylase activity, or that has been identified as being at risk of a disease or condition mediated by histone deacetylase activity. The compositions may also be administered as a prophylactic measure to prevent the development of diseases or conditions mediated by histone deacetylase activity in a healthy patient.

The compositions of the invention may be administered to a patient that has been identified as having an abnormal gut microbiota. For example, the patient may have reduced or absent colonisation by *Megasphaera,* and in particular *Megasphaera massiliensis.*

The compositions of the invention may be administered as a food product, such as a nutritional supplement.

Generally, the compositions of the invention are for the treatment of humans, although they may be used to treat animals including monogastric mammals such as poultry, pigs, cats, dogs, horses or rabbits. The compositions of the invention may be useful for enhancing the growth and performance of animals. If administered to animals, oral gavage may be used.

*Compositions*

Generally, the composition of the invention comprises bacteria. In preferred embodiments of the invention, the composition is formulated in freeze-dried form. For example, the composition of the invention may comprise granules or gelatin capsules, for example hard gelatin capsules, comprising a bacterial strain of the invention.

Preferably, the composition of the invention comprises lyophilised bacteria. Lyophilisation of bacteria is a well-established procedure and relevant guidance is available in, for example, references [35,,37].

Alternatively, the composition of the invention may comprise a live, active bacterial culture.

In preferred embodiments, the composition of the invention is encapsulated to enable delivery of the bacterial strain to the intestine. Encapsulation protects the composition from degradation until delivery at the target location through, for example, rupturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule. Guidance on encapsulation that may be useful for preparing compositions of the invention is available in, for example, references [38] and [39].

The composition may be administered orally and may be in the form of a tablet, capsule or powder. Encapsulated products are preferred because *Megasphaera* are anaerobes. Other ingredients (such as vitamin C, for example), may be included as oxygen scavengers and prebiotic substrates to improve the delivery and / or partial or total colonisation and survival *in vivo.* Alternatively, the probiotic composition of the invention may be administered orally as a food or nutritional product, such as milk or whey based fermented dairy product, or as a pharmaceutical product.

The composition may be formulated as a probiotic.

A composition of the invention includes a therapeutically effective amount of a bacterial strain of the invention. A therapeutically effective amount of a bacterial strain is sufficient to exert a beneficial effect upon a patient. A therapeutically effective amount of a bacterial strain may be sufficient to result in delivery to and / or partial or total colonisation of the patient's intestine.

A suitable daily dose of the bacteria, for example for an adult human, may be from about $1x\ 10^3$ to about $1 \times 10^{11}$ colony forming units (CFU); for example, from about $1 \times 10^7$ to about $1 \times 10^{10}$ CFU; in another example from about $1 \times 10^6$ to about $1 \times 10^{10}$ CFU; in another example from about $1 \times 10^7$ to about $1 \times 10^{11}$ CFU; in another example from about $1 \times 10^8$ to about $1 \times 10^{10}$ CFU; in another example from about $1 \times 10^8$ to about $1 \times 10^{11}$ CFU.

In certain embodiments, the dose of the bacteria is at least $10^9$ cells per day, such as at least $10^{10}$, at least $10^{11}$, or at least $10^{12}$ cells per day.

In certain embodiments, the composition contains the bacterial strain in an amount of from about $1 \times 10^6$ to about $1 \times 10^{11}$ CFU/g, respect to the weight of the composition; for example, from about $1 \times 10^8$ to about $1 \times 10^{10}$ CFU/g. The dose may be, for example, 1 g, 3g, 5g, and 10g.

In certain embodiments, the invention provides the above pharmaceutical composition, wherein the amount of the bacterial strain is from about $1 \times 10^3$ to about $1 \times 10^{11}$ colony forming units per gram with respect to a weight of the composition.

In certain embodiments, the invention provides the above pharmaceutical composition, wherein the composition is administered at a dose of between 500mg and 1000mg, between 600mg and 900mg, between 700mg and 800mg, between 500mg and 750mg or between 750mg and 1000mg. In certain embodiments, the invention provides the above pharmaceutical composition, wherein the lyophilised bacteria in the pharmaceutical composition is administered at a dose of between 500mg and 1000mg, between 600mg and 900mg, between 700mg and 800mg, between 500mg and 750mg or between 750mg and 1000mg.

Typically, a probiotic, such as the composition of the invention, is optionally combined with at least one suitable prebiotic compound. A prebiotic compound is usually a non-digestible carbohydrate such as an oligo- or polysaccharide, or a sugar alcohol, which is not degraded or absorbed in the upper digestive tract. Known prebiotics include commercial products such as inulin and transgalactooligosaccharides.

In certain embodiments, the probiotic composition of the present invention includes a prebiotic compound in an amount of from about 1 to about 30% by weight, respect to the total weight composition, (e.g. from 5 to 20% by weight). Carbohydrates may be selected from the group consisting of: fructo- oligosaccharides (or FOS), short-chain fructo-oligosaccharides, inulin, isomaltoligosaccharides, pectins, xylo-oligosaccharides (or XOS), chitosan-oligosaccharides (or COS), betaglucans, arable gum modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

The compositions of the invention may comprise pharmaceutically acceptable excipients or carriers. Examples of such suitable excipients may be found in the reference [40]. Acceptable carriers or diluents for therapeutic use are well known in the pharmaceutical art and are described, for example, in reference [41]. Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

The compositions of the invention may be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the composition of the invention is formulated as a milk-based product. The term "milk-based product" means any liquid or semi-solid milk- or whey- based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks; flavoured milks, ice cream; milk-containing food such as sweets.

In certain embodiments, the compositions of the invention contain a single bacterial strain or species and do not contain any other bacterial strains or species. Such compositions may comprise only *de minimis* or biologically irrelevant amounts of other bacterial strains or species. Such compositions may be a culture that is substantially free from other species of organism.

The compositions for use in accordance with the invention may or may not require marketing approval.

In some cases, the lyophilised bacterial strain is reconstituted prior to administration. In some cases, the reconstitution is by use of a diluent described herein.

The compositions of the invention can comprise pharmaceutically acceptable excipients, diluents or carriers.

In certain embodiments, the invention provides a pharmaceutical composition comprising: a bacterial strain of the invention; and a pharmaceutically acceptable excipient, carrier or diluent; wherein the bacterial strain is in an amount sufficient to treat a disorder when administered to a subject in need thereof; and wherein the disorder is selected from the group consisting of inflammatory bowel diseases, such as Crohn's disease or ulcerative colitis, cancer, such as prostate cancer, colorectal cancer, breast cancer, lung cancer, liver cancer or gastric cancer.

In certain embodiments, the invention provides pharmaceutical composition comprising: a bacterial strain of the invention; and a pharmaceutically acceptable excipient, carrier or diluent; wherein the bacterial strain is in an amount sufficient to treat or prevent a disease or condition mediated by HDAC. In preferred embodiments, said disease or condition is selected from the group consisting of an inflammatory or autoimmune disease, such as asthma, arthritis, psoriasis, diabetes, allograft rejection, graft-versus-host disease, or an inflammatory bowel disease, such as Crohn's disease or ulcerative colitis, or cancer, such as prostate cancer, colorectal cancer, breast cancer, lung cancer, liver cancer or gastric cancer.

In certain embodiments, the invention provides the above pharmaceutical composition, wherein the amount of the bacterial strain is from about $1 \times 10^3$ to about $1 \times 10^{11}$ colony forming units per gram with respect to a weight of the composition.

In certain embodiments, the invention provides the above pharmaceutical composition, wherein the composition is administered at a dose of 1 g, 3 g, 5 g or 10 g.

In certain embodiments, the invention provides the above pharmaceutical composition, wherein the composition is administered by a method selected from the group consisting of oral, rectal, subcutaneous, nasal, buccal, and sublingual.

In certain embodiments, the invention provides the above pharmaceutical composition, comprising a carrier selected from the group consisting of lactose, starch, glucose, methyl cellulose, magnesium stearate, mannitol and sorbitol.

In certain embodiments, the invention provides the above pharmaceutical composition, comprising a diluent selected from the group consisting of ethanol, glycerol and water.

In certain embodiments, the invention provides the above pharmaceutical composition, comprising an excipient selected from the group consisting of starch, gelatin, glucose, anhydrous lactose, free-flow lactose, beta-lactose, corn sweetener, acacia, tragacanth, sodium alginate, carboxymethyl cellulose, polyethylene glycol, sodium oleate, sodium stearate, magnesium stearate, sodium benzoate, sodium acetate and sodium chloride.

In certain embodiments, the invention provides the above pharmaceutical composition, further comprising at least one of a preservative, an antioxidant and a stabilizer.

In certain embodiments, the invention provides the above pharmaceutical composition, comprising a preservative selected from the group consisting of sodium benzoate, sorbic acid and esters of p-hydroxybenzoic acid.

In certain embodiments, the invention provides the above pharmaceutical composition, wherein said bacterial strain is lyophilised.

In certain embodiments, the invention provides the above pharmaceutical composition, wherein when the composition is stored in a sealed container at about 4∘C or about 25∘C and the container is placed in an atmosphere having 50% relative humidity, at least 80% of the bacterial strain as measured in colony forming units, remains after a period of at least about: 1 month, 3 months, 6 months, 1 year, 1.5 years, 2 years, 2.5 years or 3 years.

*Culturing methods*

The bacterial strains for use in the present invention can be cultured using standard microbiology techniques as detailed in, for example, references [42,,44].

The solid or liquid medium used for culture may be YCFA agar or YCFA medium. YCFA medium may include (per 100ml, approximate values): Casitone (1.0 g), yeast extract (0.25 g), $NaHCO_3$ (0.4 g), cysteine (0.1 g), $K_2HPO_4$ (0.045 g), $KH_2PO_4$ (0.045 g), NaCl (0.09 g), $(NH_4)_2SO_4$ (0.09 g), $MgSO_4 \cdot 7H_2O$ (0.009 g), $CaCl_2$ (0.009 g), resazurin (0.1 mg), hemin (1 mg), biotin (1 μg), cobalamin (1 μg), *p*-aminobenzoic acid (3 μg), folic acid (5 μg), and pyridoxamine (15 μg).

*Bacterial strains for use in vaccine compositions*

The inventors have identified that the bacterial strains of the invention are useful for treating or preventing diseases or conditions mediated by HDAC. This is likely to be a result of the effect that the bacterial strains of the invention have on the host immune system. Therefore, the compositions of the invention may also be useful for preventing diseases or conditions mediated by HDAC, when administered as vaccine compositions. In certain such embodiments, the bacterial strains of the invention may be killed, inactivated or attenuated. In certain such embodiments, the compositions may comprise a vaccine adjuvant. In certain embodiments, the compositions are for administration via injection, such as via subcutaneous injection.

*General*

The practice of the present invention will employ, unless otherwise indicated, conventional methods of chemistry, biochemistry, molecular biology, immunology and pharmacology, within the skill of the art. Such techniques are explained fully in the literature. See, *e.g.,* references [45] and [46,52], *etc.*

The term "comprising" encompasses "including" as well as "consisting" e.g. a composition "comprising" X may consist exclusively of X or may include something additional e.g. X + Y.

The term "about" in relation to a numerical value *x* is optional and means, for example, $x \pm 10\%$.

The word "substantially" does not exclude "completely" *e.g.* a composition which is "substantially free" from Y may be completely free from Y. Where necessary, the word "substantially" may be omitted from the definition of the invention.

References to a percentage sequence identity between two nucleotide sequences means that, when aligned, that percentage of nucleotides are the same in comparing the two sequences. This alignment and the percent homology or sequence identity can be determined using software programs known in the art, for example those described in section 7.7.18 of ref. [53]. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. [54].

Unless specifically stated, a process or method comprising numerous steps may comprise additional steps at the beginning or end of the method, or may comprise additional intervening steps. Also, steps may be combined, omitted or performed in an alternative order, if appropriate.

Various embodiments of the invention are described herein. It will be appreciated that the features specified in each embodiment may be combined with other specified features, to provide further embodiments. In particular, embodiments highlighted herein as being suitable, typical or preferred may be combined with each other (except when they are mutually exclusive).

## MODES FOR CARRYING OUT THE INVENTION

### *Example 1* - *Efficacy of bacteria on histone deacetylase activity*

*Summary*

The ability of compositions comprising bacterial strains according to the invention to alter histone deacetylase activity was investigated. Dysregulation of histone deacetylase has been implicated in the pathogenesis associated with inflammatory and autoimmune disorders and cancer.

*Material and Methods*

*Bacterial strain*

*Megasphaera massiliensis* MRx0029

*Cell line*

The cell line HT-29 was used because histone deacetylase is present.

*Method*

Cell free supernatants of stationary phase bacterial cultures were isolated by centrifugation and filtering in a 0.22 uM filter. HT-29 cells were used 3 days' post confluence and stepped down in 1 mL DTS 24 hours prior to commencement of the experiment. The HT-29 cells were challenged with 10 % cell free supernatant diluted in DTS and was is left to incubate for 48 hours. Nuclease proteins were then extracted using the Sigma Aldrich Nuclease extraction kit and samples were snap frozen prior to HDAC activity measurement. HDAC activity was assessed fluorometrically using the Sigma Aldrich (UK) kit.

*Results*

The results of the experiments are shown in Figure 1A. Figure 1A shows that MRX0029 is able reduce the levels of histone deacetylase activity.

### *Example 2* - *Further analysis of the efficacy of bacteria on histone deacetylase activity*

*Introduction*

The inventors sought to investigate the effectiveness of MRX0029 and its metabolites on HDAC inhibition.

*Material and Methods*

*Bacterial culture and cell-free supernatant collection*

Pure cultures of MRX0029 bacteria were grown anaerobically in YCFA broth until they reached their stationary growth phase. Cultures were centrifuged at 5,000 x g for 5 minutes and the cell-free supernatant (CFS) was filtered using a 0.2 μM filter (Millipore, UK). 1 mL aliquots of the CFS were stored at -80 °C until use. Sodium butyrate, hexanoic and valeric acid were obtained from Sigma Aldrich (UK) and suspensions were prepared in YCFA broth.

*SCFA and MCFA quantification of bacterial supernatants*

Short chain fatty acids (SCFAs) and medium chain fatty acids (MCFAs) from bacterial supernatants were analysed and quantified by MS Omics APS as follows. Samples were acidified using hydrochloride acid, and deuterium labelled internal standards where added. All samples were analyzed in a randomized order. Analysis was performed using a high polarity column (Zebron™ ZB-FFAP, GC Cap. Column 30 m x 0.25 mm x 0.25 μm) installed in a GC (7890B, Agilent) coupled with a quadropole detector (59977B, Agilent). The system was controlled by ChemStation (Agilent). Raw data was converted to netCDF format using Chemstation (Agilent), before the data was imported and processed in Matlab R2014b (Mathworks, Inc.) using the PARADISe software described by Johnsen, 2017, J Chromatogr A, 1503, 57-64.

*Specific HDAC activity analysis*

Specific HDAC inhibition activity was analysed for HDAC1, 2, 3, 4, 5, 6, 9 using fluorogenic assay kits for each type of HDAC (BPS Bioscience, CA). Assays were conducted according to manufacturer's instructions and each sample were performed in replicates. Cell free supernatants were diluted 1 in 10 and exposed to specific HDAC proteins provided in the kit to maintain consistency between methods.

*Results*

*MRx0029 produces the HDAC inhibiting metabolites butyrate and valeric acid*

MRx0029 supernatant showed strong HDAC inhibition and was found to produce valeric acid and hexanoic acid at mean concentrations of 5.08 mM and 1.60 mM, respectively (Figure 16A and C) (Figure 1C).

To investigate which metabolites were responsible for the strain-induced HDAC inhibition, different concentrations of hexanoic acid, valeric acid and sodium butyrate were measured for their HDAC inhibition on whole HT-29 cells and on HT-29 cell lysate. The results in Fig. IB show significant ($P<0.05$) inhibition of HDAC activity by sodium butyrate on whole cells as well as on the cell lysate, while hexanoic acid did show significant inhibitory activity. Valeric acid inhibited total HDAC activity (* ($p<0.05$), ** ($p<0.005$), *** ($P<0.001$), **** ($p<0.0001$)).

*Potent total HDAC inhibitors investigated target class I HDACs.*

The specific HDAC inhibition profile of the test bacteria strain was investigated. Specific HDAC inhibition assays (BPS Bioscience, CA) were carried out for Class I HDACs. The ability of the bacterial strain to inhibit HDAC enzymes was analysed. The results (Figure 2) demonstrate that MRX0029 is a potent inhibitor of Class 1 HDAC enzymes (HDAC1, 2 and 3), in particular HDAC2.

*Discussion*

The strain with HDAC inhibitory activity produced significant amounts of valeric acid and hexanoic acid as well as significant amounts of sodium butyrate (Figure 1C). When tested as pure substances, valeric acid and sodium butyrate resulted in significant HDAC inhibition (Figures IB and 2) ($p<0.0001$).

Interestingly, the results for specific HDAC activity show that the tested strain is a potent inhibitor of Class I HDACs, and particularly HDAC2 (Figure 2). Class I HDACs (HDAC1, 2, 3 and 8) reside in the nucleus and are ubiquitously expressed in several human cell types. HDACs 1-3 share more than 50% homology, but have distinct structures and cellular functions [55]. They are primarily involved in cell survival, proliferation and differentiation, and thus there inhibition may be useful is wide array of diseases [56,57,58,59,60] . These data show that the compositions of the invention may be useful for treating diseases mediated by HDAC.

***Example 3*** - ***Efficacy of bacterial inocula to reduce IL-6 secretion.***

*Summary*

Activation of proinflammatory cytokines has been associated with damage in inflammatory disease. Lipopolysaccharide (LPS) is a known stimulator of the proinflammatory cytokine IL-6. Human glioblastoma astrocytoma cells were treated with compositions comprising bacterial strains according to the invention in combination with LPS to observe their ability to modulate the levels of IL-6.

*Material and Methods*

*Bacterial strain*

*Megasphaera massiliensis* MRx0029

*Cell line*

MG U373 is a human glioblastoma astrocytoma derived from a malignant tumour and were purchased from Sigma-Aldrich (cat n. 08061901-1VL). MG U373 human glioblastoma astrocytoma cells were grown in MEM (Sigma Aldrich, cat n. M-2279) supplemented with 10% FBS, 1% Pen Strep, 4mM L-Glut, IX MEM Non essential Amino Acid solution and IX Sodium Pyruvate.

*Method*

Once grown the MG U373 cells were plated on 24-well plate at 100,000 cells/well. The cells were treated with LPS (1ug/mL) alone or with 10% of bacteria supernatant from MRx0029 for 24h. A control was also performed where the cells were incubated in untreated media. Afterwards the cell free supernatants were collected, centrifuged at 10,000g for 3min at 4°C. IL-6 was measured using the Human IL-6 ELISA Kit from Peprotech (cat n.#900-K16) according to manufacturer instructions.

*Results*

The results of these experiments are shown in Figure 3. Treatment of cells with LPS and the bacteria strain led to a decrease in the level of IL-6 secreted.

***Example 4* - *Efficacy of bacterial inocula to reduce NF-κB activation***

*Summary*

Activation of the NF-κB promoter leads to the production of proinflammatory cytokines including IL-1β, IL-1α, IL-18, TNFα and IL-6. The NF-κB promoter can be activated by α-synuclein and LPS by stimulating the TLR4 ligand. The ability of compositions comprising bacterial strains according to the invention to inhibit the activation of the NF-κB promoter was investigated.

*Material and Methods*

*Bacterial strain*

*Megasphaera massiliensis* MRx0029

*Cell line*

Human HEK blue TLR4 were purchased from InvivoGen (cat n. hkb-htlr4). Human HEK blue TLR4 were grown in DMEM high glucose (Sigma Aldrich, cat n. D-6171) supplemented with 10% FBS, 1% Pen Strep, 4mM L-Glut, Normocin and IX HEK Blue selection solution.

*Method*

Once grown the Human HEK blue cells were plated in 96 well plates at 25,000 cells/well in 4 replicates. Cells were treated with LPS (10 ng/mL, from Salmonella enterica serotype Typhimurium, Sigma Aldrich, cat n. L6143) alone or with 10% of bacteria supernatant from MRx0029 for 22h. The cells were subsequently spun down and 20ul of the supernatant was mixed with 200ul of Quanti Blue reagent (InvivoGen, cat n. rep-qb2), incubated for 2 h and absorbance read at 655nm.

*Results*

The results of these experiments are shown in Figure 4. Figure 4 shows that the activation of the NFκB promoter by LPS is inhibited by MRx0029.

***Example 5** - **Efficacy of bacterial inocula to alter antioxidant capacity***

*Summary*

The ability of compositions comprising bacterial strains according to the invention to alter the antioxidant capacity. The antioxidant capacity of the bacterial strain was established using the well-known ABTS (2,2'-azino-bis(3-ethylbenzothiazoline-6-sulphonic acid)) assay.

*Bacterial strain*

*Megasphaera massiliensis* MRx0029

*Method*

Bacterial cells ($10^6$ or greater) were collected and centrifuged. They were resuspended in assay buffer (using three times the pellet volume). The suspension was sonicated on ice for 5 minutes and then spun down at 12,000 x g for 10 minutes. The supernatant was removed and measured using the ABTS assay kit produced by Sigma Aldrich (code CS0790), in accordance with manufacturer's instructions.

*Results*

The results of these experiments are shown in Figure 6. Figure 6 shows that MRx0029 has an antioxidant capacity of approximately 2mM compared to Trolox.

***Example 6** - **Efficacy of bacterial inocula to alter lipid peroxidation levels***

*Summary*

The ability of compositions comprising bacterial strains according to the invention to alter lipid peroxidation levels was investigated. The thiobarbituric reactive substances assay (TBARs) was used to measure the by-products of lipid peroxidation.

*Material and Methods*

*Bacterial strain*

*Megasphaera massiliensis* MRx0029

*Method*

Bacterial cells ($10^6$ or greater) were collected and centrifuged, a wash step was performed with isotonic saline before the pellet was re-suspensed in potassium chloride assay buffer. The suspension was sonicated on ice for 10 minutes and then spun down at 10,000 x g for 10 minutes. The supernatant was removed and the level of lipid peroxidation evaluated using the thiobarbituric reactive substances assay.

*Results*

The results of the experiments are shown in Figure 6. Figure 6 shows that MRx029 is able to inhibit lipid peroxidation by approximately 20 %, which is a higher antioxidant capacity than the positive control, butylated hydroxytoluene (1% w/v).

***Example 7** - **Level of indole production in bacteria***

*Summary*

The ability of the bacteria of the invention to produce indole was investigated. Indole has been implicated in attenuating inflammation and oxidative stress.

*Material and Methods*

*Bacterial strain*

*Megasphaera massiliensis* MRx0029

ATCC 11775 is a bacterial reference strain that is known to produce indole.

*Method*

Intact bacterial cells in stationary phase were incubated with 6mM Tryptophan for 48 hours. Bacterial species which possess the enzyme tryptophanase will utilise tryptophan as a substrate to produce indole. Following the 48 hour incubation period, the supernatant was removed and added to Kovac's reagent for quantification of indole. Standards, stock solutions and reagents were prepared using standardised methods validated in-house.

*Results*

The results of the experiments are shown in Figure 7. Figure 7 shows that MRx0029 has the capacity to produce indole from tryptophan, at concentrations of approximately 0.2mM.

**Example 8** - **Efficacy of MRx0029 in reducing leukocyte infiltration in the ileum**

*1. STUDY OBJECTIVE*

The objective of this study was to determine the prophylactic efficacy of MRX029 in a DSS-induced colitis mouse model, upon repeated oral administration.

*2. MATERIALS AND METHODS*

*2.1. Test substances*

- **2.1.1 Test substances**

YCFA was readily prepared Hungate tubes containing prereduced YCFA

MRx0029 was prepared in the form of frozen glycerol stocks.

- **2.1. 2 Reference substances**

Tacrolimus - (Sigma PHR-1809 - lot LRAA8723)

Valproic Acid (Arrow Generiques - 200 mg/mL - Batch 10.15 - expiry date 11/2020)

- **2.1.3 Additional reagents**

DSS (36,000-50,000 Da) from MP Biomedicals, Cat. No: 0216011090

PBS (without Ca/Mg) from Gibco, Cat. No: 14190-094

Tween 80 from Sigma, Cat. No: P4780-100ML

o Sterile 0.9% NaCl from Lavoisier Cat. No: CIP 3400 963 340 763

o Sterile distilled water from Aguettant Cat. No: 600499

- **2.1. 4 Reference substances preparation**

Tacrolimus was prepared daily in sterile 1% Tween 80, 0.9% NaCl at a concentration of 0.1 mg/mL.
Valproic acid was prepared daily in sterile distilled water at a concentration of 20 mg/mL (dilution 1/ 10).

- **2.1. 5 Bacteria preculture**

Bacterial precultures were prepared using the following protocol using sterile techniques. One glycerol stock per strain stored at -80 °C was thawed completely and briefly vortex mixed. Only thawed stocks in which the colour of the media was light brown/yellow were used. If the colour of the thawed medium was darker or blueish the glycerol stock was discarded.
Precultures were prepared by injecting 400 µL of the glycerol stock through the septum of a Hungate tube using a 1 mL syringe with a 0.8x40 mm needle. The tube was mixed by inversion and a second Hungate tube was prepared in duplicate. The OD6oo of both inoculated Hungate tubes at t= 0 was measured (a non-inoculated Hungate tube was used as a blank). The Hungate tubes were then incubated at 37 °C for 24 h and the OD measured periodically.

- **2.1.6 Bacteria main culture for mouse administration**

**1** ml of the pre-culture with the higher OD6oo was used to inoculate fresh Hungate tube. The tube was mixed by inversion. Duplicate inoculates were prepared and cultured as described above. The $OD_{600}$ was measured as described above and measured periodically over the course of 16 hrs. The Hungate tube with the higher OD6oo, at the end point was used for dosing.

*2.2. Treatment doses*

Tacrolimus was dosed at 1 mg/kg/day

Valproic acid was dosed at 200 mg/kg/day

PBS, YCFA and bacterial cultures were dosed at 200 µL/day

*2.3. Routes of administration*

PBS, YCFA and live bacteria were be daily administered per os (PO) under a fixed volume of 200 µL/mouse

Tacrolimus will be daily administered subcutaneously (SC) under a volume of 10 mL/kg

Valproic acid will be daily administered per os (PO) under a volume of 10 mL/kg

*2.4. Animals*

Each of **sixty three,** 6 week old healthy male C57BL/6J mice were obtained from Charles River (France) and individually identified and labelled with a specific code. Each treatment group (9 animals/group) were housed in three different cages.
Animals were maintained in SPF health status according to the FELASA guidelines, and animal housing and experimental procedures were realized according to the French and European Regulations and NRC Guide for the Care and Use of Laboratory Animals. • The viability and behavior of animals was recorded every day.

- **2.4.1. Housing conditions**

Animals were maintained in housing rooms under controlled environmental conditions: Temperature: 22 ± 2°C; Humidity 55 ± 10%; F9 Filtered air; Photoperiod (12h light/12h dark); with more than 15 air exchanges per hour with no recirculation.

Animal enclosures were provided adequate space with bedding material, food and water, environmental and social enrichment (group housing) as described:

- Polycarbonate Eurostandard Type IIL or III filtered top cages
- Poplar bedding (TOPLIT SELECT FINE, JRS®, Germany),
- A04 controlled standard maintenance diet (Safe®, France),
- Tap water,
- Environmental enrichment*
- Sizzlenest and small wood stick from BioServices - Netherlands
- Mice igloo from Plexx - Netherlands.

*3. EXPERIMENTAL DESIGN AND TREATMENTS*

*3.1. In vivo studies*

Animal randomization was performed before treatment group allocation based on body weights. Specific measures were taken throughout the study to prevent cross-contamination. For example, when handling the animals, gloves were changed and sprayed with 70% ethanol solution between each treatment cage to minimize any risk of contamination. Tissue harvesting was also performed under aseptic conditions. Briefly, prior to sample harvesting, all tools, materials and the harvesting area were sprayed with 70% ethanol.

The following specific measures were also taken to prevent circadian effects and optimize group randomization and avoid false positive / negative:

- Treatments were administered at random and alternated daily to prevent the same group being treated at the same time each day
- Animal manipulation and handling was carried out at random, alternating each day, to prevent the same animals being handled at the same time points
- Groups were randomized at each time point when acquiring samples

*3.2. Dosing of animals with bacteria main culture*

Animals were dosed by extracting the dosing aliquot from the Hungate tube using a syringe and a 0.8x40 mm needle injected through the septum . The Hungate tube was mixed by inversion before the dosing aliquot was extracted. The first 50-100 μL through the gavage needle and each mouse was dose with 200 μL of culture by oral gavage

*3.3. In vivo study*

The following table indicates the study groups.

| Group | No. Animals | DSS (Days 0 to 7) | Treatment (Day -7 to Day 6) | Route | Sacrificed |
|---|---|---|---|---|---|
| 1 | 9 | - | PBS | PO | Day 7 |
| 2 | 9 | - | YCFA | PO | Day 7 |
| 3 | 9 | 3% | PBS | PO | Day 7 |
| 4 | 9 | 3% | YCFA | PO | Day 7 |

(continued)

| Group | No. Animals | DSS (Days 0 to 7) | Treatment (Day -7 to Day 6) | Route | Sacrificed |
|---|---|---|---|---|---|
| 5 | 9 | 3% | MRx0029 in YCFA | PO | Day 7 |
| 6 | 9 | 3% | valproic acid | PO | Day 7 |
| 7 | 9 | 3% | PBS (Day -7 to Day -1) Tacrolimus (Days 0 to 6) | PO | Day 7 |

Treatments were carried out as follows:
Days -7 to Day -1: Treatment with bacteria and reference substances according the treatment table above

- Once daily oral administration of PBS, YCF A or Bacteria - 200 μL per mouse
- Once daily oral administration of Valproic acid at 200 mg/kg/day under a volume of 10 mL/kg From Day O to Day +7 : DSS administration
- Administration of 3% DSS in the drinking water

From Day O to Day +6 : Treatment with bacteria and reference substances

- Once daily oral administration of PBS, YCFA or Bacteria - 200 μL per mouse
- Once daily oral administration of Valproic acid-200 mg/kg in sterile distilled water - 10mL/kg
- Once daily SC administration of Tacrolimus - 1 mg/kg in sterile 1% Tween80, 0.9% NaCl - 10 mL/kg

Day +7 : Sacrifice of all groups and tissue harvesting

- Euthanasia of animals was performed under gas anesthesia (Isoflurane) followed by exsanguination and cervical dislocation. Euthanasia methods used are those recommended for mice and rats by European directive 20 I 0/63/CE and the procedure describing euthanasia methods was approved by IACUC.
- Laparotomy and ileum harvesting, just upstream of the caecum (from 0.5 cm and on) - all tissue harvested from exactly the same area between all mice:
    - 1.5 cm swiss-rolled ileum was harvested for histology, the closest to the caecum

*3.4. Histology*

Ileum Swiss-rolls specimens were embedded in paraffin and sections of 5 μm thickness were cut and mounted on SuperFrost Ultra plus glass slides. HP (Hematoxylin-Phloxin) staining & AB-PAS (Alcian Blue - Periodic Acid Schiff) staining were performed to visualize histomorphologic changes. A scoring based on edema, erosion, loss of crypts/goblet cells and infiltrates was established on each animal, using the criteria in the following table:

| Score | Leucocytic cell clusters (in lamina propria) | Epithelial cell damage - Erosion | Depletion of Goblet cells | Edema |
|---|---|---|---|---|
| 0 | None | None | None | None |
| 1 | Minimal < 10% | Minimal | Minimal < 20% | Minimal |
| 2 | Mild 10.25% | Mild | Mi d 21-35% | Mild |
| 3 | Moderate 26-50% | Moderate | Moderate 36-50% | Moderate |
| 4 | Marked > 50% | Marked | Marked > 50% | Marked |

***4. RESULTS***

The ileum histology scores of each of the animals in each of the seven treatment groups are shown in the table below.

| Group # | Cage ID | Mouse ID | Leucocytes Infiltration | Erosion (epithelial cell damage) | Depletion of goblet cells | Edema | COLITIS SCORING (sum) | COLITIS SCORING mean ± SEM |
|---|---|---|---|---|---|---|---|---|
| **PBS w/o DSS** | cage 01 | 1 | 0 | 0 | 0 | 0 | 0 | |
| GROUP 1 | | 2 | 1 | 0 | 0 | 0 | **1** | **0.63** |
| | | 3 | 0 | 0 | 0 | 0 | **0** | ± |
| | cage 02 | 4 | 0 | 0 | 0 | 0 | **0** | 0.11 |
| | | 5 | 1 | 1 | 0 | 0 | 2 | |
| | | 6 | 2 | 0 | 0 | 0 | **2** | |
| | cage 03 | 7 | 0 | 0 | 0 | 0 | **0** | |
| | | 8 | | | | | | |
| | | 9 | 0 | 0 | 0 | 0 | **0** | |
| **YCFA w/o DSS** | cage 04 | 10 | 0 | 0 | 0 | 0 | **0** | |
| GROUP 2 | | 11 | 0 | 0 | 0 | 0 | **0** | **0.50** |
| | | 12 | 0 | 0 | 0 | 0 | **0** | + |
| | cage 05 | 13 | 0 | 0 | 0 | 0 | **0** | 0.12 |
| | | 14 | 2 | 0 | 0 | 0 | **2** | |
| | | 15 | 1 | 1 | 0 | 0 | **2** | |
| | cage 06 | 16 | 0 | 0 | 0 | 0 | **0** | |
| | | 17 | 0 | 0 | 0 | 0 | **0** | |
| | | 18 | | | | | | |
| **PBS** | cage 07 | 19 | 1 | 0 | 0 | 0 | **1** | |
| GROUP 3 | | 20 | 1 | 0 | 0 | 0 | **1** | **1.22** |
| | | 21 | 2 | 0 | 0 | 0 | **2** | ± |
| | cage 08 | 22 | 1 | 0 | 0 | 0 | **1** | 0.09 |
| | | 23 | 2 | 0 | 0 | 0 | **2** | n=9 |
| | | 24 | 0 | 0 | 0 | 0 | **0** | |

(continued)

| Group # | Cage ID | Mouse ID | Leucocytes Infiltration | Erosion (epithelial cell damage) | Depletion of goblet cells | Edema | COLITIS SCORING (sum) | COLITIS SCORING mean ± SEM |
|---|---|---|---|---|---|---|---|---|
| | cage 09 | 25 | 0 | 0 | 0 | 0 | **0** | |
| | | 26 | 2 | 0 | 0 | 0 | **2** | |
| | | 27 | 2 | 0 | 0 | 0 | **2** | |
| **YCFA** | cage 10 | 28 | 0 | 1 | 0 | 0 | **1** | |
| | | 29 | 1 | 0 | 0 | 0 | **1** | **1.33** |
| | | 30 | 1 | 0 | 0 | 0 | **1** | ± |
| GROUP 4 | cage 11 | 31 | 1 | 0 | 0 | 0 | **1** | 0.12 |
| | | 32 | 2 | 1 | 0 | 0 | **3** | n=9 |
| | | 33 | 2 | 0 | 0 | 0 | **2** | |
| | cage 12 | 34 | 0 | 0 | 0 | 0 | **0** | |
| | | 35 | 0 | 0 | 0 | 0 | **0** | |
| | | 36 | 2 | 1 | 0 | 0 | 3 | |
| **MRx0029** | cage 13 | 37 | 1 | 0 | 0 | 0 | 1 | |
| | | 38 | 1 | 1 | 0 | 0 | 2 | 0.89 |
| | | 39 | 1 | 0 | 0 | 0 | 1 | ± |
| GROUP 6 | cage 14 | 40 | 1 | 0 | 0 | 0 | 1 | 0.07 |
| | | 41 | 1 | 0 | 0 | 0 | 1 | n=9 |
| | | 42 | 0 | 0 | 0 | 0 | 0 | |
| | cage 15 | 43 | 1 | 0 | 0 | 0 | 1 | |
| | | 44 | 1 | 0 | 0 | 0 | 1 | |
| | | 45 | 0 | 0 | 0 | 0 | 0 | |

(continued)

| Group # | Cage ID | Mouse ID | Leucocytes Infiltration | Erosion (epithelial cell damage) | Depletion of goblet cells | Edema | COLITIS SCORING (sum) | COLITIS SCORING mean ± SEM |
|---|---|---|---|---|---|---|---|---|
| **Valproic acid** 200mg/kg PO GROUP 6 | cage 16 | 46 | 2 | 0 | 0 | 0 | 2 | 0.67 + 0.10 n=9 |
| | | 47 | 1 | 0 | 0 | 0 | 1 | |
| | | 48 | 0 | 0 | 0 | 0 | 0 | |
| | cage 17 | 49 | 1 | 0 | 0 | 0 | 1 | |
| | | 50 | 2 | 0 | 0 | 0 | 2 | |
| | | 51 | 0 | 0 | 0 | 0 | 0 | |
| | cage 18 | 52 | 0 | 0 | 0 | 0 | 0 | |
| | | 53 | 0 | 0 | 0 | 0 | 0 | |
| | | 54 | 0 | 0 | 0 | 0 | 0 | |
| **Tacrolimus** 1mg/kg SC GROUP 7 | cage 19 | 55 | 0 | 0 | 0 | 0 | 0 | 0.44 ± 0.08 n=9 |
| | | 56 | 0 | 0 | 0 | 0 | 0 | |
| | | 57 | 0 | 0 | 0 | 0 | 0 | |
| | cage 20 | 58 | 2 | 0 | 0 | 0 | 2 | |
| | | 59 | 0 | 0 | 0 | 0 | 0 | |
| | | 60 | 1 | 0 | 0 | 0 | 1 | |
| | cage 21 | 61 | 1 | 0 | 0 | 0 | 1 | |
| | | 62 | 0 | 0 | 0 | 0 | 0 | |
| | | 63 | 0 | 0 | 0 | 0 | 0 | |

None of the treatment groups showed a reduction in the number of goblet cells or edema. No significant epithelial cell damage/erosion was detected.

The majority of animals in the vehicle only control DSS groups (Groups 3 and 4) showed a mild increase in leukocyte infiltration in comparison to non-diseased Groups 1 and 2. DSS animals treated with Tacrolimus, a known immunosuppressant, reduced leukocyte infiltration comparable to controls. Similar reductions were observed in the valproic acid and bacteria treatment Groups 5 and 6. This indicates that the bacteria is effective at reducing leukocyte infiltration in the ileum comparable to the known therapeutic Tacrolimus.

***Example 9* - *Stability testing***

A composition described herein containing at least one bacterial strain described herein is stored in a sealed container at 25°C or 4°C and the container is placed in an atmosphere having 30%, 40%, 50%, 60%, 70%, 75%, 80%, 90% or 95% relative humidity. After 1 month, 2 months, 3 months, 6 months, 1 year, 1.5 years, 2 years, 2.5 years or 3 years, at least 50%, 60%, 70%, 80% or 90% of the bacterial strain shall remain as measured in colony forming units determined by standard protocols.

***Example 10* - *Neurochemical production* - *metabolites in the brain***

*Background*

The level of neurochemical factors, neuropeptides and neurotransmitters that play a key role in neurological processes were measured during the *ex vivo* screening in brain tissue of mice fed with MRx0029.

*Methods*

Animals

BALBc (Envigo, UK) adult male mice were group housed under a 12 h light-dark cycle; standard rodent chow and water were available ad libitum. All experiments were performed in accordance with European guidelines following approval by University College Cork Animal Ethics Experimentation Committee. Animals were 8 weeks old at the start of the experiment.

Study Design

Animals were allowed to habituate to their holding room for one week after arrival into the animal unit. They receive oral gavage (200μL dose) of live biotherapeutics at a dose of 1 X $10^9$ CFU for 6 consecutive days between 15:00 and 17:00. On day 7, the animals were decapitated, and tissues are harvested for experimentation.

Tissue Collection

Animals were sacrificed in a random fashion regarding treatment and testing condition; sampling occurred between 9.00 a.m. and 1:00 p.m. Trunk blood was collected in potassium EDTA (Ethylene Diamine Tetra Acetic Acid) tubes and spun for 15 min at 4000 g. Plasma was isolated and stored at -80°C for further analysis. The brain was quickly excised, dissected and each brain region was snap-frozen on dry ice and stored at -80°C for further analysis.

Analysis

Neurochemical factor, neuropeptide and neurotransmitter concentrations were analysed by HPLC on samples from the brainstem. Briefly, brainstem tissue was sonicated in 500 μl of chilled mobile phase spiked with 4 ng/40 μl of N-Methyl 5-HT (Sigma Chemical Co., UK) as internal standard. The mobile phase contained 0.1 M citric acid, 5.6 mM octane-1-sulphonic acid (Sigma), 0.1 M sodium dihydrogen phosphate, 0.01 mM EDTA (Alkem/Reagecon, Cork) and 9% (v/v) methanol (Alkem/Reagecon) and was adjusted to pH 2.8 using 4 N sodium hydroxide (Alkem/Reagecon). Homogenates were then centrifuged for 15 min at 22,000 × g at 4 °C and 40 μl of the supernatant injected onto the HPLC system which consisted of a SCL 10-Avp system controller, LECD 6A electrochemical detector (Shimadzu), a LC-10AS pump, a CTO-10A oven, a SIL-10A autoinjector (with sample cooler maintained at 40 C) and an online Gastorr Degasser (ISS, UK). A reverse-phase column (Kinetex 2.6 u C18 100 × 4.6 mm, Phenomenex) maintained at 30 °C was employed in the separation (Flow rate 0.9 ml/min). The glassy carbon working electrode combined with an Ag/AgCl reference electrode (Shimdazu) operated a +0.8 V and the chromatograms generated were analyzed using Class-VP 5 software (Shimadzu). The neurotransmitters were identified by their characteristic retention times as determined by standard injections, which run at regular intervals during the sample analysis. The ratios of peak heights of analyte versus internal standard were measured and compared with standard injection. Results were expressed as ng of neurotransmitter per g fresh weight of tissue.

*Results - neurotransmitter production*

The results are shown in Figure 8, which shows that in brains of mice fed with MRx0029, noradrenaline (p=0.0507), serotonin and 5-HIAA levels were increased.

***Example 11* - *Tryptophan hydroxylase expression***

*Background*

Tryptophan hydroxylase is an enzyme involved in the production of serotonin. The inventors thus sought to investigate whether the *Megasphaera massiliensis* strain MRx0029 can induce the upregulated expression of the tryptophan hydroxylase genes TPH1 and TPH2 in neuron-like cells. This may explain how MRx0029 increases the level of serotonin *in vivo.*

*Material and Methods*

Neuroblastoma SH-SY5Y cells were grown in 50% MEM 50% nutrient mixture F-12 ham media supplemented with 2 mM L-glutamine, 10% heat-inactivated FBS, 100 U/ml penicillin and 100 μg/ml streptomycin. Cells were plated in 10 cm dishes at a density of $2\times10^6$ cells. After 24h rest, cells were treated in growth medium (containing 1% FBS) with 10% MRx0029 supernatant or $YCFA^+$, for 24h. Cells were next collected, and total RNA was isolated according to the RNeasy mini kit protocol (Qiagen). cDNA was made using the high capacity cDNA reverse transcription kit (Applied Biosystems). Primer sequences are shown in Table 1. Gene expression was measured by qPCR. B-actin was used as internal control. Fold-change was calculated according to the 2^(-ΔΔct) method. A second set of similar experiments were conducted, except cells were plated in a six-well dish at a density of $0.5\times10^6$ cells/well. After 24h rest, cells were treated in growth medium (containing 1% FBS) with 5% bacterial supernatants or $YCFA^+$, for 72h. Total RNA was analysed as described above.

Controls where cells were either left untreated or incubated for an equivalent time in $YCFA^+$ medium were performed alongside. $YCFA^+$ medium has the following composition:

| | |
|---|---|
| Bacto casitione | 1.0 g |
| Yeast extract | 0.25 g |
| Sodium hydrogen carbonate | 0.4 g |
| Glucose | 0.2 g |
| Cellobiose | 0.2 g |
| Soluble starch | 0.2 g |
| Mineral solution 1 | 15 ml |
| Mineral solution 2 | 15 ml |
| SCFA solution | 0.31 ml |
| Haemin solution | 1 ml |
| Vitamin solution 1 | 100 μl |
| Vitamin solution 2 | 100 μl |
| Resazurin solution | 0.1 ml |
| Cysteine | 0.1 g |
| d. H2O to a total volume of: | 100 m |

**Mineral solution 1:** $K_2HPO_4$ -3.0 g; d.$H_2O$ to a total volume of 11

**Mineral solution 2:** $KH_2PO_4$ -3.0 g; $(NH_4)_2SO_4$ - 6.0 g; NaCl-6.0 g; $MgSO_4$ - 0.6 g; $CaCl_2$ - 0.6 g; d. H2O to a total volume of 1 1

**Resazurin solution:** 0.1% powdered resazurin in 100 ml distilled water.

**Short chain fatty acid solution:** Acetic acid -17 ml; Propionic acid-6 ml; n-Valeric acid-1 ml; IsoValeric acid-1 ml; Iso-Butyric acid- 1 ml

**Haemin solution:** KOH-0.28 g Ethanol 95%-25 ml; Haemin-100 mg; d. $H_2O$ to a total volume of 100 ml

**Vitamin solution 1:** Biotin-1 mg; Cobalamin-1 mg; p-Aminobenzoic acid-3 mg; Folic acid-5 mg; Pyridoxamine-15 mg; d. $H_2O$ to a total volume of 100 ml

**Vitamin solution 2:** Thiamine-5 mg; Riboflavin-5 mg; d. $H_2O$ to a total volume of 100 ml

*Results*

The results displayed in Figure 9 show that when cells are incubated with 10% MRx0029 bacterial cell-free supernatant for 24 h, the level of expression of TPH1 increases 5-fold, relative to untreated or $YCFA^+$-treated controls. The level of expression of TPH2 also increases 30-fold relative to untreated controls.

The results displayed in Figure 10 show that when cells are incubated with 5% MRx0029 bacterial cell-free supernatant for 72 h, the level of expression of TPH1 increases 5-fold, relative to untreated or $YCFA^+$-treated controls. The level of expression of TPH2 also increases 30-fold relative to untreated controls.

***Example 12* - *Serotonin transporter expression***

*Background*

The SLC6A4 gene encodes serotonin transporter. Serotonin transporter is a biomarker of differentiated serotonergic neurons. Serotonin transporter is also expressed by epithelial cells lining the intestines and removes serotonin from the interstitial space. The inventors thus sought to determine whether a bacterial strain of the species *M.massilensis* could upregulate serotonergic markers in neuron-like cells.

*Material and Methods*

Identical sets of experiments were carried out as described in Example 2. Primer sequences for the SLC6A4 gene are shown in Table 2.

Table 2 - primer sequences for SLC6A4 and β-actin

| Gene | Forward | Reverse |
|---|---|---|
| SLC6A4 | AATCTGCCGATTTTCAAAG (SEQ ID NO:7) | GTGTTGTAGTAGGAAGCAATG (SEQ ID NO:8) |
| β-actin | GATCAAGATCATTGCTCCTC (SEQ ID NO:5) | TTGTCAAGAAAGGGTGTAAC (SEQ ID NO:6) |

*Results*

The results displayed in Figure 11 shows that when cells are incubated with 10% MRx0029 bacterial cell-free supernatant for 24 h, the expression of SLC6A4 is upregulated 3-fold, relative to untreated controls but there was no difference with YCFA+ treated cells. The results displayed in Figure 12 shows that when cells are incubated with 5% MRx0029 bacterial cell-free supernatant for 72 h, the expression of SLC6A4 is upregulated 3-fold, relative to untreated controls and about 2-fold relative to YCFA+ treated cells. These data indicate that the compositions of the invention may be effective for treating inflammatory bowel disease by increasing serotonin transporter expression and removing serotonin from the gastrointestinal tract.

***Example 13 - Tryptophan hydroxylase and serotonin transporter expression analysis in Caco2 cells***

*Introduction*

The majority of serotonin is produced in the gut. Gut serotonin is thought to play an important communicative role between the gut and the brain. Therefore, the inventors sought to determine whether MRx0029 could increase the expression of TPH1 and SLC6A4 in gut-like cells.

To this end, the inventors incubated differentiated Caco2 cells with MRx0029 bacterial cell-free supernatant.

Differentiated Caco2 cells form polarized apical/mucosal and basolateral/serosal membranes that are impermeable and are structurally and functionally similar to epithelial cells of the small intestine.

*Materials and Methods*

Caco2 cells seeded on 12 well plates and differentiated for 10 days; then they were serum-starved for 12 hours and subsequently exposed to 10% supernatant derived from stationary phase MRx0029 for 24h. Cells were collected, and total RNA was isolated according to the RNeasy mini kit protocol (Qiagen). cDNA was made using the high capacity cDNA reverse transcription kit (Applied Biosystems). Gene expression was measured by qPCR. β-actin was used as internal control. Fold change was calculated according to the 2^(-ΔΔct) method. Primer sequences are displayed below.

*Results*

The results displayed in Figure 13 shows that when differentiated Caco2 cells are incubated with 10% MRx0029 bacterial cell-free supernatant for 24 h, the expression of TPH1 is upregulated almost 3-fold, relative to untreated and YCFA$^+$-treated controls. The results displayed in Figure 14 shows that the incubation increases the expression of SLC6A4 more than 3-fold, relative to untreated controls. These data indicate that the compositions of the invention may be effective for treating inflammatory bowel disease by increasing serotonin transporter expression and removing serotonin from the gastrointestinal tract.

***Example 14 - GPR109α RNA expression in differentiated Caco-2 cells***

GPR109a is a G-protein coupled receptor expressed in the lumen-facing apical membrane of colonic and intestinal epithelial cells. GPR109a expression silencing is found in colon cancers cell lines, and the induction of its expression has been reported to induce tumour cell apoptosis in the presence of bacterial fermentation products such as butyrate [61]. GPR109a is also able to supress inflammation, and in particular colonic inflammation [62].

HT29mtx cells seeded on 12 well plates and differentiated for 10 days; then they were serum-starved for 12 hours and subsequently exposed to 10% supernatant derived from stationary phase bacteria for 24h. Cells were collected, and total RNA was isolated according to the RNeasy mini kit protocol (Qiagen). cDNA was made using the high capacity cDNA reverse transcription kit (Applied Biosystems). Gene expression was measured by qPCR. βactin was used as internal control. Fold change was calculated according to the 2^(-ΔΔct) method [63]. The sequences of the forward and reverse primers used are provided as SEQ ID NO: 2 and 3, respectively.

Differentiated Caco-2 form polarised apical/mucosal and basolateral/serosal membranes that are impermeable and are structurally and functionally similar to epithelial cells of the small intestine. Treatment of Caco-2 cells with MRx0029 elicited increased expression of GPR109a (Figure 18A). Also, Caco-2 treated with phorbol-12-myristate-13-acetate (PMA) supernatant exhibited greater expression of GPR109a RNA, than treatment with PMA alone (or PMA in YCFA medium) - see Figure 18B. Therefore, these data suggest that compositions of the invention may be useful in the treatment of cancers, especially metastatic cancers, in particular metastatic colorectal cancer or small bowel cancer such as small bowel adenocarcinoma. These data also suggest that compositions of the invention may effect such treatment through the mechanism of inducing apoptosis, as a result of GPR109a expression. These data also suggest that MRx0029 has anti-inflammatory activities and may be useful for the treatment of inflammatory disorders, and in particular inflammatory bowel disease.

***Example 15* - *metabolite analysis***

*Introduction*

The gut microbiota, with its immense diversity and metabolic capacity, represents a huge metabolic reservoir for production of a vast variety of molecules. The inventors sought to determine what short chain fatty acids and medium chain fatty acids are produced and consumed by the *M.massiliensis* strain NCIMB 42787 and other *M.massiliensis* strain identified herein as Ref 1, Ref 2 and Ref 3.

*Material and Methods*

*Bacterial culture and cell-free supernatant collection*

Pure cultures of bacteria were grown anaerobically in YCFA broth until they reached their stationary growth phase. Cultures were centrifuged at 5,000 x g for 5 minutes and the cell-free supernatant (CFS) was filtered using a 0.2

μM filter (Millipore, UK). 1 mL aliquots of the CFS were stored at -80 °C until use. Sodium butyrate, hexanoic and valeric acid were obtained from Sigma Aldrich (UK) and suspensions were prepared in YCFA broth.

*SCFA and MCFA quantification of bacterial supernatants*

Short chain fatty acids (SCFAs) and medium chain fatty acids (MCFAs) from bacterial supernatants were analysed and quantified by MS Omics APS as follows. Samples were acidified using hydrochloride acid, and deuterium labelled internal standards where added. All samples were analysed in a randomized order. Analysis was performed using a high polarity column (Zebron™ ZB-FFAP, GC Cap. Column 30 m x 0.25 mm x 0.25 μm) installed in a GC (7890B, Agilent) coupled with a quadropole detector (59977B, Agilent). The system was controlled by ChemStation (Agilent). Raw data was converted to netCDF format using Chemstation (Agilent), before the data was imported and processed in Matlab R2014b (Mathworks, Inc.) using the PARADISe software described in [64].

*Results*

As shown in Figures 15-17, strain 42787 produces valeric acid, butyrate and hexanoic acid and consumes propionate and acetate. The inventors also found other strains of the species *M.massiliensis* that produce comparable levels of valeric acid, hexanoic acid and butyrate and that consume similar amounts of acetate and propionate.

***Example 16 - suppression of enolase 2***

Figure 19 demonstrates that MRx0029 has a statistically-significant effect suppressing neuron specific enolase(NSE)/enolase 2. NSE is thought to support increased tumour cell metabolic demands, protect tumour cells from stressful conditions and promote their invasion and migration [65]. It is also implicated in progression of metastatic melanoma [66], survival and progression in small cell lung cancer [67], and prognosis of adenosqamous lung carcinoma [68]. Therefore, the compositions of the invention are expected to be effective for treating and preventing cancer, in particular, metastatic melanoma, small cell lung cancer and adenosqamous lung carcinoma.

In addition, enolase may have pro-inflammatory effects [69], so these data also indicate that the compositions of the invention may be useful for the treatment or prevention of autoimmune and inflammatory disorders.

***Example 17* - *Metabolite analysis***

Further to the data provided in Example 17, Figure 20 demonstrates what other short chain fatty acids are produced and consumed by the *M.massiliensis* strain NCIMB 42787 and other strains deposited under accession numbers NCIMB 43385, NCIMB 43388 and NCIMB 43389.

*M. massiliensis* strain NCIMB 42787 reduces formic acid while increasing levels of 2-methyl-propanoic and 3-methyl-propanoic acid (Figure 20). Therefore, strain NCIMB 42787 produces 2-methyl-propanoic and 3-methyl-propanoic acid and consumes formic acid. The inventors also found that other of the deposited strains produce comparable levels of 2-methyl-propanoic and 3-methyl-propanoic acid and consume similar amounts of formic acid.

***Example 18 - Downregulation of IL-6***

*Introduction*

Bacterial strains were investigated for their ability to reduce secretion of IL-6 by the astrocytoma cell line U373 in the presence of the immunostimulant LPS.

*Materials and methods*

Human glioblastoma astrocytoma cell line (U373), were maintained in 25ml MEME 4.5 g/L D-glucose supplemented with 10% heat-inactivated FBS, 4mM L-Glutamine, 100 U/ml penicillin, 100 μg/ml streptomycin and 5 μg/ml plasmocin, 1% Non-Essential Amino Acids, 1% Sodium Pyruvate (referred to as full growth media).

Cells were plated in 24-well plates at a density of 100,000 cells/well in 1ml of full growth media and left to rest at 37°C/5% $CO_2$ for 72h. On the day of the treatment, the media was removed from each well, cells were rinsed with 0.5 ml wash media (serum free MEME), 0.9ml stimulation media (MEME media containing 2% FBS) containing 1 μg/ml LPS was added to the appropriate wells and incubated at 37°C and 5% $CO_2$. After 1h pre-incubation, cells were removed from $CO_2$ incubator and treated with 100 μl bacteria supernatant. YCFA+ media was used as control. Cells were then incubated for a further 24h at 37°C/5% $CO_2$, after which cell-free supernatants were collected and spun down at 10,000g at 4°C for 3 min. Samples were aliquoted in 1.5 ml microtubes and stored in -80°C for hIL-6 ELISA.

*Results and conclusions*

Figure 21 demonstrates that *M.massiliensis* strain NCIMB 42787 causes a significant suppression of IL-6 secretion in U373 cells compared to the LPS and LPS media controls. The inventors also identified that all of the deposited strains triggered a significant reduction in IL-6 secretion.

***Example 19 - Suppression of NFκB activation***

*Introduction*

Bacterial strains were investigated for their ability to reduce activation of the NFκB-AP1 promoter in HEK-TLR4 cells.

*Materials and methods*

HEK293-Blue reporter cells stably expressing human TLR4 (HEK-TLR4), were cultured according to the manufacturer's instructions. Briefly, HEK-TLR4 cells were maintained in DMEM 4.5g/L D-glucose supplemented with 10% (v/v) heat-inactivated FBS, 4mM L-Glutamine, 100U/ml penicillin, 100 μg/ml streptomycin, 100 μg/ml normocin, 1x HEK-Blue selection media.

For the experiment, cells were washed with PBS, dissociated in PBS and collected in growth media. Cells were plated in 96-well plates at a density of 25,000 cells/well for HEK-TLR4. To evaluate the responsiveness, cells were treated with either 10 ng/ml LPS in the presence or absence of 10% (V/V) bacteria supernatants and incubated in a $CO_2$ incubator. Treatments proceeded for 22h at 37°C/5% $CO_2$, after which the detection of Secreted embryonic alkaline phosphatase (SEAP) activity from cell culture supernatant was performed using QUANTI-blue solution according to manufacturer's instructions. Briefly, 20 μl of cell free supernatants was collected and analysed for the presence of SEAP by mixing with 200 μl QUANTI-Blue detection media. After 2h incubation at 37°C, optical density was measured at 655nm on a microplate reader (iMark microplate, Bio-Rad).

*Results*

Figure 22 demonstrates that *M.massiliensis* strain NCIMB 42787 reduces activation of the NFκB promoter in the presence of LPS compared to the LPS media control. In addition, the inventors identified that other deposited strains showed a similar trend towards reduction in the activation of the NFκB promoter.

***Example 20* - *Suppression of enolase 2***

*Materials and methods*

Neuroblastoma cell line SH-SY5Y, were grown in 50% MEM and 50% Nutrient Mixture F-12 Ham media supplemented with 2 mM L-Glutamine, 10% heat-inactivated FBS, 100 U/ml penicillin and 100 μg/ml streptomycin. SH-SY5Y were plated in 6 well plates at a density of $0.5\times10^6$ cells. After 24h, cells were treated in differentiation medium (growth medium containing 1% FBS) with 10% bacterial supernatants or YCFA+ for 17h. Cells were collected, and total RNA was isolated according to the RNeasy mini kit protocol (Qiagen). cDNA was made using the High Capacity cDNA reverse transcription kit (Applied Biosystems). Gene expression was measured by qPCR. GAPDH was used as internal control. Fold change was calculated according to the $2^{(-\Delta\Delta ct)}$ method. Primer sets used are listed as SEQ ID NOs: 19, 20, 21 and 22.

*Results*

Figure 23 demonstrates *M. massiliensis* strain NCIMB 42787 has a statistically-significant effect of suppressing neuron specific enolase(NSE)/enolase 2. In addition, the inventors also found that the deposited strains trigger a statistically-significant reduction of Enolase 2 compared to the YCFA culture control. In particular, strains deposited under accession numbers NCIMB 43385, NCIMB 43388, NCIMB 43389, NCIMB 43386 and NCIMB 43387 caused a significant suppression of enolase 2.

*Conclusion*

Accordingly, in line with the comments in Example 16 above, the compositions of the invention, in certain embodiments comprising the exemplary deposited strains, are expected to be effective for treating and preventing cancer, in particular, metastatic melanoma, small cell lung cancer and adenosqamous lung carcinoma.

***Example 21 - Reduction of IL-6production in U373 glioblasoma astrocytoma cells***

*Introduction*

Stationary phase bacterial cell-free supernatants of the *M. massiliensis* strain deposited under accession number NCIMB 42787 were screened for capacity to induce an anti-inflammatory response in the U373 glioblastoma astrocytoma cell line after treatment with lipopolysaccharide (LPS).

*Materials and methods*

U373 is a human glioblastoma astrocytoma cell line. Cells (passage 20th-37th) were maintained in 25ml MEME supplemented with 10% heat-inactivated FBS, 4mM L-Glutamine, 100U/ml penicillin, 100μg/ml streptomycin and 5μg/ml plasmocin, 1% Non-Essential Amino Acids, 1% Sodium Pyruvate (referred to throughout as full growth media). Cells were plated in 24-well plates at a density of 100,000 cells/well in 1ml of full growth media and left to rest at 37°C and 5% $CO_2$ for 72h.

Isolation of BCFS

Bacterial cell-free supernatants (BCFS) were obtained from stationary phase cultures (inoculated from an overnight culture of a subbed colony from a streaked freezer stock) by centrifuging 10 ml of cultures at 5000xg for 5min and filtering using a 0.2μM filter (Millipore, UK). 1ml aliquots of the bacterial cell-free supernatants were stored at -80°C until use.

Treatment of the cells

On the day of the treatment, the media was removed from each well, cells were rinsed with 0.5ml wash media (serum free MEME), 0.9ml stimulation media (MEME media containing 2% FBS) containing 1μg/ml LPS was added to the appropriate wells and incubated at 37°C and 5% $CO_2$. Cells were pre-treated for 1h with LPS. Afterwards, cells were removed from $CO_2$ incubator and treated with 100μl *(i.e.* 10%) of stationary phase bacterial cell-free supernatants of NCIMB 42787.

Following each treatment, cells were incubated for 24h incubation at 37°C and 5% $CO_2$. Afterwards cell-free supernatants were collected and centrifuged at 10,000xg at 4°C for 3min. Samples were aliquoted in 1.5ml microtubes and stored in -80°C for hIL-6 ELISA.

Secretion of IL-6 was analysed using hIL-6 Standard ELISA Kits, according to the manufacturer's protocol in the cell-free supernatants from U373 cells treated as described above. Samples were measured at 405nm with correction wavelength set at 655nm on a microplate reader (iMark, Bio-Rad). Raw data were plotted and analysed using GraphPad Prism 7 software.

*Results*

NCIMB 42787 displayed a strong reduction of IL-6 secretion in U373 cells after treatment with LPS (about 50% reduction relative to the positive control) (see Figure 24A). Figure 24B demonstrates that in the presence of LPS, NCIMB 42787 causes a significant reduction in the secretion of IL-6 in U373 cells compared to the media control. NCIMB 42787 did not significantly increase the basal level of IL-6 compared to the media control.

***Example 22 - Modulation of cytokine secretion in HMC3 cells exposed to TNFα upon treatment with M. massiliensis strain NCIMB 42787***

*Introduction*

HMC3 cells were treated with TNFα, and secretion of IL-6 was measured upon treatment with stationary phase bacterial cell-free supernatants of NCIMB 42787.

*Materials and methods*

Human microglia HMC3 cells were grown in glutamine-supplemented EMEM media containing 15% heat inactivated FBS and 100U/ml penicillin and 100μg/ml streptomycin. HMC3 cells were plated in 24 well plates at a density of 50,000 cells/well. Cells were left in $CO_2$ incubator to rest for 48h. The cells were then washed in blank EMEM and pre-treated in 2% FBS growth media with 10ng/ml TNF-α for 1h. Thereafter 10% cell-free bacterial supernatants for NCIMB 42787 stationary growth cultures (isolated as described above) were added to TNF-α-treated and untreated wells and incubated in $CO_2$ incubator at 37°C for 24h. Cell-free supernatants were collected and centrifugated at 10,000xg for 3min and 4°C. Samples were aliquoted in 1.5ml microtubes and stored in -80°C for hIL-6 ELISA (performed as outlined above).

Statistical Analysis

Normally distributed data are presented as mean ± SEM; One-way Anova (Sidak's multiple comparison test) was used to analyse the data presented in this paper. A p value <0.05 was deemed significant in all cases.

*Results*

NCIMB 42787 significantly reduces IL-6 secretion in TNF-α-treated HMC3 cells (Figure 24C). Interestingly, this strain did not induce IL-6 secretion by these cells in the absence of stimulus (Figure 24C).

Accordingly, in certain embodiments, the compositions of the present invention reduce secretion of IL-6 in human microglial cells. Therefore, in certain embodiments, the compositions of the present invention are useful in the treatment of neuronal inflammation and brain inflammatory disorders.

***Example 23 - Inhibition of NF-κB promoter activation in HEK-TLR4 cells by M. massiliensis NCIMB 42787***

*Introduction*

To verify whether treatment with NCIMB 42787 would be able to interfere with NF-κB-Ap1 promoter activity induced by engagement of TLR4, HEK-TLR4 cells were treated with cell-free bacterial supernatants for NCIMB 42787 alone or in combination with LPS.

*Materials and methods*

HEK293-Blue reporter cells stably expressing human TLR4 (HEK-TLR4), were cultured according to the manufacturer's instructions. Briefly, HEK-TLR4 cells were maintained in DMEM 4.5g/L D-glucose supplemented with 10% (v/v) heat-inactivated FBS, 4 mM L-Glutamine, 100 U/ml penicillin, 100μg/ml streptomycin, 100μg/ml normocin, IX HEK-Blue selection media.

Briefly, cells were washed with PBS, dissociated in PBS and collected in growth media. Cells were plated in 96-well plates at a density of 25,000 cells/well. To evaluate the effect of bacteria strains on LPS inducing NF-κB promoter activation, cells were treated with 10ng/ml LPS in presence or absence of 10% supernatants (isolated as described above) and incubated in a $CO_2$ incubator. Treatments proceeded for 22h at 37°C and 5% CO, after which the detection of Secreted Embryonic Alkaline Phosphatase (SEAP) activity from cell culture supernatant was performed using QUANTI-blue solution according to manufacturer's instructions. Briefly, 20μl of cell-free supernatant was collected and analysed for the presence of SEAP by mixing with 200μl of sterile-filtered QUANTI-Blue detection media. After 2h incubation at 37°C, optical density was measured at 655nm on a microplate reader (iMark microplate, Bio-Rad).

Statistical Analysis

Normally distributed data are presented as mean ± SEM; One-way Anova (Sidak's multiple comparison test) was used to analyse the data presented in this paper. A p value <0.05 was deemed significant in all cases.

*Results and conclusion*

NCIMB 42787 significantly inhibited NF-κB-Ap1 promoter activation induced by LPS (Figure 24D). This strain did induce NF-κB-Ap1 promoter activation on its own.

A reduction in NF-κB promoter activation in the presence of an adjuvant would reduce the inflammatory responses triggered by the NF-κB cascade.

### *Example 24 - M. massiliensis strains display intrinsic antioxidant capacity*

*Introduction*

To capture the synergistic and redox interactions among the different molecules present in the NCIMB 42787 supernatant, three biochemical assays aimed at characterising this strains antioxidant potential were used: the indole production assay, the total radical-trapping antioxidant parameter (TRAP) using the 2,2-diphenyl-1-picrylhydrazyl (DPPH) assay and the Trolox equivalent antioxidant capacity (TEAC) assay.

Indole derivatives have antioxidant and cytoprotective activity. The indole test is used to determine the ability of an organism to convert the amino acid tryptophan to form indole. The free-radical scavenging ability of antioxidants can be predicted from standard one-electron potentials by evaluating the capacity of an antioxidant to reduce an oxidant through colour change. The TEAC assay measures the antioxidant capacity of a compound or a mixture of compounds.

*Materials and methods*

The bacterial strain NCIMB42787 was grown to stationary phase. The BCFS were prepared as outlined above.

Quantification of Bacterial Indole Production from L-tryptophan

Bacterial Indole production was quantified using an assay described previously[70]. Bacteria were cultured to stationary phase of growth. 0.5mM indole in YCFA+ media was used as a positive chemical control in this assay. The Indole assay was performed using 24-well (non-treated) assay plates. 100mM tryptophan solution in HCl was dispensed into each well to give a final concentration of 6mM. 1ml stationary phase bacterial culture was added to each well and incubated for a further 48h. Assay plates were centrifuged at 3,500xg at RT for 10min. The supernatant was retained, and the pellet discarded. In a 96-well plate 140μl supernatant was dispensed in triplicate. 140μl Kovac's reagent was added and the absorbance read at 540nm using a BioRad iMark microplate absorbance reader. The standard curve was prepared by plotting absorbance as a function of final Indole concentration (mM). Indole concentration of the test sample was calculated using the equation extrapolated from linear regression of the standard curve.

2,2-Diphenyl-1-picrylhydrazyl (DPPH) free-radical assay

BCFS were thawed at 4°C for approximately 2h prior to use. All samples were diluted 1:2 in 1.5ml microfuge tubes using sterile 5mM PBS pH7 yielding a final volume of 1ml. A stock solution of 500μM Trolox in 5mM PBS, pH7 was prepared to make the standard curve. Lazaroid antioxidant U83836E was included (200μM in 100% methanol) as a positive control. The DPPH assay was performed in a 96-well plate as described previously[71] with minor modifications made. In brief, 10μl sample/standard/control was added, in triplicate, to corresponding wells of a 96-well plate. 200μl of a 200μmol/L DPPH solution was added to three empty wells as a control. 190μl of 200μmol/L DPPH was added to sample/standard/control wells and plates incubated in the dark for 30min at RT. Absorbance was read at 515nm using a BioRad iMark microplate absorbance reader. DPPH radical scavenging activity was calculated as follows:

$$\text{DPPH radical scavenging activity (\%)} = [1-(A_{sample} - A_{blank})/A_{control})]*\text{Dilution factor}*100$$

where $A_{sample}$ was the average absorbance of sample + 200μmol/ L DPPH, $A_{control}$ was the average absorbance of methanol DPPH without sample, and $A_{blank}$ is the average absorbance of YCFA media blank.

2,2'-azino-bis-3-ethylbenzthiazoline-6-sulphonic acid (ABTS) assay

The total antioxidant capacity assay was performed using the Antioxidant Assay Kit according to the manufacturer's instructions. Briefly, all samples were diluted 1:4 in IX assay buffer. In a 96-well plate, 10μl standard/control/sample was added in triplicate. 20μl myoglobin working solution was added to all standard/control/sample wells. 150μl ABTS substrate solution was added to each well and the absorbance measured at 405nm using a BioRad iMark microplate absorbance reader.

Statistical Analysis

Normally distributed data are presented as mean ± SEM; One-way Anova (Sidak's multiple comparison test) was used to analyse the data presented in this paper. A p value <0.05 was deemed significant in all cases.

*Results*

NCIMB 42787 displayed clear indole-forming capacity (Figure 25A). In addition, NCIMB 42787 acts as a radical scavenger in the DPPH assay and has a high total antioxidant capacity when compared a standard solution of Trolox, a water-soluble antioxidant derivative of Vitamin E (Figure 25B).

***Example 25 - M. massiliensis strains display protection of SH-SY5Y cells from oxidative stress***

*Introduction*

The ability of bacterial cell-free supernatant of NCIMB 42787 to protect U373, HMC3 and retinoic acid (RA)-differentiated SH-SY5Y cells from reactive oxygen species (ROS) generated by treatment with Tert-Butyl Hydrogen Peroxide (TBHP) was evaluated.

*Materials and methods*

To evaluate ROS production, U373 cells and HMC3 were plated in black 96 well plates at a density of 10,000 cells/well. U373 cells were rested for 72h while HMC3 were left to rest for 48h. Cells were washed with pre-warmed PBS and stained with 10μM DCFDA molecular probe for 20min in growth medium containing 2% FBS. Afterwards, the cells were washed with pre-warmed PBS again and treated with 100μM TBHP in the presence or absence of 10% BCFS for 2h.

Neuroblastoma SH-SY5Y cells were grown in 50% MEM and 50% Nutrient Mixture F-12 Ham media supplemented with 2mM L-Glutamine, 10% heat-inactivated FBS, 100U/ml penicillin and 100μg/ml streptomycin. Cells were plated in growth medium on a black 96-well plates at 5,000 cells/well and placed in $CO_2$ incubator. After 24h, media was replaced with differentiation medium (growth medium containing 1% FBS) and 10μM retinoic acid (RA). Differentiation medium was replaced every other day and cells were used after 10 days of differentiation. On Day 10, cells were washed with pre-warmed PBS and stained with 10μM DCFDA molecular probe for 20min in growth medium containing 1% FBS. Then, cells were washed with pre-warmed PBS again and treated with 100μM TBHP in the presence or absence of 10% BCFS for 2h.

Fluorescence intensity was measured using a TECAN plate reader at Excitation 485nm/Emission 530nm. Raw data were plotted and analysed using GraphPad Prism 7 software.

*Results*

In differentiated SH-SY5Y cells, NCIMB 42787 treatment resulted in significant protection from ROS induced by TBHP (Figure 25F).

***Example 26 - M. massiliensis strains produce butyric, valeric and hexanoic acid***

*Materials and methods*

SCFA extraction from YCFA+ and YCFA+ spiked with a standard mix of SCFAs (40 mM acetic acid and 20 mM formic acid, propionic acid, butyric acid, valeric acid and hexanoic acid) was conducted according to the method of De Baere *et al.*[72].

HPLC analysis of SCFAs

HPLC detection and quantification of SCFAs was conducted according to the method of De Baere et al.[72] with slight modifications. Briefly, HPLC analysis was performed using a Waters e2695 HPLC system equipped with a Waters Photodiode Array (PDA) detector 2998 (Waters Limited, Elstree, UK). HPLC analysis of SCFAs standards, SCFAs extracted from MRx0005 and MRx0029 BCFS and MRx0005 and MRx0029 hexane, diethyl ether, ethyl acetate, acetonitrile and methanol extracts were performed using an Xselect® HSS T3 3.5 μm 4.6×150mm LC column (Waters Limited, Elstree, UK). The LC analysis was performed using the photodiode array detector (PDA) set to analyse wavelengths of 200-800nm. SCFA detection and quantification was performed at 210nm. The mobile phase consisted in 25mM sodium phosphate buffer in HPLC water (pH adjusted to 3.0 using phosphoric acid (A) and acetonitrile (B). The LC method for SCFA detection and quantification was run using the solvent system with the following gradient: t0' A=95%, B=5%; t10' A=95%, B=5%; t30' A=30%, B=70%; t31' A=0%, B=100%; t36' A=0%, B=100%; t38' A=5%, B=95%; t60' A=5%, B=95%; flow=1ml/min.

A seven-point calibration curve was prepared for each SCFA by injecting 20μl of a two-fold serial dilution of a SCFA (40mM acetic acid and 20mM formic acid, propionic acid, butyric acid, valeric acid and hexanoic acid). Quantification- extraction efficiency was calculated using the formula below:

[SCFA in YCFA+ spiked and extracted]/[SCFA in YCFA+ spiked not extracted]

Extraction efficiency was used to determine the concentrations of individual SCFAs in each sample. The production of specific SCFAs was calculated by subtracting the amount of corresponding SCFA present in the unspiked media control.

Targeted metabolomics: bacterial metabolites and fatty acid analysis

Sample analysis was carried out by MS-Omics (Copenhagen, Denmark). A mixed pooled sample (QC sample) was created by taking an aliquot from each sample. This sample was analysed with regular intervals throughout the sequence. Matrix effects were tested for quantified compounds by spiking the QC sample in a minimum of two levels.

For GC-metabolite analysis, samples were derivatized with methyl chloroformate using a slightly modified version of the protocol described by Smart *et al.*[73]. All samples were analysed in a randomized order. Analysis was performed using GC (7890B, Agilent) coupled with a quadrupole detector (59977B, Agilent). Raw data was converted to netCDF format using Chemstation (Agilent), before the data was imported and processed in Matlab R2014b (Mathworks, Inc.) using the PARADISe software described by Johnsen *et al.*[74]

For SCFA analysis, samples were acidified using hydrochloric acid, and deuterium-labelled internal standards were added. Analysis was performed using a high-polarity column (Zebron™ ZB-FFAP, GC Cap. Column 30mX0.25mmX0.25μm) installed in a GC (7890B, Agilent) coupled with a quadrupole detector (59977B, Agilent). Raw data was converted to netCDF format using Chemstation (Agilent), before the data was imported and processed in Matlab R2014b (Mathworks, Inc.) using the PARADISe software described by Johnsen *et al.*[74].

*Results*

Fatty acid analysis, using targeted metabolomics, demonstrated that NCIMB 42787 produces butanoic (butyric), pentanoic (valeric) and hexanoic (caproic) acid, both in the linear and branched forms (C4-C6) (Figure 26A). Moreover, the ratio of 4-hydroxy-phenylacetic acid:media was increased in NCIMB 42787 cell-free supernatant. HPLC analysis of cell-free supernatants was used to monitor the production of formic, acetic, propionic, butyric, valeric, and hexanoic acid (based on retention time and absorbance spectrum of relevant SCFAs) by NCIMB 42787. Representative chromatograms for SCFA standards overlaid to NCIMB 42787 cell-free supernatants extracted for SCFAs are reported in Figure 26C. HPLC analysis confirmed the production of butyric, valeric and hexanoic acid by NCIMB 42787.

***Example 27 - M. massiliensis methanolic fractions containing butyrate show anti-inflammatory activity in U373 cells***

To investigate the role of SCFAs in reducing secretion of IL-6, U373 cells were treated with increasing concentrations of sodium butyrate (SB), sodium valerate (SV) and hexanoic acid (HA).

*Methods*

U373 cells were prepared as described above. Cells were pre-treated for 1h with 1μg/ml LPS indicated above and incubated at 37°C and 5% $CO_2$. After 1h pre-incubation, cells were removed from $CO_2$ incubator and treated with increasing concentration of fresh prepared Sodium Butyrate (SB), Sodium Valerate (SV) and Hexanoic Acid (HA).

Statistical Analysis

Normally distributed data are presented as mean ± SEM; One-way Anova (Sidak's multiple comparison test) was used to analyse the data presented in this paper. A p value <0.05 was deemed significant in all cases.

*Results and conclusions*

The concentrations tested covered the range of concentrations measured in the cell-free supernatants for the different fatty acids and took into account the fact that only 10% of the above-mentioned supernatants was used in the cell-based assays. Only SB inhibited LPS-induced secretion of IL-6 in U373 cells in a concentration-dependent manner (Figure 27A). HA did not inhibit IL-6 secretion after challenge with LPS. None of the SFCAs tested induced per se secretion of IL-6 above the basal level (untreated cell control). Only SB (at the highest concentration tested) decreased the basal level of IL-6 (Figure 27A and B). The reconstituted mixture of the three SCFAs reproduced the biological activity of NCIMB 42787 cell-free supernatant, both in the presence and absence of LPS.

Accordingly, in certain embodiments, the production of butyric acid drives the reduction in IL-6 secretion. In this way, in certain embodiments, the bacterial strains of the invention are useful in the treatment of inflammatory or autoimmune disorders via the production of butyric acid which drives the reduction of IL-6 secretion.

***Example 28 - SCFAs generated by NCIMB 42787 are at least partially responsible for anti-inflammatory activity***

*Introduction*

In order to further confirm whether the anti-inflammatory activity of NCIMB 42787 was due at least in part to SCFAs, cell-free bacterial supernatant was fractionated with different solvents of increasing polarity. HPLC analysis of the de-proteinased crude extracts (hexane, F5; diethyl ether, F4; ethyl acetate, F3; acetonitrile, F4; methanol, F1) of this strain supernatants was conducted to analyse the biochemical complexity of the stationary phase cell-free supernatants of NCIMB 42787, as well as to sub-fractionate compounds based on polarity and solubility.

*Methods*

Sequential solvent extractions- preparation of crude extracts

Three biological replicates of NCIMB 42787 strain BCFSs and YCFA+ (media control) were extracted sequentially with HPLC-grade hexane (HEX), diethyl ether (DE), ethyl acetate (EtOAc), acetonitrile (ACN) and methanol (MeOH). Briefly, 20ml of BCFS were placed in glass vials and extracted at room temperature (RT) in 20ml of HEX on a rotary shaker (70rpm) for 30min. A total of three extractions were performed on each BCFS and YCFA+ media control. The remaining aqueous layers were then extracted at RT in 20ml of DE, EtOAc on a MX-RD-Pro rotary shaker (70rpm) for 30min a total of three times. The combined extracts of each sample were dried under reduced pressure in an R-300 rotary evaporator equipped with a V-300 vacuum pump (Büchi, Flawil, Switzerland) at a temperature not exceeding 30°C. The resulting extracts were re-solubilised in 2ml of corresponding solvent and aliquoted in four 1.5ml Eppendorf tubes (500μl each corresponding to 5ml of original sample). The remaining aqueous layers were then extracted at RT in 20ml of DE, EtOAc on a MX-RD-Pro rotary shaker (70rpm) for 30min a total of three times. The combined extracts of each sample were dried under reduced pressure in a R-300 rotary evaporator equipped with a V-300 vacuum pump (Büchi, Flawil, Switzerland) at a temperature not exceeding 30°C. The resulting extracts were re-solubilised in 2ml of corresponding solvent and aliquoted in four 1.5ml Eppendorf tubes (500μl each corresponding to 5ml of original sample).

The remaining aqueous layers were evaporated to dryness using an R-300 rotary evaporator. The resulting dry extracts were extracted for 30min in 20ml of ACN a total of three times. The ACN extracts were combined, evaporated to dryness using a rotary evaporator, resolubilised in 2ml of ACN and aliquoted in four 1.5ml Eppendorf tubes (500μl each). The remaining dry extracts (ACN insoluble portion of the extracts) were then extracted for 30min in 20ml of MeOH a total of three times. The MeOH extracts were combined, evaporated to dryness using an R-300 Rotary Evaporator, resolubilised in 2ml of MeOH and aliquoted in four 1.5ml Eppendorf tubes (500μl each).

Aliquots of the crude extracts were kept overnight at -20°C inducing the precipitation of proteinaceous components. Following overnight precipitation, each aliquot was centrifuged at 10,000xg for 6min and transferred to a new 2ml tube. Overnight precipitation was repeated three times after which extracts were dried in a RVC 2-18 CDPlus speedvac (Christ, Osterode am Harz, Germany) and weighed. All dried aliquots of each extract were stored at -80°C until further use.

Treatment

U373 cells were prepared as described above. Cells were pre-treated for 1h with 1μg/ml LPS as indicated above. Afterwards, cells were removed from $CO_2$ incubator and treated with 100μl of the different fractions. Fractions from media were used as controls. Cell-free supernatants were collected 24h after treatment and analysed by ELISA for IL-6 secretion (as outlined above).

Statistical Analysis

Normally distributed data are presented as mean ± SEM; One-way Anova (Sidak's multiple comparison test) was used to analyse the data presented in this paper. A p value <0.05 was deemed significant in all cases.

*Results*

HPLC analysis confirmed the selective extraction and crude fractionation of compounds present in the deproteinased supernatants. The methanolic fraction F1 of NCIMB 42787 decreased IL-6 production and appeared to recapitulate the activity of the unfractionated supernatant, further indicating that the presence of butyrate is associated with the anti-inflammatory activity of this strain (see Figure 28A).
In the absence of LPS, only the unfractionated NCIMB 42787 retained its ability to induce IL-6 (Figure 28B).

***Example 29 - NCIMB 42787 significantly reduces the production of TNFα by stimulated splenocytes***

Live biotherapeutic strains were screened *ex vivo* for efficacy of immune marker production in splenocytes isolated from BALB/c mice and stimulated with LPS or ConA.
Figure 29 displays the ability of compositions of the invention to significantly decrease production of the pro-inflammatory cytokine TNFα. TNFα triggers significant inflammatory responses, and so the ability to reduce production of this cytokine will reduce inflammation.
Accordingly, in certain embodiments, the compositions of the invention drive reduction of TNFα levels. In certain embodiments, the compositions of the invention are therapeutically beneficial in light of the reduction of TNFα.

***Example 30 - Megasphaera strain deposited under accession number NCIMB 43385 significantly increases the expression of the glucocorticoid receptor in the hippocampus and amygdala***

BALB/c mice were administered live biotherapeutic and tissues were isolated for analysis of gene expression using qPCR.
Figure 30 demonstrates that NCIMB 43385 triggers an increase in the expression of the glucocorticoid receptor (Nr3c1) in both the hippocampus and amygdala.
The use of glucocorticoids in the treatment of chronic inflammatory conditions is well known, in particular in light of their role in suppressing pro-inflammatory cytokines. In addition, the glucocorticoid pathway has been therapeutically exploited in cancer because it can trigger anti-proliferative and anti-angiogenic responses. Therefore, in certain embodiments, the compositions of the invention trigger an increase in the expression of the glucocorticoid receptor. In other embodiments, the compositions of the invention are therapeutically beneficial for treating inflammatory disorders due to the increased expression of the glucocorticoid receptor. In other embodiments, the compositions of the invention are therapeutically beneficial for treating cancer due to the increase in expression of the glucocorticoid receptor which promotes anti-proliferative and/or anti-angiogenic responses.

## Sequences

SEQ ID NO:1 (consensus 16S rRNA sequence for *Megasphaera massiliensis* strain MRx0029)

TGAGAAGCTTGCTTCTTATCGATTCTAGTGGCAAACGGGTGAGTAACGCGTAAGCAACCTGCCCTTCA
GATGGGGACAACAGCTGGAAACGGCTGCTAATACCGAATACGTTCTTTCCGCCGCATGACGGGAAGA
AGAAAGGGAGGCCTTCGGGCTTTCGCTGGAGGAGGGGCTTGCGTCTGATTAGCTAGTTGGAGGGGTAA
CGGCCCACCAAGGCGACGATCAGTAGCCGGTCTGAGAGGATGAACGGCCACATTGGGACTGAGACAC
GGCCCAGACTCCTACGGGAGGCAGCAGTGGGGAATCTTCCGCAATGGACGAAAGTCTGACGGAGCAA
CGCCGCGTGAACGATGACGGCCTTCGGGTTGTAAAGTTCTGTTATATGGGACGAACAGGACATCGGTT
AATACCCGGTGTCTTTGACGGTACCGTAAGAGAAAGCCACGGCTAACTACGTGCCAGCAGCCGCGGTA
ATACGTAGGTGGCAAGCGTTGTCCGGAATTATTGGGCGTAAAGGGCGCGCAGGCGGCATCGCAAGTC
GGTCTTAAAAGTGCGGGGCTTAACCCCGTGAGGGGACCGAAACTGTGAAGCTCGAGTGTCGGAGAGG
AAAGCGGAATTCCTAGTGTAGCGGTGAAATGCGTAGATATTAGGAGGAACACCAGTGGCGAAAGCGG
CTTTCTGGACGACAACTGACGCTGAGGCGCGAAAGCCAGGGGAGCAAACGGGATTAGATACCCCGGT
AGTCCTGGCCGTAAACGATGGATACTAGGTGTAGGAGGTATCGACTCCTTCTGTGCCGGAGTTAACGC
AATAAGTATCCCGCCTGGGGAGTACGGCCGCAAGGCTGAAACTCAAAGGAATTGACGGGGGCCCGCA
CAAGCGGTGGAGTATGTGGTTTAATTCGACGCAACGCGAAGAACCTTACCAAGCCTTGACATTGATTG
CTACGGAAAGAGATTTCCGGTTCTTCTTCGGAAGACAAGAAAACAGGTGGTGCACGGCTGTCGTCAGC
TCGTGTCGTGAGATGTTGGGTTAAGTCCCGCAACGAGCGCAACCCCTATCTTCTGTTGCCAGCACCTCG
GGTGGGGACTCAGAAGAGACTGCCGCAGACAATGCGGAGGAAGGCGGGGATGACGTCAAGTCATCAT
GCCCCTTATGGCTTGGGCTACACACGTACTACAATGGCTCTTAATAGAGGGAAGCGAAGGAGCGATCC
GGAGCAAACCCCAAAAACAGAGTCCCAGTTCGGATTGCAGGCTGCAACTCGCCTGCATGAAGCAGGA
ATCGCTAGTAATCGCAGGTCAGCATACTGCGGTGAATACGTTCCCGGGCCTTGTACACACCGCCCGTC
ACACCACGAAAGTCATTCACACCCGAAGCCGGTGAGGCAACCGCAAG

Primers used for qPCR

| Name | Forward sequence | Reverse sequence |
|---|---|---|
| *GPR109a* | ATGTTGGCTATGAACCGCCAG (SEQ ID NO: 2) | GCTGCTGTCCGATTGGAGA (SEQ ID NO: 3) |

Primer sequences for TPH1 and β-actin

| Gene | Forward | Reverse |
|---|---|---|
| TPH1 | AAAGAGCGT ACAGGTTTTTC (SEQ ID NO:4) | GTCTCACATATTGAGTGCAG (SEQ ID NO:5) |
| TPH2 | CACTATTGTGACGCTGAATC (SEQ ID NO:6) | AGCTCAGAACCATACATGAG (SEQ ID NO:7) |
| β-actin | GATCAAGATCATTGCTCCTC (SEQ ID NO:8) | TTGTCAAGAAAGGGTGTAAC (SEQ ID NO:9) |

Primer sequences for SLC6A4 and β-actin

| Gene | Forward | Reverse |
|---|---|---|
| SLC6A4 | AATCTGCCGATTTTCAAAG (SEQ ID NO:10) | GTGTTGTAGTAGGAAGCAATG (SEQ ID NO:11) |
| β-actin | GATCAAGATCATTGCTCCTC (SEQ ID NO:12) | TTGTCAAGAAAGGGTGTAAC (SEQ ID NO:13) |

SEQ ID NO: 14 (consensus 16S rRNA sequence for the *Megasphaera* strain deposited under accession number NCIMB 43385)

GGCTGGTTCCTTGCGGTTGCCTCACCGGCTTCGGGTGTGAATGACTTTCGTGGTGTGACG
GGCGGTGTGTACAAGGCCCGGGAACGTATTCACCGCAGTATGCTGACCTGCGATTACTA
GCGATTCCTGCTTCATGCAGGCGAGTTGCAGCCTGCAATCCGAACTGGGACTCTGTTTTT
GGGGTTTGCTCCGGATCGCTCCTTCGCTTCCCTCTATTAAGAGCCATTGTAGTACGTGTG
TAGCCCAAGCCATAAGGGGCATGATGACTTGACGTCATCCCCGCCTTCCTCCGCATTGTC
TGCGGCAGTCTCTTCTGAGTCCCCACCCTTAGTGCTGGCAACAGAAGATAGGGGTTGCG
CTCGTTGCGGGACTTAACCCAACATCTCACGACACGAGCTGACGACAGCCGTGCACCAC
CTGTTTTCTTGTCTTCCGAAGAAGAACCGGAAATCTCTTTCCGTAGCAATCAATGTCAAG
GCTTGGTAAGGTTCTTCGCGTTGCGTCGAATTAAACCACATACTCCACCGCTTGTGCGGG
CCCCCGTCAATTCCTTTGAGTTTCAGCCTTGCGGCCGTACTCCCCAGGCGGATACTTAT
TGCGTTAACTCCGGCACAGAAGGAGTCGATACCTCCTACACCTAGTATCCATCGTTTACG
GCCAGGACTACCGGGGTATCTAATCCCGTTTGCTCCCCTGGCTTTCGCGCCTCAGCGTCA
GTTGTCGTCCAGAAAGCCGCTTTCGCCACTGGTGTTCCTCCTAATATCTACGCATTTCAC
CGCTACACTAGGAATTCCGCTTTCCTCTCCGACACTCGAGCTTCACAGTTTCGGTCCCCT
CACGGGGTTAAGCCCCGCACTTTTAAGACCGACTTGCGATGCCGCCTGCGCGCCCTTTAC
GCCCAATAATTCCGGACAACGCTTGCCACCTACGTATTACCGCGGCTGCTGGCACGTAG
TTAGCCGTGGCTTTCTCTTACGGTACCGTCAGGGATAACGGGTATTGACCGCTATCCTGT
TCGTCCCATATAACAGAACTTTACAACCCGAAGGCCGTCATCGTTCACGCGGCGTTGCTC

CGTCAGACTTTCGTCCATTGCGGAAGATTCCCCACTGCTGCCTCCCGTAGGAGTCTGGGC
CGTGTCTCAGTCCCAATGTGGCCGTTCATCCTCTCAGACCGGCTACTGATCGTCGCCTTG
GTGGGCCGTTACCCCTCCAACTAGCTAATCAGACGCAAGCCCCTCCTCCAGCGAAAGCC
CGAAGGCCTCCCTTTCTTCATCCCGTCATGCGGCGGAAAGAACGTATTCGGTATTAGCA
GCCGTTTCCAGCTGTTGTCCCCATCTGAAGGGCAGGTTGCTTACGCGTTACTCACCCGTT
TGCCACTCGAATTGATAAGAAGCAAGCTTCTCATC

SEQ ID NO: 15 (consensus 16S rRNA sequence for the *Megasphaera massiliensis* strain deposited under accession number NCIMB 43388)

GGCTGGTTCCTTGCGGTTGCCTCACCGGCTTCGGGTGTGAATGACTTTCGTGGTGTGACG
GGCGGTGTGTACAAGGCCCGGGAACGTATTCACCGCAGTATGCTGACCTGCGATTACTA
GCGATTCCTGCTTCATGCAGGCGAGTTGCAGCCTGCAATCCGAACTGGGACTCTGTTTTT
GGGGTTTGCTCCGGATCGCTCCTTCGCTTCCCTCTATTAAGAGCCATTGTAGTACGTGTG
TAGCCCAAGCCATAAGGGGCATGATGACTTGACGTCATCCCCGCCTTCCTCCGCATTGTC
TGCGGCAGTCTCTTCTGAGTCCCCACCCGAGGTGCTGGCAACAGAAGATAGGGGTTGCG
CTCGTTGCGGGACTTAACCCAACATCTCACGACACGAGCTGACGACAGCCGTGCACCAC
CTGTTTTCTTGTCTTCCGAAGAAGAACCGGAAATCTCTTTCCGTAGCAATCAATGTCAAG
GCTTGGTAAGGTTCTTCGCGTTGCGTCGAATTAAACCACATACTCCACCGCTTGTGCGGG
CCCCCGTCAATTCCTTTGAGTTTCAGCCTTGCGGCCGTACTCCCCAGGCGGGATACTTAT
TGCGTTAACTCCGGCACAGAAGGAGTCGATACCTCCTACACCTAGTATCCATCGTTTACG
GCCAGGACTACCGGGGTATCTAATCCCGTTTGCTCCCCTGGCTTTCGCGCCTCAGCGTCA
GTTGTCGTCCAGAAAGCCGCTTTCGCCACTGGTGTTCCTCCTAATATCTACGCATTTCAC
CGCTACACTAGGAATTCCGCTTTCCTCTCCGACACTCGAGCTTCACAGTTTCGGTCCCCT
CACGGGGTTAAGCCCCGCACTTTTAAGACCGACTTGCGATGCCGCCTGCGCGCCCTTTAC
GCCCAATAATTCCGGACAACGCTTGCCACCTACGTATTACCGCGGCTGCTGGCACGTAG
TTAGCCGTGGCTTTCTCTTACGGTACCGTCAAAGACACCGGGTATTAACCGATGTCCTGT
TCGTCCCATATAACAGAACTTTACAACCCGAAGGCCGTCATCGTTCACGCGGCGTTGCTC
CGTCAGACTTTCGTCCATTGCGGAAGATTCCCCACTGCTGCCTCCCGTAGGAGTCTGGGC
CGTGTCTCAGTCCCAATGTGGCCGTTCATCCTCTCAGACCGGCTACTGATCGTCGCCTTG
GTGGGCCGTTACCCCTCCAACTAGCTAATCAGACGCAAGCCCCTCCTCCAGCGAAAGCC
CGAAGGCCTCCCTTTCTTCTTCCCGTCATGCGGCGGAAAGAACGTATTCGGTATTAGCAG
CCGTTTCCAGCTGTTGTCCCCATCTGAAGGGCAGGTTGCTTACGCGTTACTCACCCGTTT
GCCACTAGAATCGATAAGAAGCAAGCTTCTCATGTCTTCT

SEQ ID NO: 16 (consensus 16S rRNA sequence for the *Megasphaera massilliensis* strain deposited under accession number NCIMB 43389)

CGACGGCTGGTTCCTTGCGGTTGCCTCACCGGCTTCGGGTGTGAATGACTTTCGTGGTGT
GACGGGCGGTGTGTACAAGGCCCGGGAACGTATTCACCGCAGTATGCTGACCTGCGATT

ACTAGCGATTCCTGCTTCATGCAGGCGAGTTGCAGCCTGCAATCCGAACTGGGACTCTG TTTTTGGGGTTTGCTCCGGATCGCTCCTTCGCTTCCCTCTATTAAGAGCCATTGTAGTACG TGTGTAGCCCAAGCCATAAGGGGCATGATGACTTGACGTCATCCCCGCCTTCCTCCGCAT TGTCTGCGGCAGTCTCTTCTGAGTCCCCACCCGAGGTGCTGGCAACAGAAGATAGGGGT TGCGCTCGTTGCGGGACTTAACCCAACATCTCACGACACGAGCTGACGACAGCCGTGCA CCACCTGTTTTCTTGTCTTCCGAAGAAGAACCGGAAATCTCTTTCCGTAGCAATCAATGT CAAGGCTTGGTAAGGTTCTTCGCGTTGCGTCGAATTAAACCACATACTCCACCGCTTGTG CGGGCCCCCGTCAATTCCTTTGAGTTTCAGCCTTGCGGCCGTACTCCCCAGGCGGATAC TTATTGCGTTAACTCCGGCACAGAAGGAGTCGATACCTCCTACACCTAGTATCCATCGTT TACGGCCAGGACTACCGGGGTATCTAATCCCGTTTGCTCCCCTGGCTTTCGCGCCTCAGC GTCAGTTGTCGTCCAGAAAGCCGCTTTCGCCACTGGTGTTCCTCCTAATATCTACGCATT TCACCGCTACACTAGGAATTCCGCTTTCCTCTCCGACACTCGAGCTTCACAGTTTCGGTC CCCTCACGGGGTTAAGCCCCGCACTTTTAAGACCGACTTGCGATGCCGCCTGCGCGCCCT TTACGCCCAATAATTCCGGACAACGCTTGCCACCTACGTATTACCGCGGCTGCTGGCAC GTAGTTAGCCGTGGCTTTCTCTTACGGTACCGTCAAAGACACCGGGTATTAACCGATGCC CTGTTCGTCCCATATAACAGAACTTTACAACCCGAAGGCCGTCATCGTTCACGCGGCGTT GCTCCGTCAGACTTTCGTCCATTGCGGAAGATTCCCCACTGCTGCCTCCCGTAGGAGTCT GGGCCGTGTCTCAGTCCCAATGTGGCCGTTCATCCTCTCAGACCGGCTACTGATCGTCGC CTTGGTGGGCCGTTACCCCTCCAACCAGCTAATCAGACGCAAGCCCCTCCTCCAGCGAA AGCCCGAAGGCCTCCCTTTCTTCTTCCCGTCATGCGGCGGAAAGAACGTATTCGGTATTA GCAGCCGTTTCCAGCTGTTGTCCCCATCTGAAGGGCAGGTTGCTTACGCGTTACTCACCC GTTTGCCACTAGAATCGATAAGAAGCAAGCTTCTCATGTCTTCTCGTTCGACTTGCAT

SEQ ID NO: 17 (consensus 16S rRNA sequence for the *Megasphaera* strain deposited under accession number NCIMB 43386)

CGACGGCTGGTTCCTTGCGGTTGCCTCACCGGCTTCGGGTGTGAATGACTTTCGTGGTGT GACGGGCGGTGTGTACAAGGCCCGGGAACGTATTCACCGCAGTATGCTGACCTGCGATT ACTAGCGATTCCTGCTTCATGCAGGCGAGTTGCAGCCTGCAATCCGAACTGGGACTCTG TTTTTGGGGTTTGCTCCGGATCGCTCCTTCGCTTCCCTCTATTAAGAGCCATTGTAGTACG TGTGTAGCCCAAGCCATAAGGGGCATGATGACTTGACGTCATCCCCGCCTTCCTCCGCAT TGTCTGCGGCAGTCTCTTCTGAGTCCCCACCCTTAGTGCTGGCAACAGAAGATAGGGGTT GCGCTCGTTGCGGGACTTAACCCAACATCTCACGACACGAGCTGACGACAGCCGTGCAC CACCTGTTTTCTTGTCTTCCGAAGAAGAACCGGAAATCTCTTTCCGTAGCAATCAATGTC AAGGCTTGGTAAGGTTCTTCGCGTTGCGTCGAATTAAACCACATACTCCACCGCTTGTGC GGGCCCCCGTCAATTCCTTTGAGTTTCAGCCTTGCGGCCGTACTCCCCAGGCGGATACT TATTGCGTTAACTCCGGCACAGAAGGAGTCGATACCTCCTACACCTAGTATCCATCGTTT ACGGCCAGGACTACCGGGGTATCTAATCCCGTTTGCTCCCCTGGCTTTCGCGCCTCAGCG TCAGTTGTCGTCCAGAAAGCCGCTTTCGCCACTGGTGTTCCTCCTAATATCTACGCATTT

CACCGCTACACTAGGAATTCCGCTTTCCTCTCCGACACTCGAGCTTCACAGTTTCGGTCC
CCTCACGGGGTTAAGCCCCGCACTTTTAAGACCGACTTGCGATGCCGCCTGCGCGCCCTT
TACGCCCAATAATTCCGGACAACGCTTGCCACCTACGTATTACCGCGGCTGCTGGCACG
TAGTTAGCCGTGGCTTTCTCTTACGGTACCGTCAGGGATAACGGGTATTGACCGCTATCC
TGTTCGTCCCATATAACAGAACTTTACAACCCGAAGGCCGTCATCGTTCACGCGGCGTTG
CTCCGTCAGACTTTCGTCCATTGCGGAAGATTCCCCACTGCTGCCTCCCGTAGGAGTCTG
GGCCGTGTCTCAGTCCCAATGTGGCCGTTCATCCTCTCAGACCGGCTACTGATCGTCGCC
TTGGTGGGCCGTTACCCCTCCAACTAGCTAATCAGACGCAAGCCCCTCCTCCAGCGAAA
GCCCGAAGGCCTCCCTTTCTTCATCCCGTCATGCGGCGGAAAGAACGTATTCGGTATTAG
CAGCCGTTTCCAGCTGTTGTCCCCATCTGAAGGGCAGGTTGCTTACGCGTTACTCACCCG
TTTGCCACTCGAATTGATAAGAAGCAAGCTTCTCATCTCTTCTCGTTCGACTGCA

SEQ ID NO: 18 (consensus 16S rRNA sequence for the *Megasphaera* strain deposited under accession number NCIMB 43387)

TCGAACGGCTGGTTCCTTGCGGTTGCCTCACCGGCTTCGGGTGTGAATGACTTTCGTGGT
GTGACGGGCGGTGTGTACAAGGCCCGGGAACGTATTCACCGCAGTATGCTGACCTGCGA
TTACTAGCGATTCCTGCTTCATGCAGGCGAGTTGCAGCCTGCAATCCGAACTGGGACTCT
GTTTTTGGGGTTTGCTCCGGATCGCTCCTTCGCTTCCCTCTATTAAGAGCCATTGTAGTAC
GTGTGTAGCCCAAGCCATAAGGGGCATGATGACTTGACGTCATCCCCGCCTTCCTCCGC
ATTGTCTGCGGCAGTCTCTTCTGAGTCCCCACCCTTAGTGCTGGCAACAGAAGATAGGG
GTTGCGCTCGTTGCGGGACTTAACCCAACATCTCACGACACGAGCTGACGACAGCCGTG
CACCACCTGTTTTCTTGTCTTCCGAAGAAGAACCGGAAATCTCTTTCCGTAGCAATCAAT
GTCAAGGCTTGGTAAGGTTCTTCGCGTTGCGTCGAATTAAACCACATACTCCACCGCTTG
TGCGGGCCCCCGTCAATTCCTTTGAGTTTCAGCCTTGCGGCCGTACTCCCCAGGCGGGAT
ACTTATTGCGTTAACTCCGGCACAGAAGGAGTCGATACCTCCTACACCTAGTATCCATCG
TTTACGGCCAGGACTACCGGGGTATCTAATCCCGTTTGCTCCCCTGGCTTTCGCGCCTCA
GCGTCAGTTGTCGTCCAGAAAGCCGCTTTCGCCACTGGTGTTCCTCCTAATATCTACGCA
TTTCACCGCTACACTAGGAATTCCGCTTTCCTCTCCGACACTCGAGCTTCACAGTTTCGG
TCCCCTCACGGGGTTAAGCCCCGCACTTTTAAGACCGACTTGCGATGCCGCCTGCGCGCC
CTTTACGCCCAATAATTCCGGACAACGCTTGCCACCTACGTATTACCGCGGCTGCTGGCA
CGTAGTTAGCCGTGGCTTTCTCTTACGGTACCGTCAGGGATAACGGGTATTGACCGCTAT
CCTGTTCGTCCCATATAACAGAACTTTACAACCCGAAGGCCGTCATCGTTCACGCGGCGT
TGCTCCGTCAGACTTTCGTCCATTGCGGAAGATTCCCCACTGCTGCCTCCCGTAGGAGTC
TGGGCCGTGTCTCAGTCCCAATGTGGCCGTTCATCCTCTCAGACCGGCTACTGATCGTCG
CCTTGGTGGGCCGTTACCCCTCCAACTAGCTAATCAGACGCAAGCCCCTCCTCCAGCGA
AAGCCCGAAGGCCTCCCTTTCTTCATCCCGTCATGCGGCGGAAAGAACGTATTCGGTATT
AGCAGCCGTTTCCAGCTGTTGTCCCCATCTGAAGGGCAGGTTGCTTACGCGTTACTCACC
CGTTTGCCACTCGAATTGATAAGAAGCAAGCTTCTCATCTCTTCTCGTTCGACTTGCA

Primers used for qPCR

| Name | Forward sequence | Reverse sequence |
|---|---|---|
| NSE | CCCTGTATCGTAAGAACGGT (SEQ ID NO: 19) | GCCACCATTGATCACGTTGA (SEQ ID NO: 20) |
| GAPDH | GGTATCGTGGAAGGACTCATG (SEQ ID NO: 21) | ATGCCAGTGAGCTTCCCGTTC (SEQ ID NO: 22) |

**REFERENCES**

[1] Spor et al. (2011) Nat Rev Microbiol. 9(4):279-90.
[2] Eckburg et al. (2005) Science. 10;308(5728):1635-8.
[3] Macpherson et al. (2001) Microbes Infect. 3(12):1021-35
[4] Macpherson et al. (2002) Cell Mol Life Sci. 59(12):2088-96.
[5] Mazmanian et al. (2005) Cell 15;122(1):107-18.
[6] Frank et al. (2007) PNAS 104(34):13780-5.
[7] Scanlan et al. (2006) J Clin Microbiol. 44(11):3980-8.
[8] Kang et al. (2010) Inflamm Bowel Dis. 16(12):2034-42.
[9] Machiels et al. (2013) Gut. 63(8):1275-83.
[10] WO 2013/050792
[11] WO 03/046580
[12] WO 2013/008039
[13] WO 2014/167338
[14] Goldin and Gorbach (2008) Clin Infect Dis. 46 Suppl 2:S96-100.
[15] Azad et al. (2013) BMJ. 347:f6471.
[16] Terry and Margolis (2017) Handb Exp Pharmacol. 239: 319-342.
[17] Padmanabhan et al. (2013) Standards in Genomic Sciences 8:525-538
[18] Mąsco et al. (2003) Systematic and Applied Microbiology, 26:557-563.
[19] Srutková et al. (2011) J. Microbiol. Methods, 87(1):10-6.
[20] Leoni et al. (2005) Mol. Med., 11(1-12):1-15.
[21] Glauben, et al. (2006) Journal of Immunology, 176(8): 5015-5022
[22] Walmsley et al 1998 Gut 43: 29-32
[23] Gasche et al 200 Inflammatory Bowel Diseases 6: 8-15
[24] Fahy (2009) Proc Am Thorac Soc 6.256-259
[25] Reddy et al (2008) J Clin. Invest. 118:2562-73
[26] Leng et al (2006) Exp. Hematol. 34:776-787
[27] Tao et al (2007) Nat. Med. 13:1299-1307
[28] Song et al (2005) APMIS 113: 264-268
[29] Zimmerman et al (2007) Cancer Res 67: 9074-54
[30] Zhang et al (2005) Breast Cancer Res Treat 94: 11-16
[31] Abbas and Gupta (2008) Epigenetics 3: 300-309
[32] Minamiya et al (2011) Lung Cancer 74: 300-4
[33] Jung et al (2012) J Cell Biochem 113: 2167-2177
[34] Quint et al 2011 Virchows Arch 459: 129-139
[35] Miyamoto-Shinohara et al. (2008) J. Gen. Appl. Microbiol., 54, 9-24.
[36] Cryopreservation and Freeze-Drying Protocols, ed. by Day and McLellan, Humana Press.
[37] Leslie et al. (1995) Appl. Environ. Microbiol. 61, 3592-3597.
[38] Mitropoulou et al. (2013) J Nutr Metab. (2013) 716861.
[39] Kailasapathy et al. (2002) Curr Issues Intest Microbiol. 3(2):39-48.
[40] Handbook of Pharmaceutical Excipients, 2nd Edition, (1994), Edited by A Wade and PJ Weller
[41] Remington's Pharmaceutical Sciences, Mack Publishing Co. (A. R. Gennaro edit. 1985)
*[42]* Handbook of Microbiological Media, Fourth Edition (2010) Ronald Atlas, CRC Press.
*[43]* Maintaining Cultures for Biotechnology and Industry (1996) Jennie C. Hunter-Cevera, Academic Press

[44] Strobel (2009) Methods Mol Biol. 581:247-61.
[45] Gennaro (2000) Remington: The Science and Practice of Pharmacy. 20th edition, ISBN: 0683306472.
*[46]* Molecular Biology Techniques: An Intensive Laboratory Course, (Ream et al., eds., 1998, Academic Press).
*[47]* Methods In Enzymology (S. Colowick and N. Kaplan, eds., Academic Press, Inc.)
*[48]* Handbook of Experimental Immunology, Vols. I-IV (D.M. Weir and C.C. Blackwell, eds, 1986, Blackwell Scientific Publications)
[49] Sambrook et al. (2001) Molecular Cloning: A Laboratory Manual, 3rd edition (Cold Spring Harbor Laboratory Press).
[50] Handbook of Surface and Colloidal Chemistry (Birdi, K.S. ed., CRC Press, 1997)
[51] Ausubel et al. (eds) (2002) Short protocols in molecular biology, 5th edition (Current Protocols).
*[52]* PCR (Introduction to Biotechniques Series), 2nd ed. (Newton & Graham eds., 1997, Springer Verlag)
[53] Current Protocols in Molecular Biology (F.M. Ausubel et al., eds., 1987) Supplement 30
[54] Smith & Waterman (1981) Adv. Appl. Math. 2: 482-489
[55]West and Johnstone, (2014) J Clin Invest, 124, 30-39
[56] Glauben et al (2006) J Immunol. 176, 5015-5022
[57] Angiolilli et al. (2017) Ann Rheum Dis. 76, 277-285
[58] Gonneaud et al (2014). J Inflamm. 11: 43
[59] Alenghat et al. (2013). Nature. 504: 153-157
[60] Felice et al (2015). Ailment Pharmacol Ther. 41: 26-38
[61] Thangaraju et al. (2009). Cancer Res. 67, 9: 2826-2832
[62] Singh et al. (2014) Immunity. 16;40(1):128-39.
[63] Livak & Schmittgen (2001). Methods. 25,4:402-8.
[64] Johnsen et al (2017) Journal of Chromatography A. 1503: 57-64
[65] Vizin and Kos (2015) Radiol Oncol. 49(3): 217-226
[66] Selvan et al. (2008) AACR Annual Meeting Apr 12-16, 2008
[67] Bonner et al. (2000) Clinical Cancer Research 6:597-601
[68] Zhi et al. (2016) Oncotarget. 7(40):64798-64809.
[69] Bae et al. (2012) J Immunol. 2012 Jul 1;189(1):365-72.
[70] Sasaki-Imamura et al. (2010) Appl Environ Microbiol 76(13), 4260-4268
[71] Chai et al. (2012) Evid Based Complement Alternat Med, 603678
[72] De Baere et al. (2013) J Pharm Biomed Anal, 80: 107-115
[73] Smart et al. (2010) Nat Protoc, 5(10), 1709-1729
[74] Johnsen et al. (2017) J Chromatogr A, 1503, 57-64

The invention is further characterised by the following numbered non-limiting embodiments:

1. A composition comprising a bacterial strain of the genus *Megasphaera,* for use in the treatment or prevention of an autoimmune or inflammatory disorder or cancer.
2. The composition of embodiment 1, for use in the treatment of asthma, arthritis, psoriasis, diabetes, allograft rejection, graft-versus-host disease, an inflammatory bowel disease, such as Crohn's disease or ulcerative colitis, or prostate cancer, colorectal cancer, breast cancer, lung cancer, liver cancer or gastric cancer.
3. The composition according to any preceding embodiment, for use in the treatment or prevention of a disease or condition mediated by Class I HDAC activity.
4. The composition according to any preceding embodiment, for use in a method of selectively inhibiting Class I HDAC activity in the treatment of a condition mediated by Class I HDAC activity.
5. The composition according to any preceding embodiment, wherein the composition is for use in selectively inhibiting HDAC1, HDAC2 or HDAC3 in a disease or condition mediated by HDAC1, HDAC2 or HDAC3 activity.
6. The composition according to any preceding embodiment, for use in a patient with elevated HDAC activity.
7. The composition of any preceding embodiment, wherein the bacterial strain has a 16S rRNA sequence that is at least 95%, 96%, 97%, 98%, 99%, 99.5% or 99.9% identical to the 16S rRNA sequence of a bacterial strain of the genus *Megasphaera.*
8. The composition of any preceding embodiment, wherein the bacterial strain has a 16s rRNA gene sequence that is at least 95%, 96%, 97%, 98%, 99%, 99.5% or 99.9% identical to any one of SEQ ID NOs:14, 15, 16, 17 or 18 or wherein the bacterial strain has a 16s rRNA gene sequence represented by any one of SEQ ID NOs: 14, 15, 16, 17 or 18.
9. The composition of any preceding embodiment, wherein the bacterial strain is of *Megasphaera massiliensis.*
10. The composition of any preceding embodiment, wherein the bacterial strain has a 16s rRNA gene sequence that is at least 95%, 96%, 97%, 98%, 99%, 99.5% or 99.9% identical to SEQ ID NO:1 or wherein the bacterial strain has a 16s rRNA gene sequence represented by SEQ ID NO:1.

11. The composition of any preceding embodiment, wherein the composition is for oral administration.

12. The composition of any preceding embodiment, wherein the composition comprises one or more pharmaceutically acceptable excipients or carriers.

13. The composition of any preceding embodiment, wherein the bacterial strain is lyophilised.

14. A food product comprising the composition of any preceding embodiment, for the use of any preceding embodiment.

15. A method of treating or preventing a disease or condition mediated by histone deacetylase activity, comprising administering a composition comprising a bacterial strain of the genus *Megasphaera* to a patient in need thereof.

16. A cell of the *Megasphaera massiliensis* strain deposited under accession number NCIMB 42787, or a derivative thereof.

17. A cell of the *Megasphaera massiliensis* strain deposited under accession number NCIMB 42787, or a derivative thereof, for use in therapy, preferably for use in the treatment or prevention of a disease or condition as defined in one of embodiments 1-4.

18. A bacterial strain for use in therapy, wherein the bacterial strain has a 16S rRNA sequence that is at least 95%, 96%, 97%, 98%, 99%, 99.5% or 99.9% identical to any one of SEQ ID NOs:14, 15, 16, 17 or 18.

19. A bacterial strain having the 16S rRNA sequence represented by any one of SEQ ID NOs: 14, 15, 16, 17 or 18 for use in therapy.

## Claims

1. A composition comprising a bacterial strain which produces valeric acid, wherein the bacterial strain is not *Megasphaera elsdenii,* for use in the treatment or prevention of cancer in a human subject.

2. The composition of claim 1, wherein the composition includes a therapeutically effective amount of a bacterial strain of the invention.

3. The composition for use of claim 1, for use in the treatment of prostate cancer, colorectal cancer, breast cancer, lung cancer, liver cancer or gastric cancer.

4. The composition for use according to any preceding claim, for use in a method of selectively inhibiting Class I HDAC activity in the treatment of the cancer.

5. The composition for use according to any preceding claim, for use in a subject with elevated HDAC activity.

6. The composition for use according to any preceding claim, wherein the composition is used in combination with a chimeric antigen receptor T (CAR-T) cell therapy.

7. The composition for use of any preceding claim, wherein the bacterial strain has a 16s rRNA gene sequence that is at least 95%, 96%, 97%, 98%, 99%, 99.5% or 99.9% identical to any one of SEQ ID NOs:14, 15, 16, 17 or 18 or wherein the bacterial strain has a 16s rRNA gene sequence represented by any one of SEQ ID NOs: 14, 15, 16, 17 or 18.

8. The composition for use of any preceding claim, wherein the bacterial strain is of the genus *Megasphaera.*

9. The composition for use of claim 8, wherein the bacterial strain is of *Megasphaera massiliensis.*

10. The composition for use of any preceding claim, wherein the bacterial strain has a 16s rRNA gene sequence that is at least 95%, 96%, 97%, 98%, 99%, 99.5% or 99.9% identical to SEQ ID NO:1 or wherein the bacterial strain has a 16s rRNA gene sequence represented by SEQ ID NO:1.

11. The composition for use of any preceding claim, wherein the composition is for oral administration.

12. The composition for use of any preceding claim, wherein the composition comprises one or more pharmaceutically acceptable excipients or carriers.

13. The composition for use of any preceding claim, wherein the bacterial strain is lyophilised.

14. A food product comprising the composition of any preceding claim, for the use of any preceding claim.

15. The composition for use according to any preceding claim, wherein the bacterial strain is of the *Megasphaera massiliensis* strain deposited under accession number NCIMB 42787.

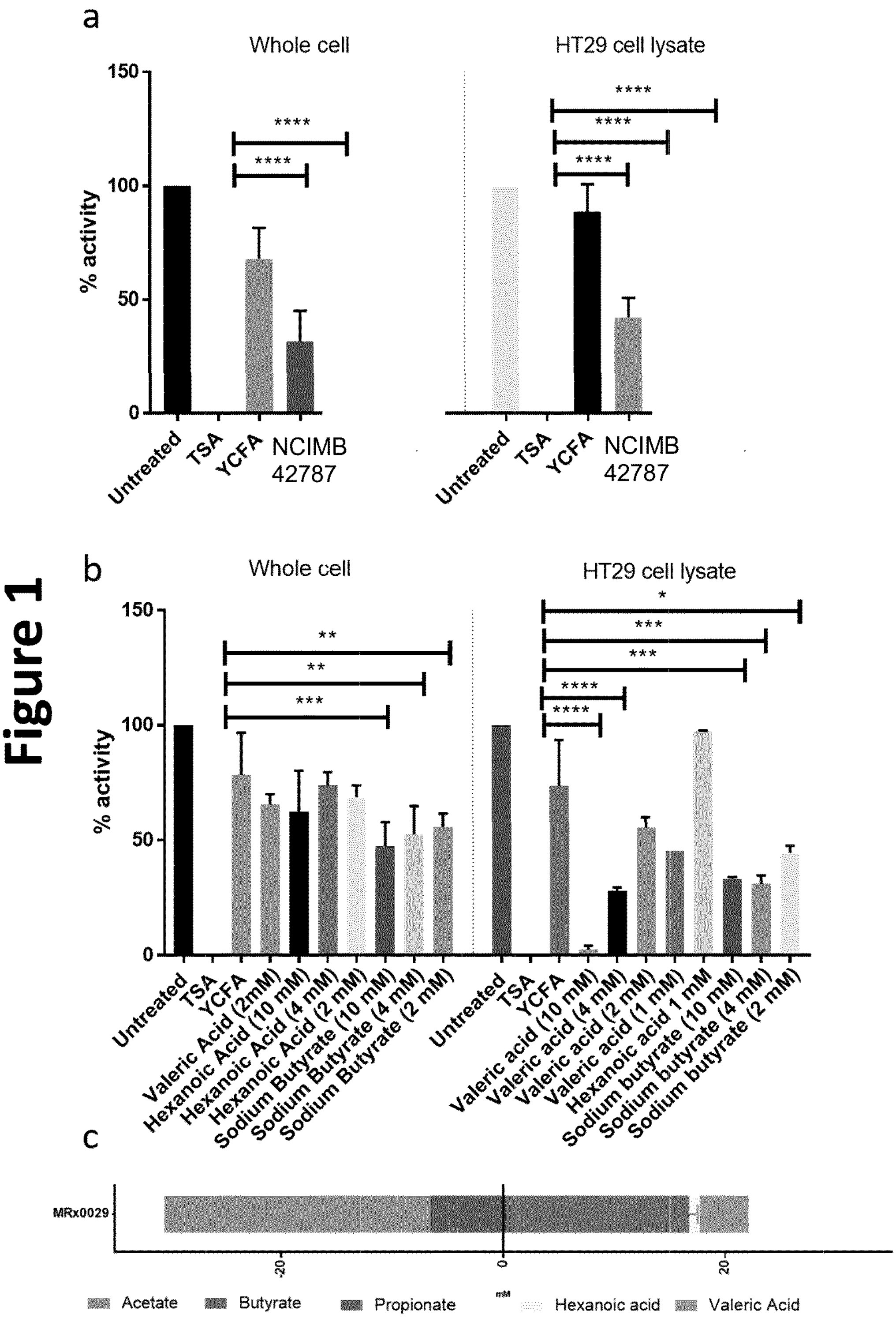
a
Whole cell
HT29 cell lysate
150
100
50
0
% activity
****
****
****
****
****
Untreated
TSA
YCFA
NCIMB
42787
b
Whole cell
HT29 cell lysate
**
**
***
*
***
***
****
****
Untreated
TSA
YCFA
Valeric Acid (2mM)
Hexanoic Acid (10 mM)
Hexanoic Acid (4 mM)
Hexanoic Acid (2 mM)
Sodium Butyrate (10 mM)
Sodium Butyrate (4 mM)
Sodium Butyrate (2 mM)
Valeric acid (10 mM)
Valeric acid (4 mM)
Valeric acid (2 mM)
Valeric acid (1 mM)
Hexanoic acid 1 mM
Sodium butyrate (10 mM)
Sodium butyrate (4 mM)
Sodium butyrate (2 mM)
c
MRx0029
-20
0
20
mM
Acetate
Butyrate
Propionate
Hexanoic acid
Valeric Acid

Figure 1

Figure 2

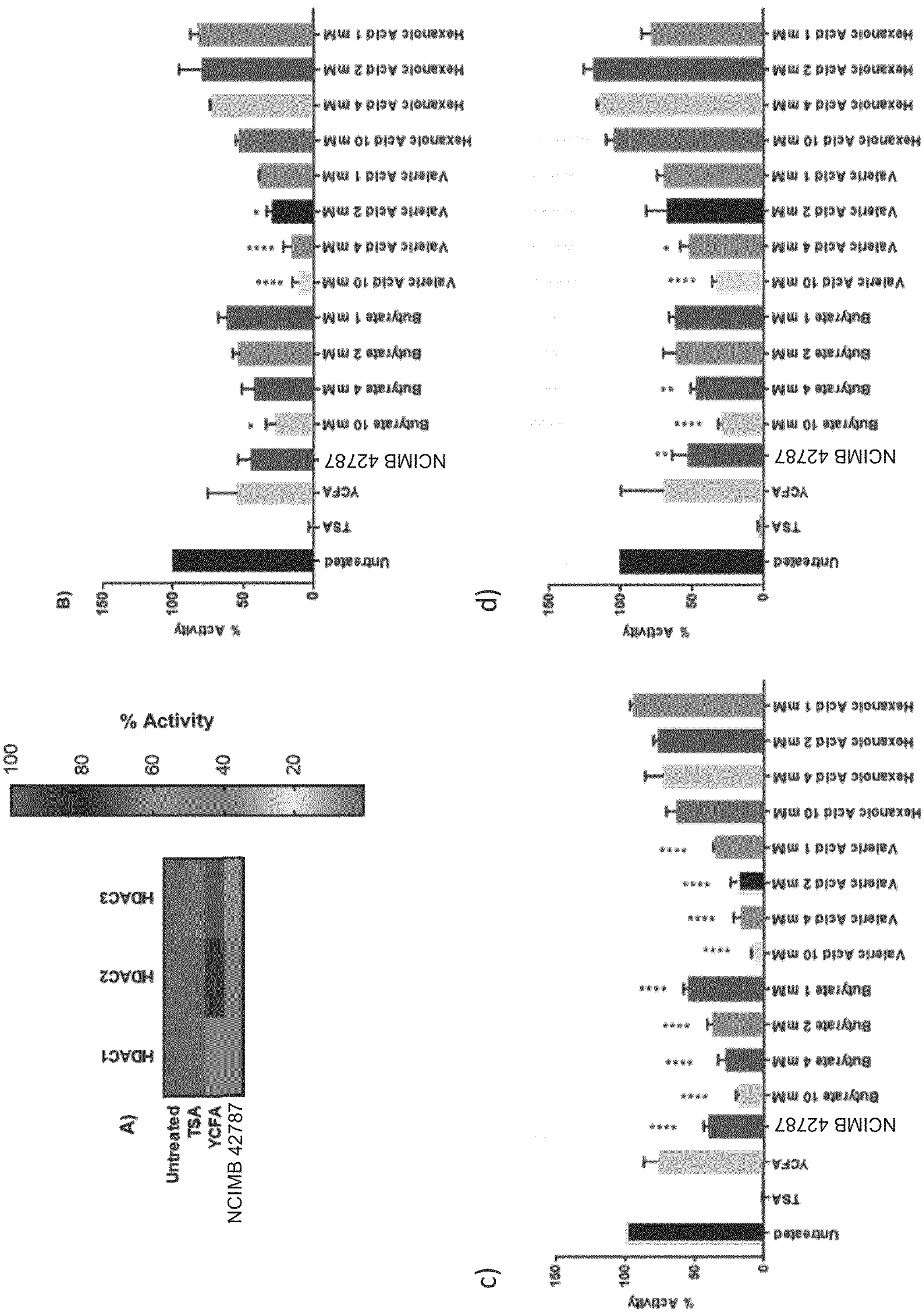
A)
Untreated
TSA
YCFA
NCIMB 42787
HDAC1
HDAC2
HDAC3
% Activity
100
80
60
40
20
B)
c)
d)
% Activity
150
100
50
0
Untreated
TSA
YCFA
NCIMB 42787
Butyrate 10 mM
Butyrate 4 mM
Butyrate 2 mM
Butyrate 1 mM
Valeric Acid 10 mM
Valeric Acid 4 mM
Valeric Acid 2 mM
Valeric Acid 1 mM
Hexanoic Acid 10 mM
Hexanoic Acid 4 mM
Hexanoic Acid 2 mM
Hexanoic Acid 1 mM

Figure 3

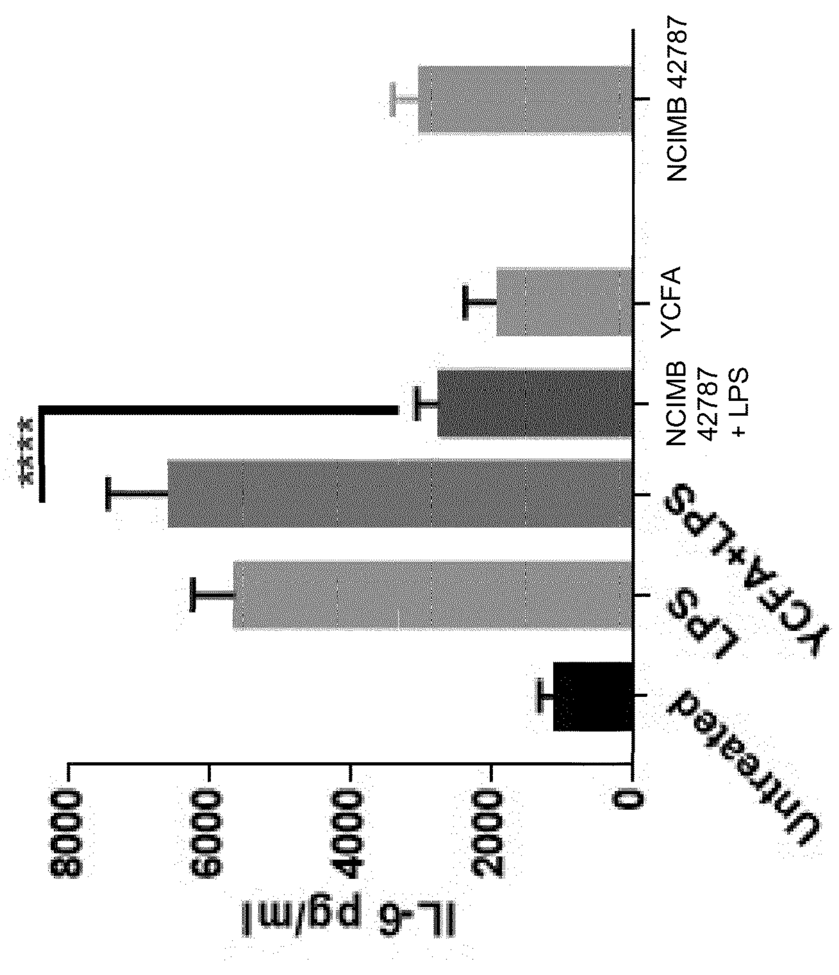
IL-6 pg/ml
8000
6000
4000
2000
0
Untreated
LPS
YCFA+LPS
NCIMB 42787 + LPS
YCFA
NCIMB 42787
****

# Figure 4

Inhibition of LPS-induced NFkB-AP1 activation in HEK-TLR4

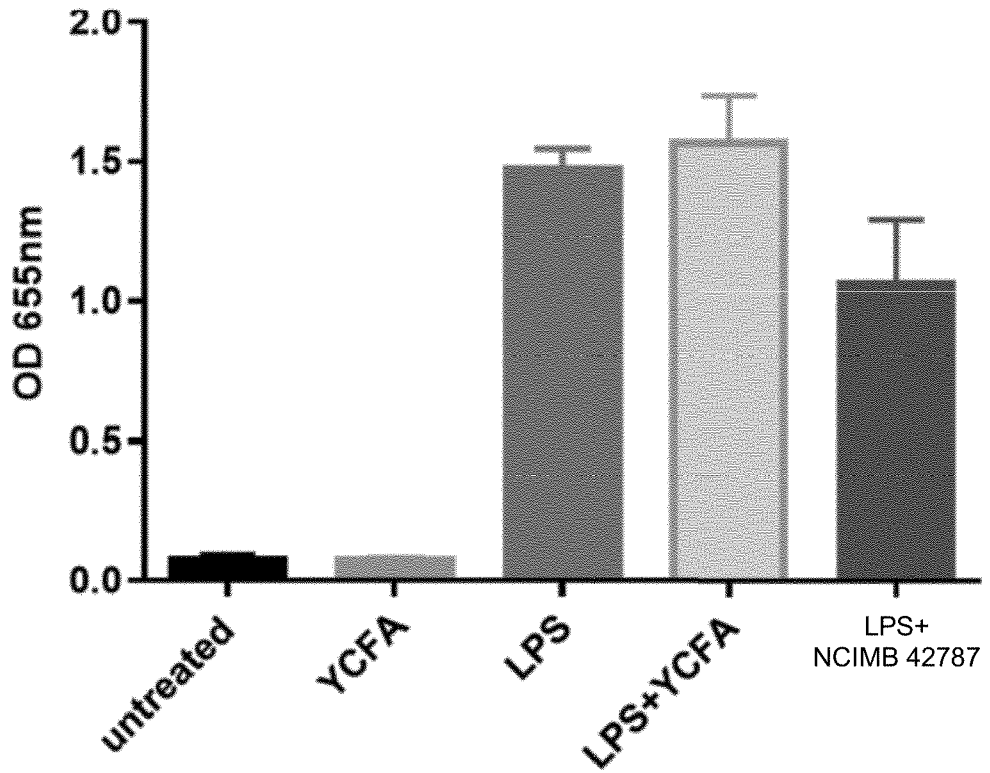

Figure 5

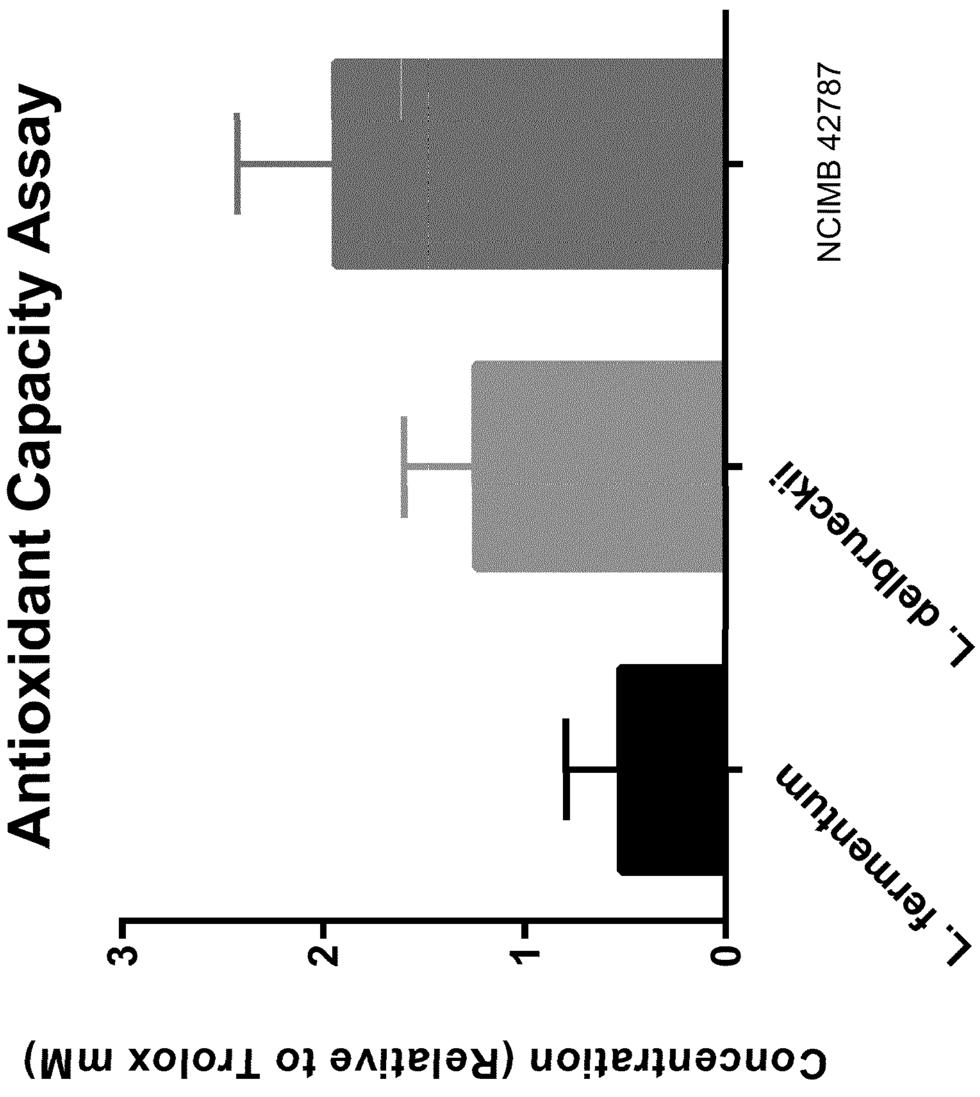
Antioxidant Capacity Assay
Concentration (Relative to Trolox mM)
0
1
2
3
L. fermentum
L. delbrueckii
NCIMB 42787

Figure 6

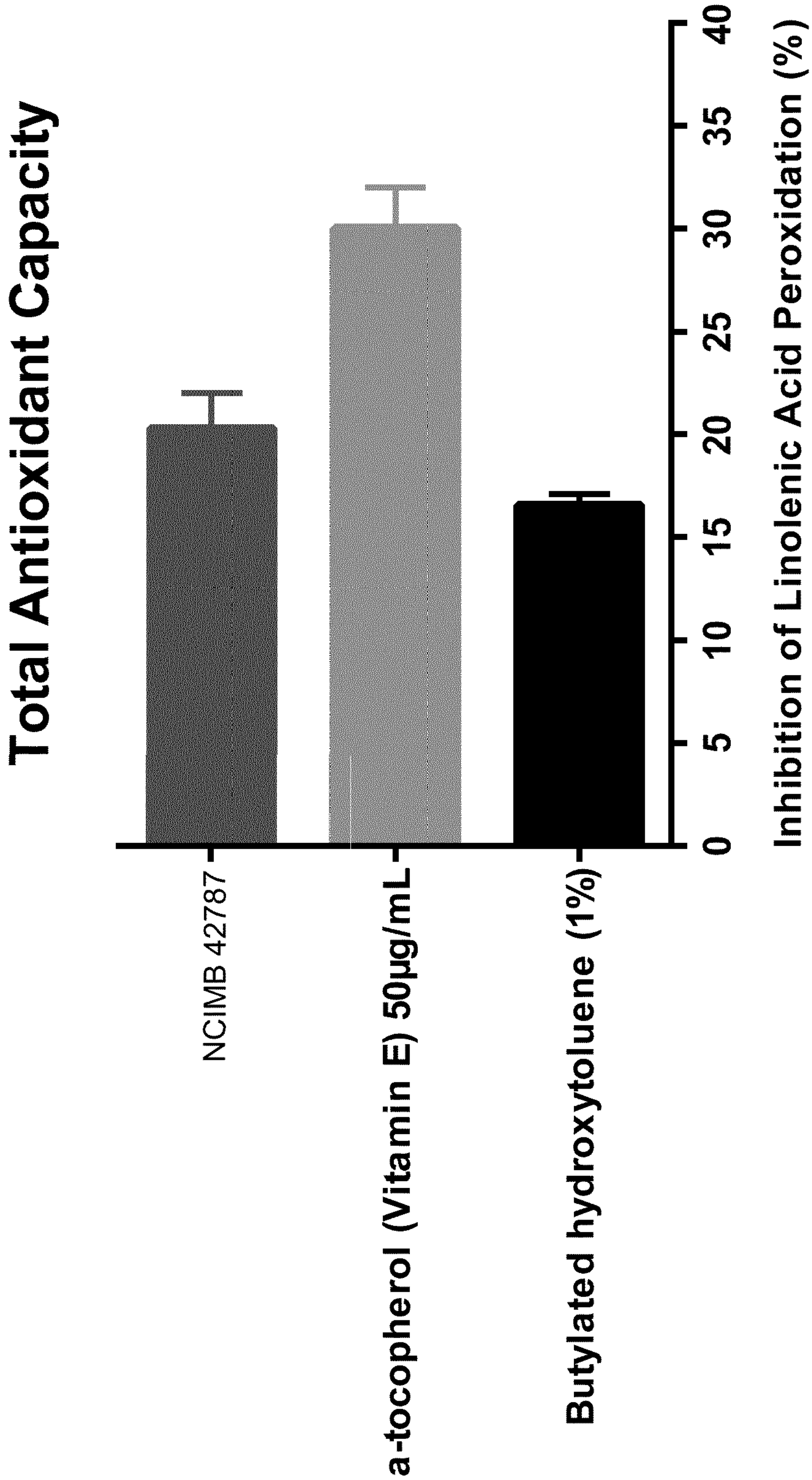
Total Antioxidant Capacity
NCIMB 42787
a-tocopherol (Vitamin E) 50µg/mL
Butylated hydroxytoluene (1%)
0
5
10
15
20
25
30
35
40
Inhibition of Linolenic Acid Peroxidation (%)

Figure 7
Indole Assay

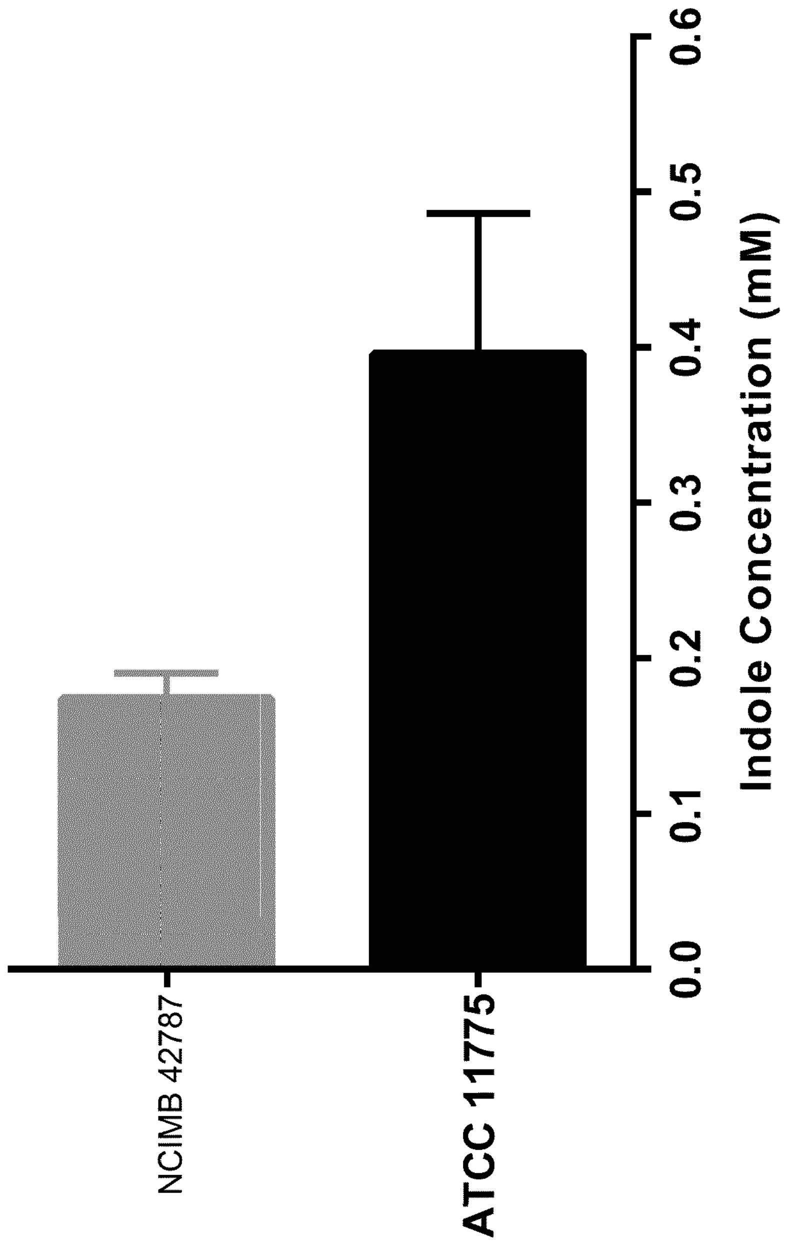

Figure 8

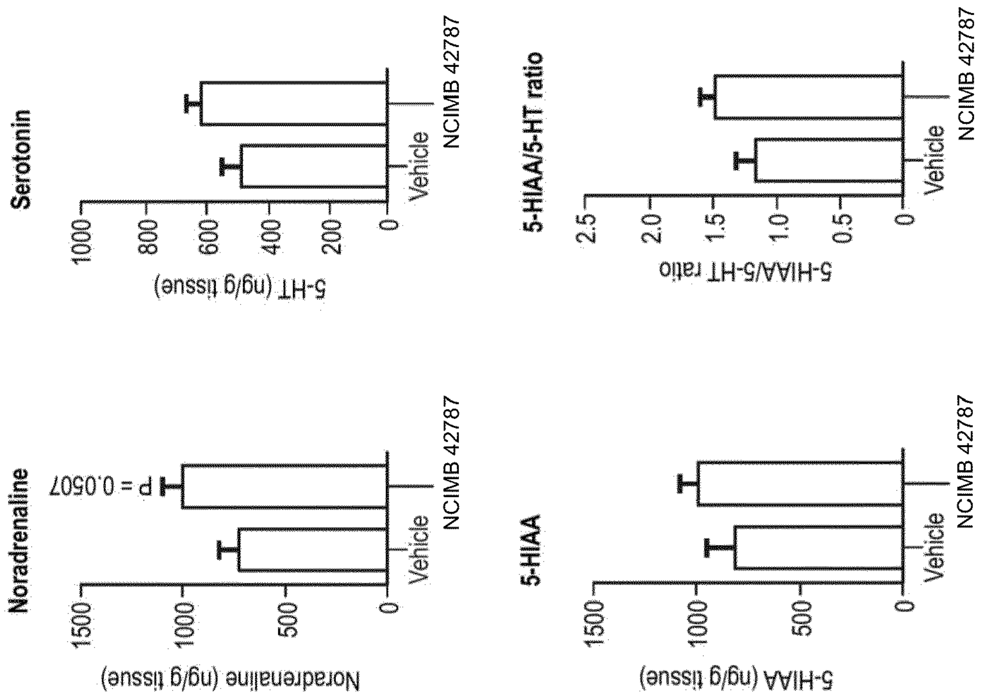
Noradrenaline
Noradrenaline (ng/g tissue)
1500
1000
500
0
Vehicle
NCIMB 42787
P = 0.0507
Serotonin
5-HT (ng/g tissue)
1000
800
600
400
200
0
Vehicle
NCIMB 42787
5-HIAA
5-HIAA (ng/g tissue)
1500
1000
500
0
Vehicle
NCIMB 42787
5-HIAA/5-HT ratio
5-HIAA/5-HT ratio
2.5
2.0
1.5
1.0
0.5
0
Vehicle
NCIMB 42787

Figure 9

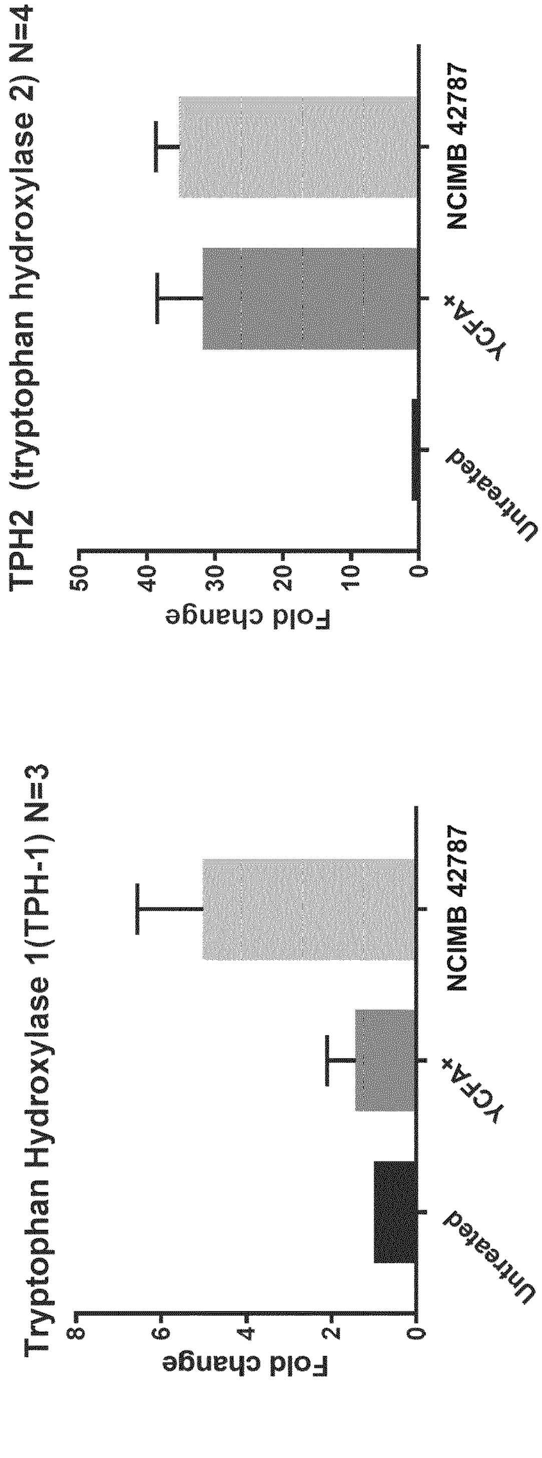

Tryptophan Hydroxylase 1(TPH-1) N=3
Fold change
0
2
4
6
8
Untreated
YCFA+
NCIMB 42787
TPH2 (tryptophan hydroxylase 2) N=4
Fold change
0
10
20
30
40
50
Untreated
YCFA+
NCIMB 42787

Figure 10

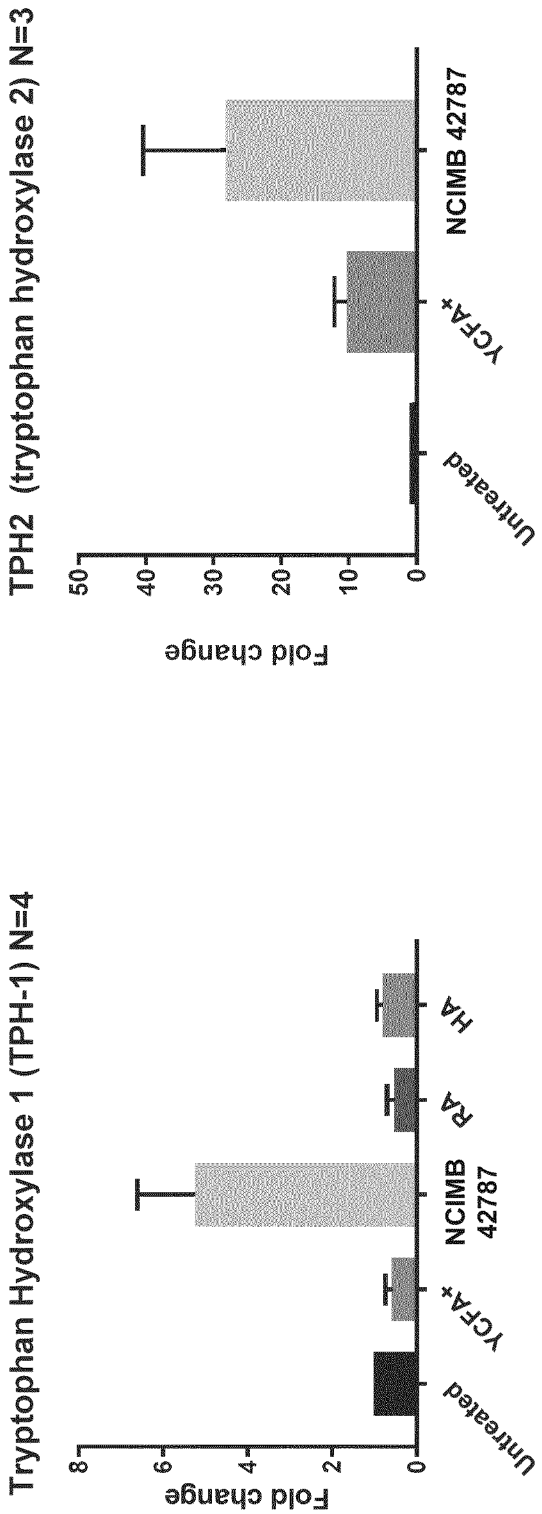
TPH2 (tryptophan hydroxylase 2) N=3
Fold change
0
10
20
30
40
50
Untreated
YCFA+
NCIMB 42787
Tryptophan Hydroxylase 1 (TPH-1) N=4
Fold change
0
2
4
6
8
Untreated
YCFA+
NCIMB 42787
RA
HA

Figure 11

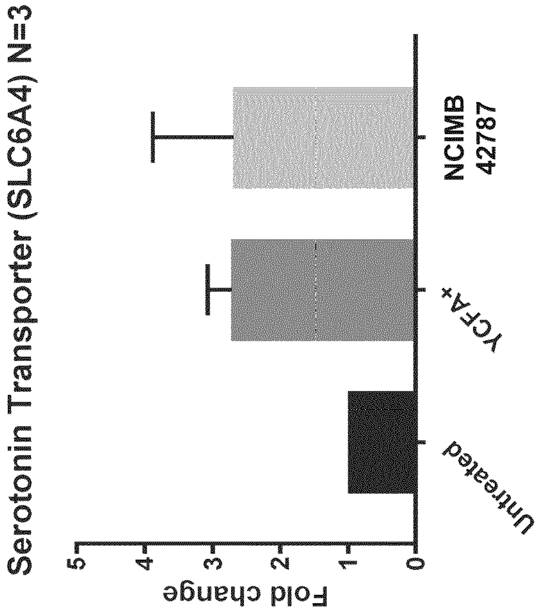
Serotonin Transporter (SLC6A4) N=3
Fold change
0
1
2
3
4
5
Untreated
YCFA+
NCIMB
42787

Figure 12

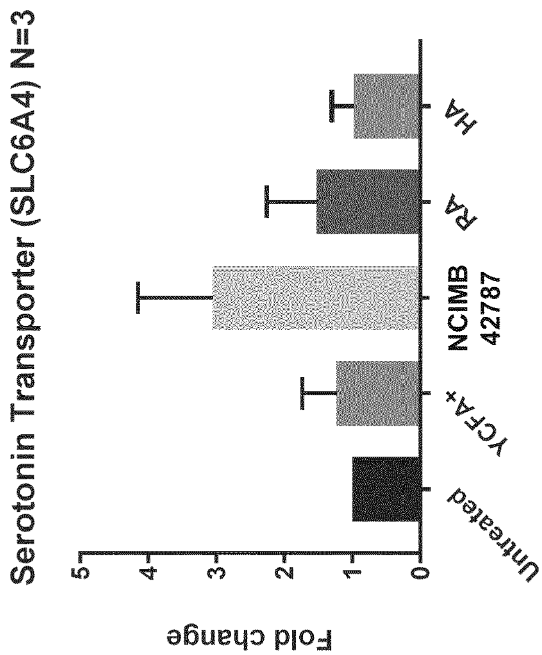
Serotonin Transporter (SLC6A4) N=3
Fold change
0
1
2
3
4
5
Untreated
YCFA+
NCIMB 42787
RA
HA

Figure 13

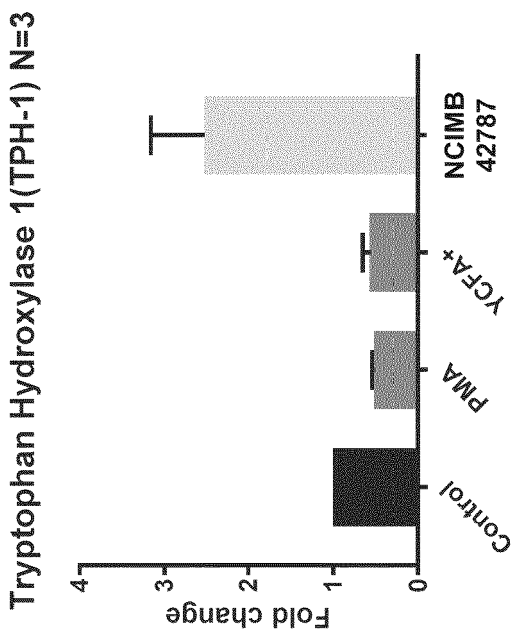
Tryptophan Hydroxylase 1(TPH-1) N=3
Fold change
4
3
2
1
0
Control
PMA
YCFA+
NCIMB 42787

Figure 14

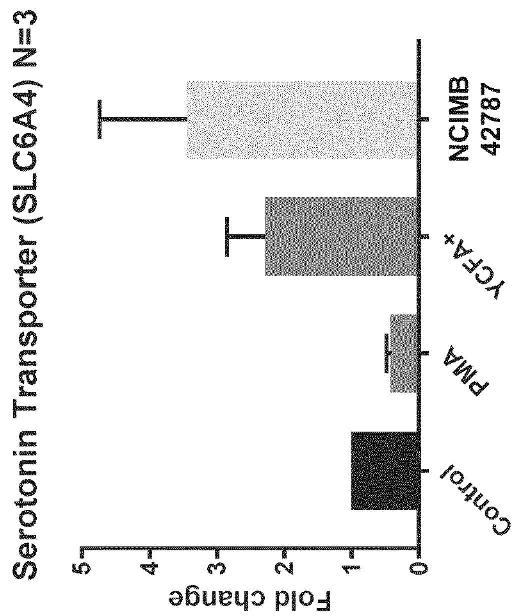
Serotonin Transporter (SLC6A4) N=3
Fold change
0
1
2
3
4
5
Control
PMA
YCFA+
NCIMB 42787

Figure 15

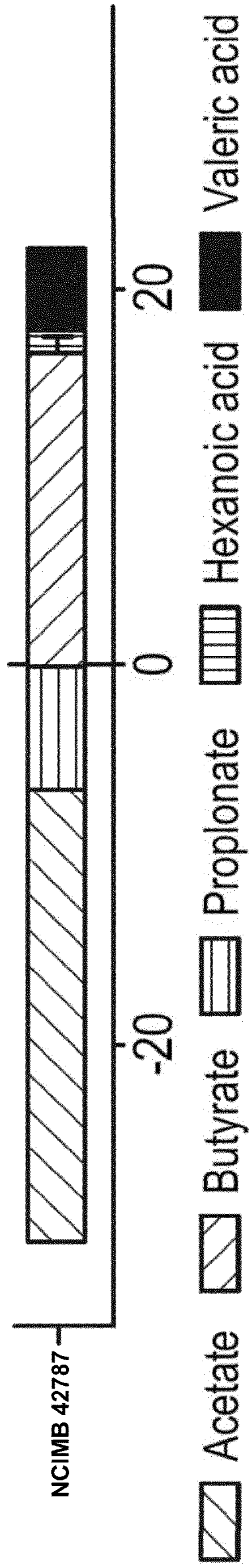

NCIMB 42787
-20
0
20
Acetate
Butyrate
Proplonate
Hexanoic acid
Valeric acid

Figure 16

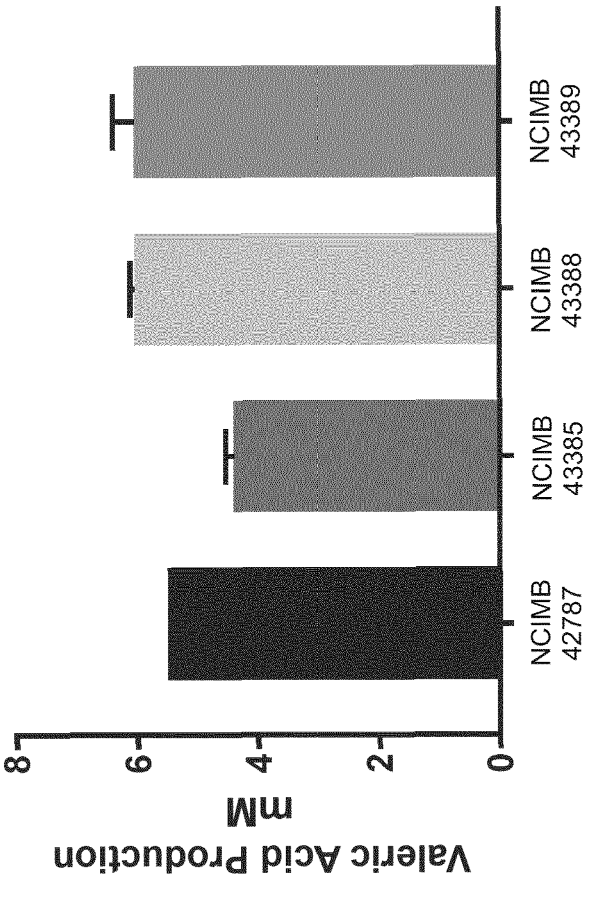
Valeric Acid Production mM
0
2
4
6
8
NCIMB 42787
NCIMB 43385
NCIMB 43388
NCIMB 43389

Figure 17

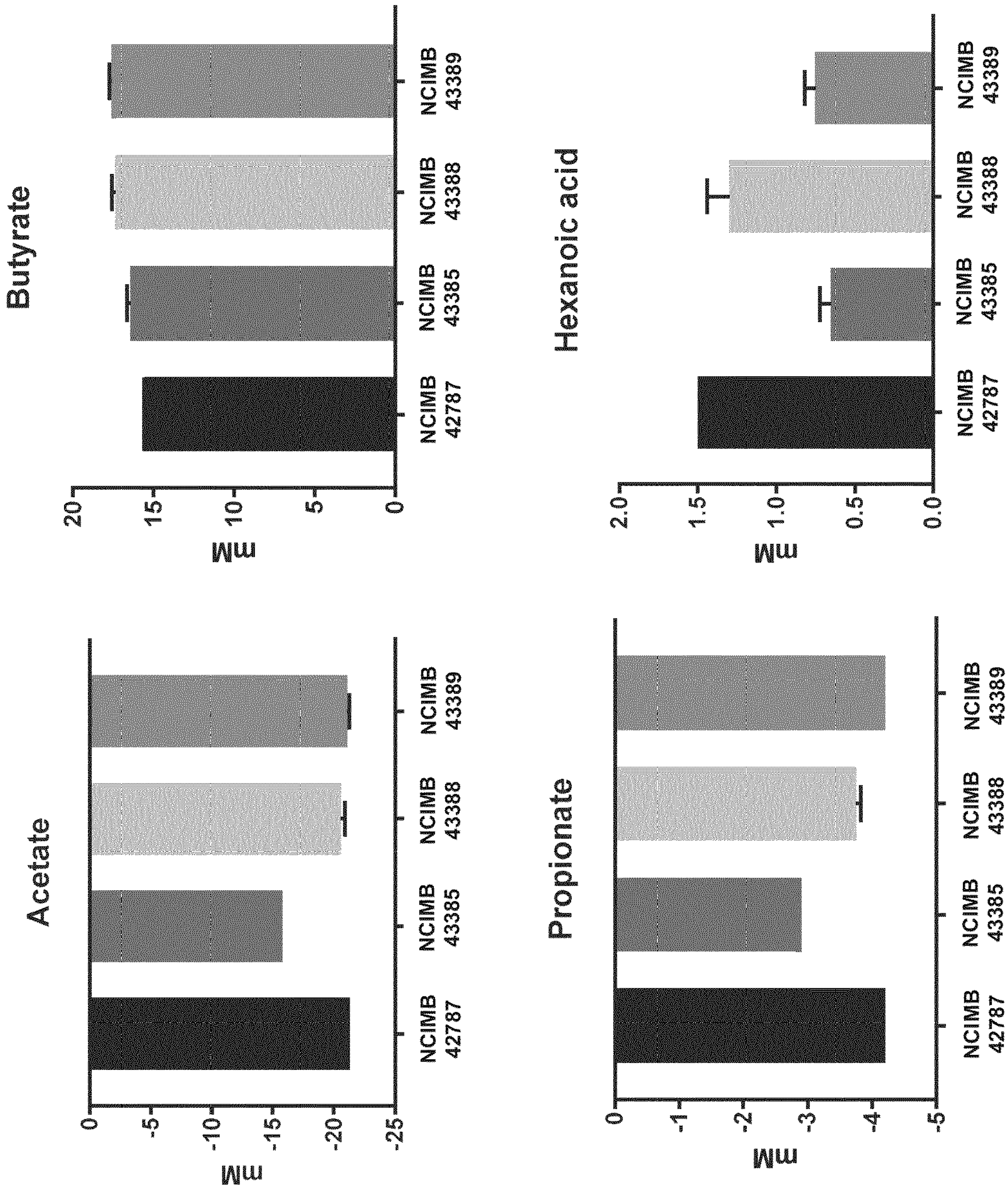
Acetate
Butyrate
Propionate
Hexanoic acid
mM
NCIMB 42787
NCIMB 43385
NCIMB 43388
NCIMB 43389
0
-5
-10
-15
-20
-25
20
15
10
5
-1
-2
-3
-4
2.0
1.5
1.0
0.5
0.0

Figure 18

Caco2 cells – GPR109a expression

A

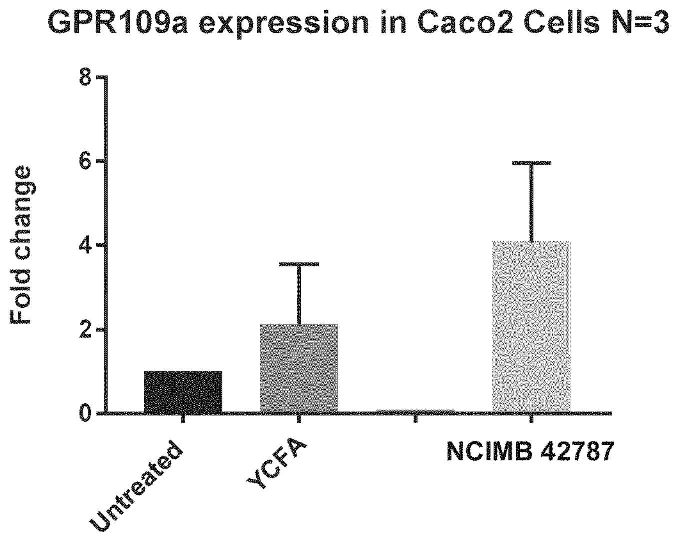


B

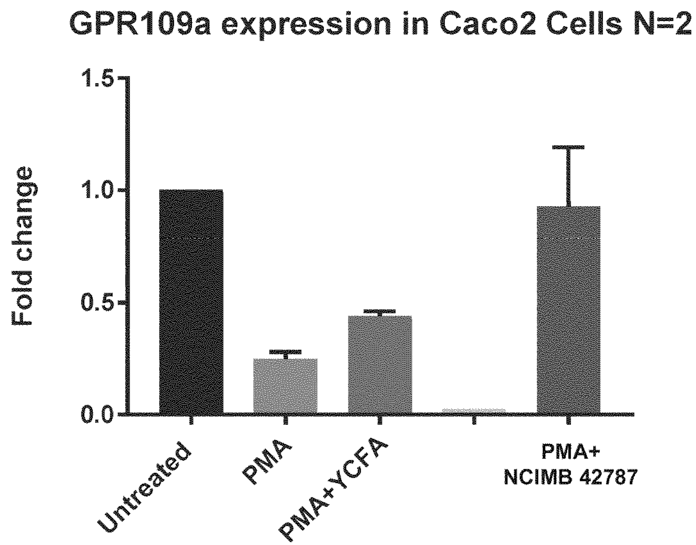

Figure 19

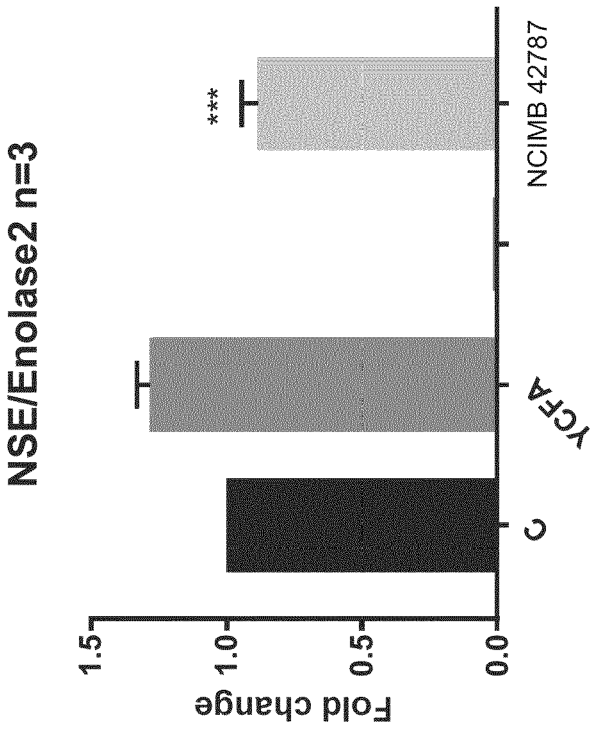

Figure 20

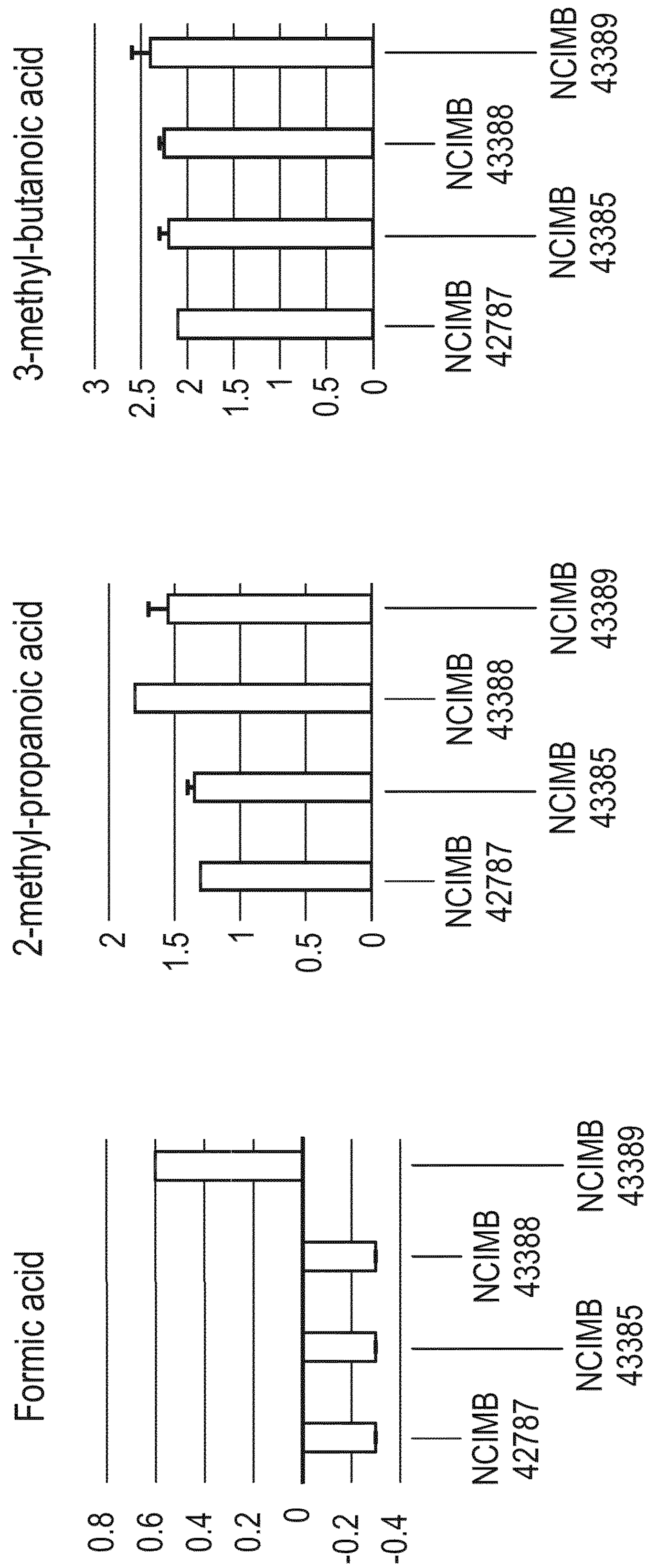

3-methyl-butanoic acid
3
2.5
2
1.5
1
0.5
0
NCIMB 42787
NCIMB 43385
NCIMB 43388
NCIMB 43389
2-methyl-propanoic acid
2
1.5
1
0.5
0
NCIMB 42787
NCIMB 43385
NCIMB 43388
NCIMB 43389
Formic acid
0.8
0.6
0.4
0.2
0
-0.2
-0.4
NCIMB 42787
NCIMB 43385
NCIMB 43388
NCIMB 43389

Figure 21

**IL-6 downregulation (with LPS)**

**secretion of IL-6 in U373 cells N=3**

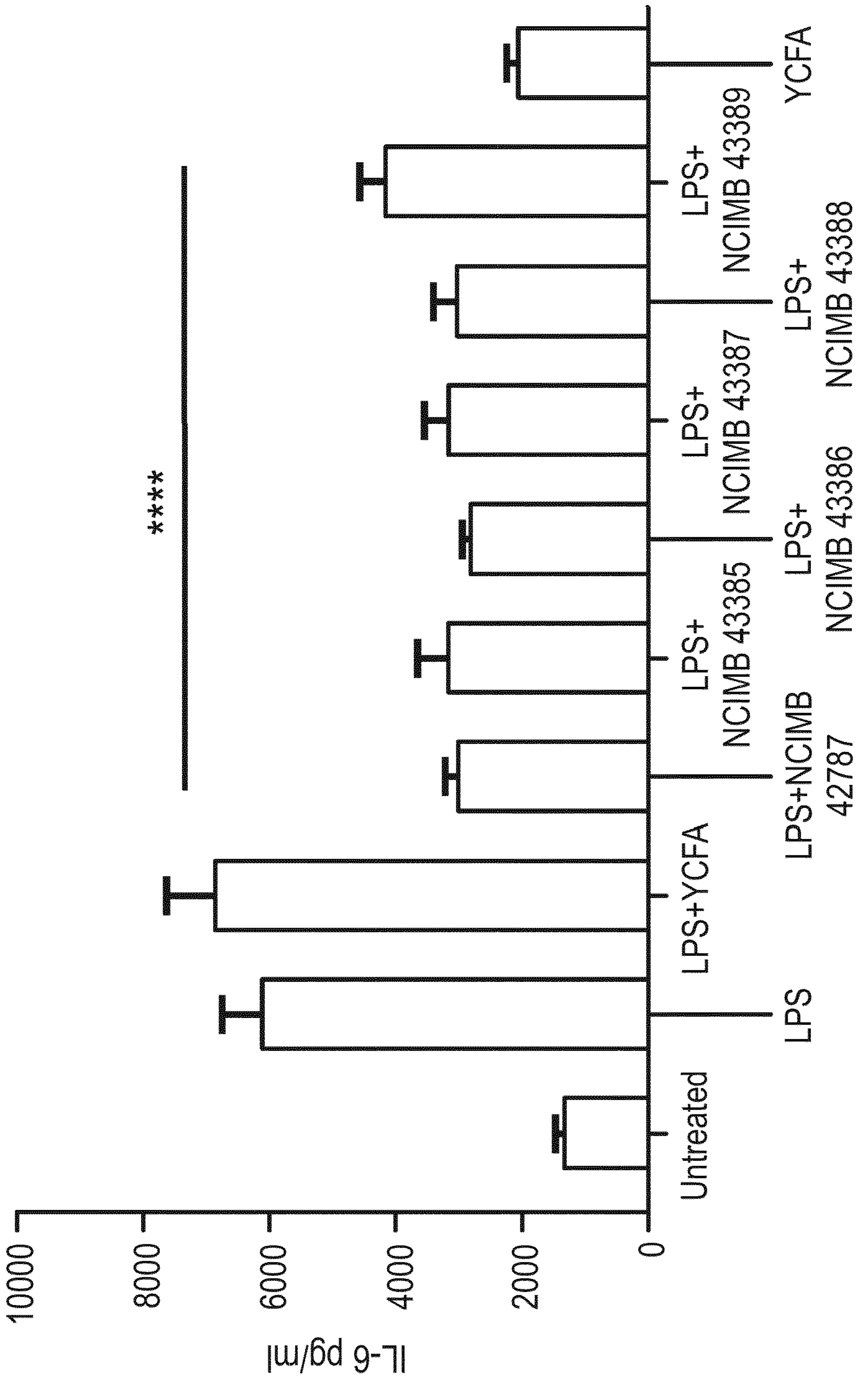

Figure 22

**Activation of NFkB-AP1 promoter in HEK-TLR4 N=3**

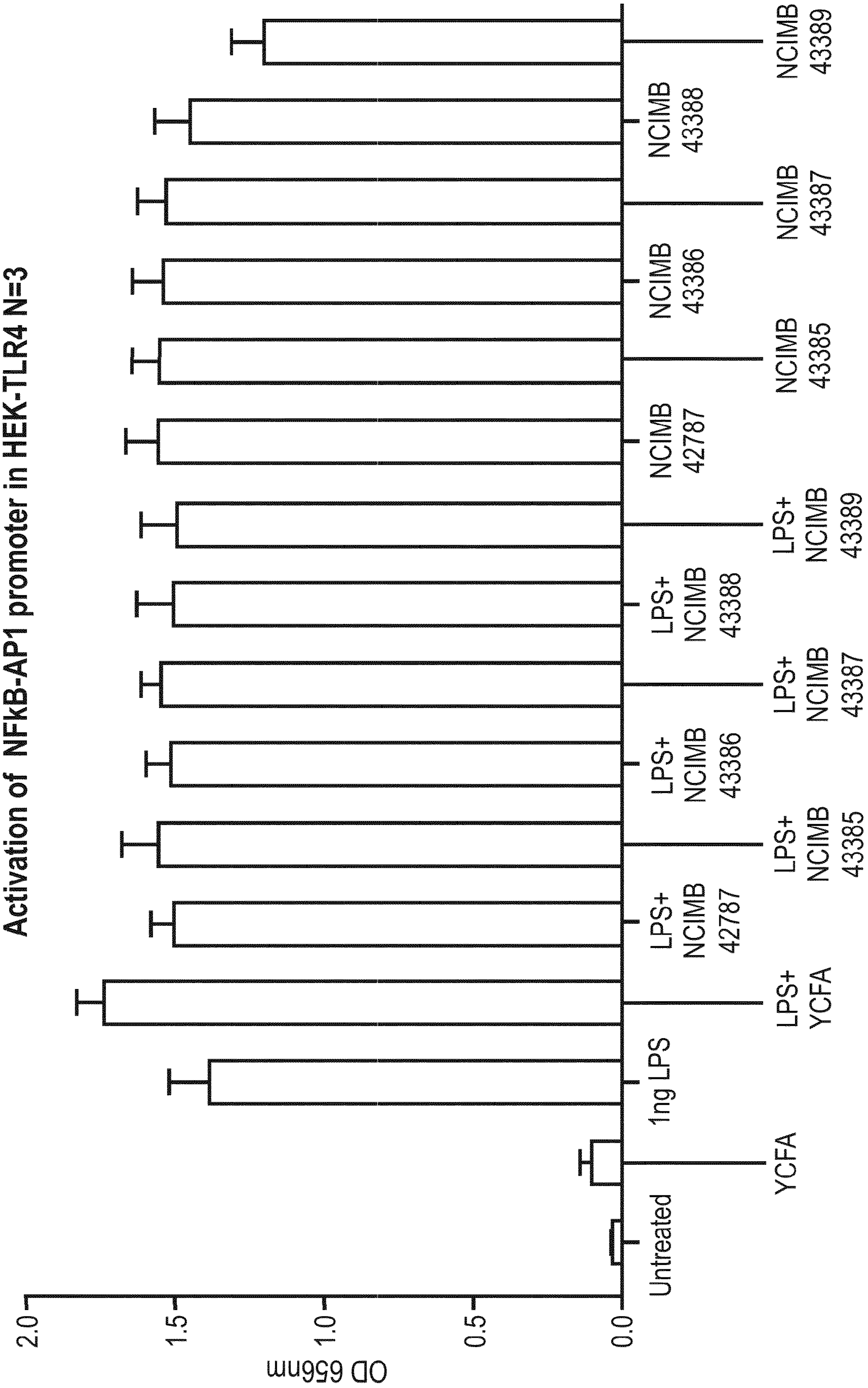

Figure 23

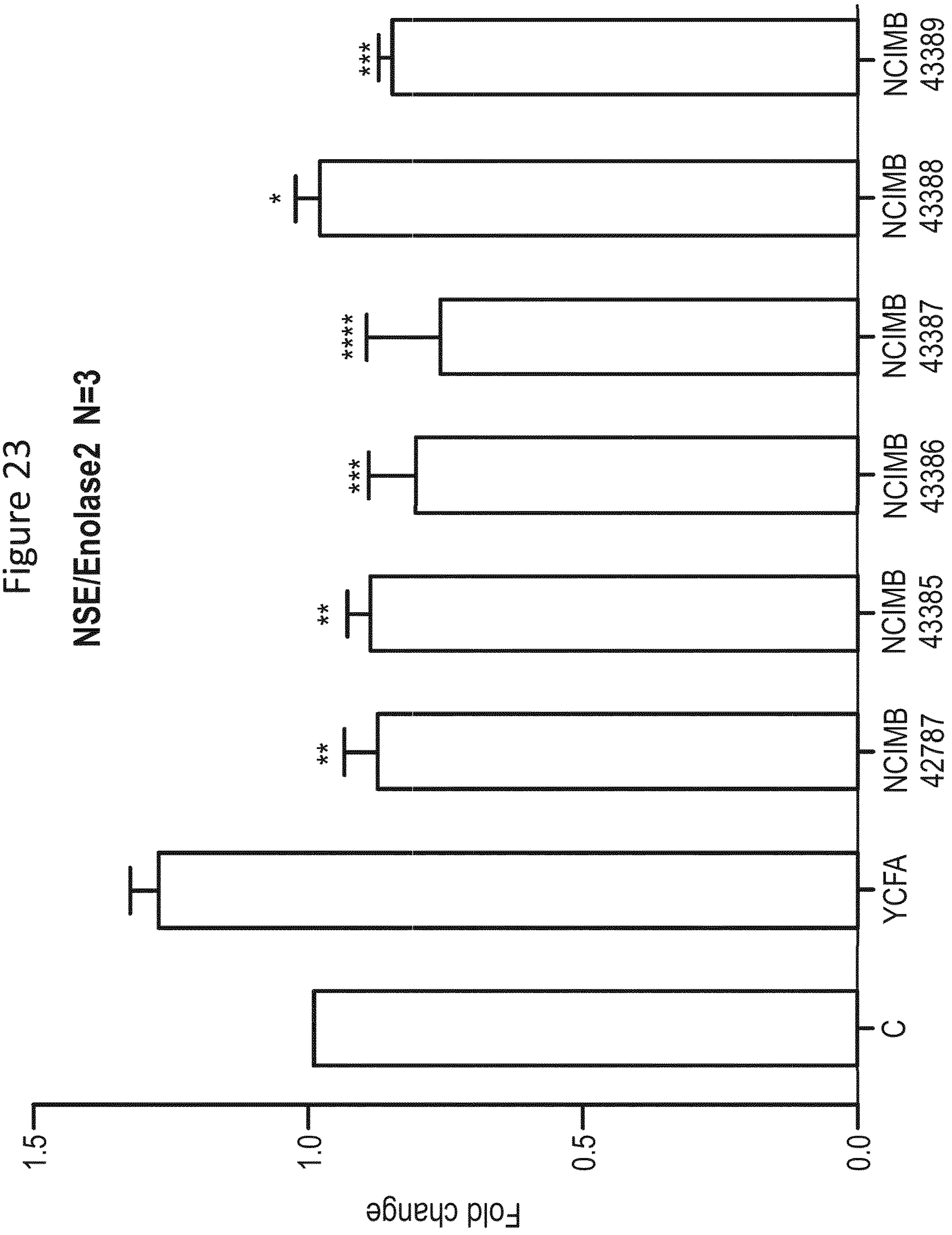
NSE/Enolase2 N=3
Fold change
0.0
0.5
1.0
1.5
C
YCFA
NCIMB 42787
NCIMB 43385
NCIMB 43386
NCIMB 43387
NCIMB 43388
NCIMB 43389
**
**
***
****
*
***

Figure 24A

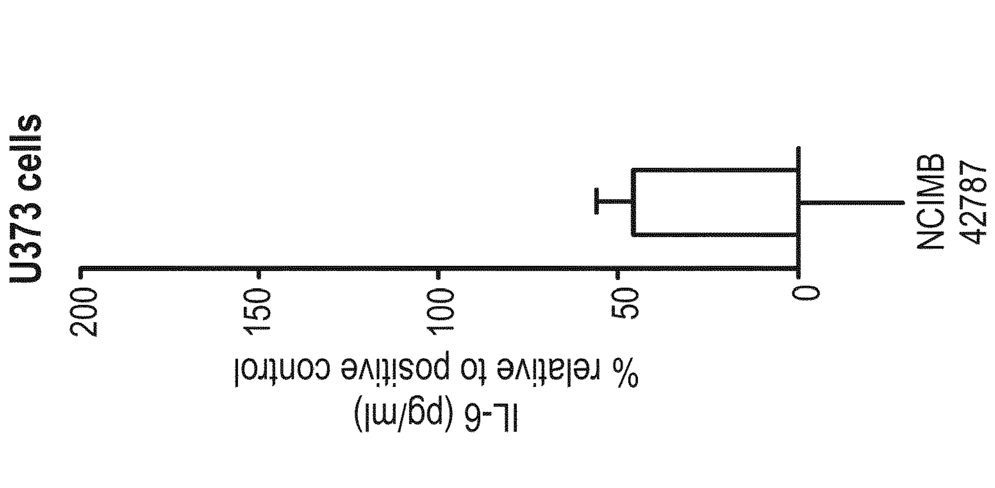


Figure 24B

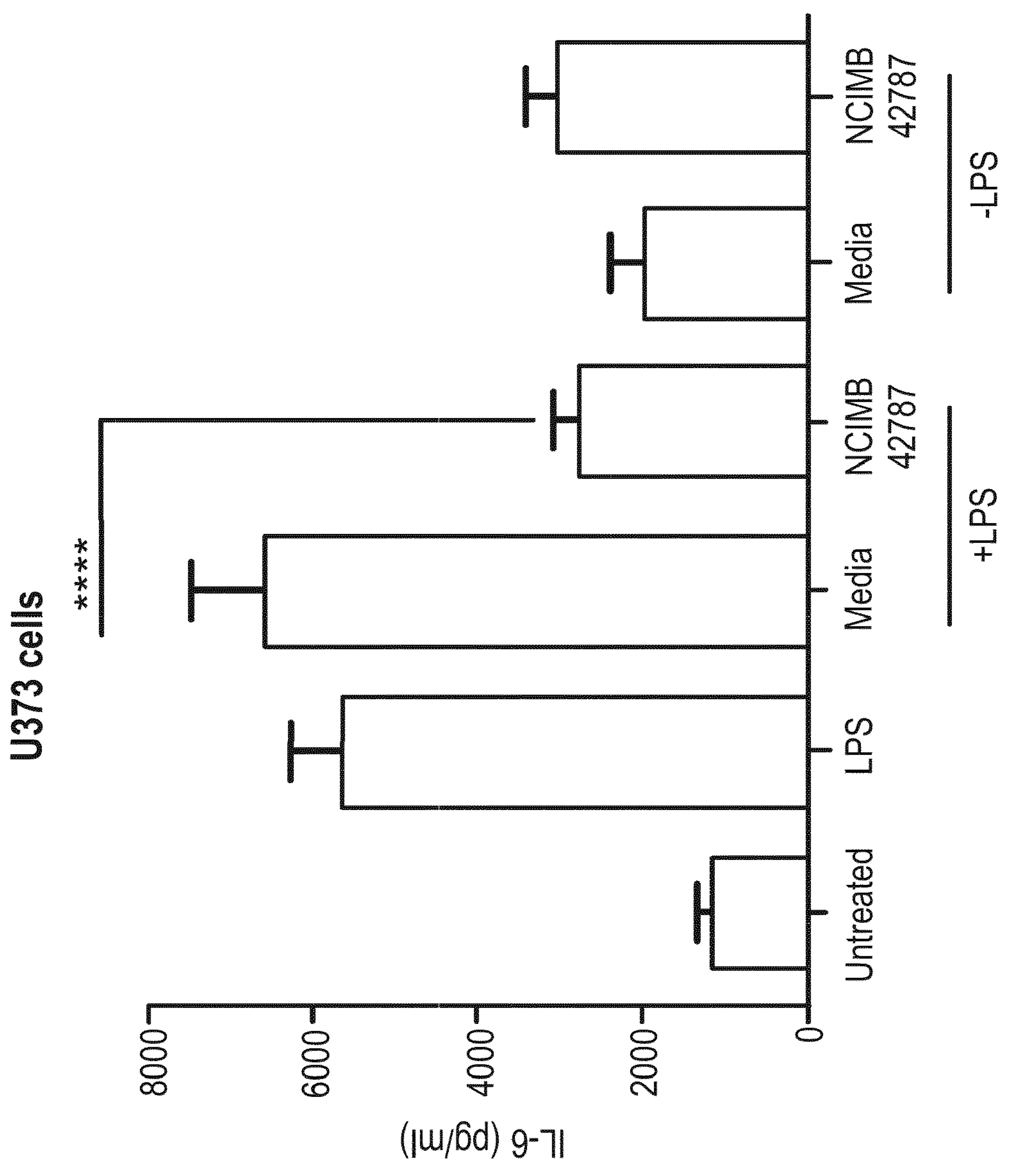

Figure 24C

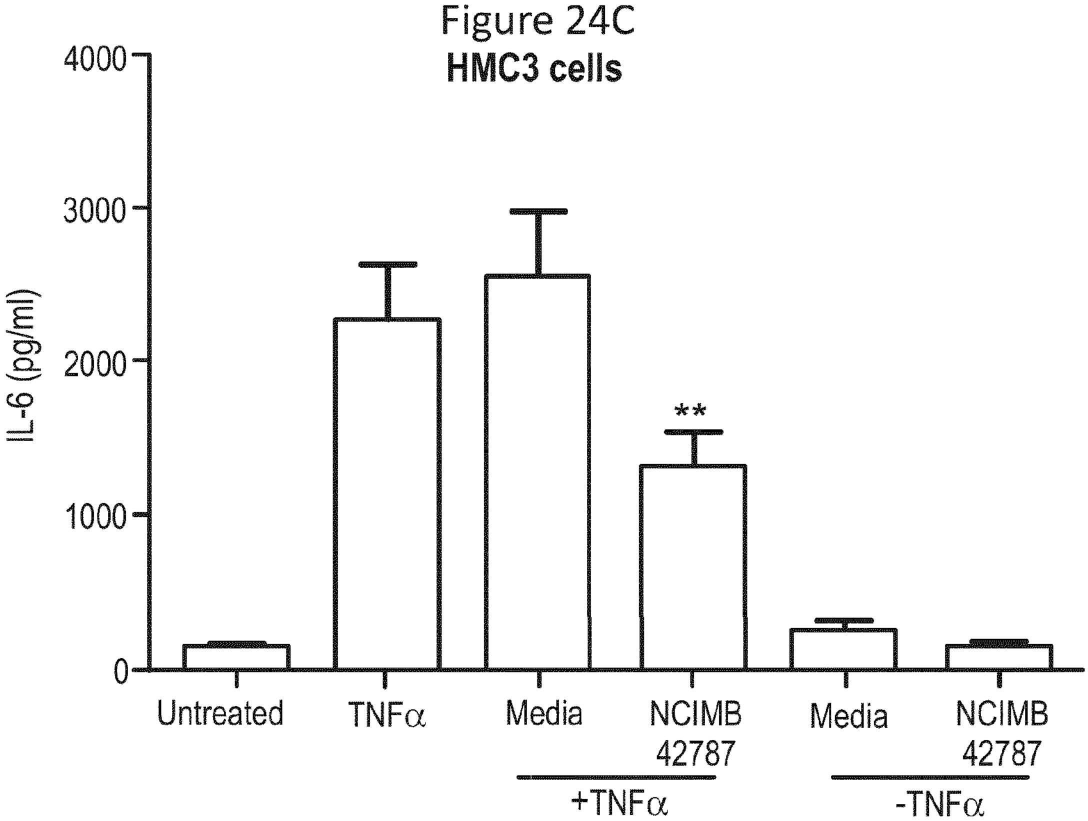


Figure 24D

HEK-TLR4 cells

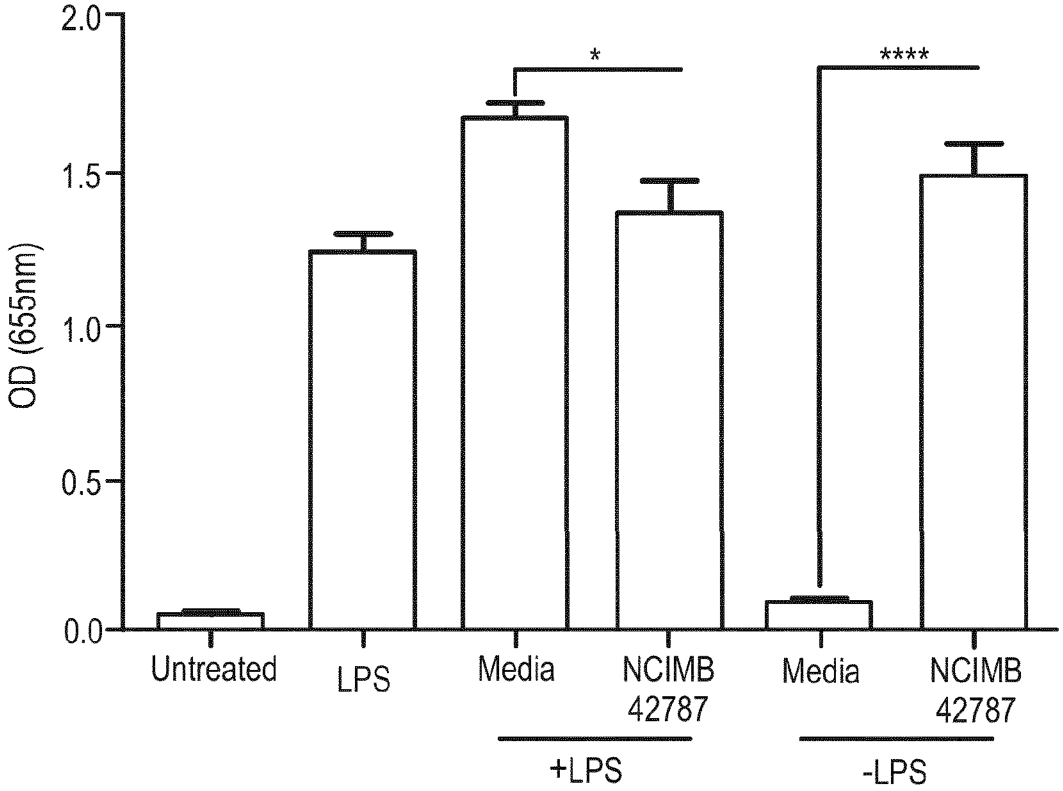

Figure 25

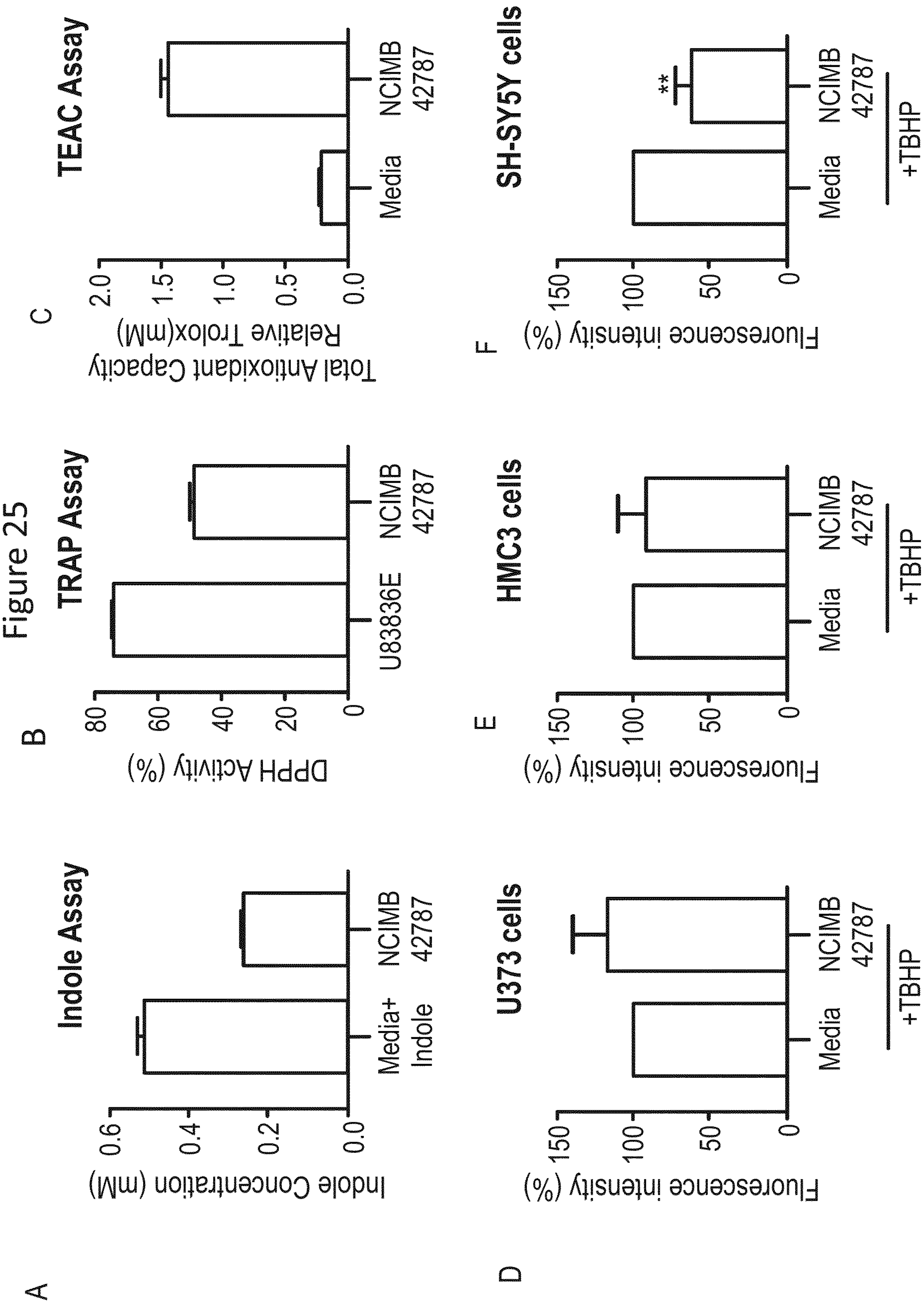
A
Indole Assay
Indole Concentration (mM)
0.0
0.2
0.4
0.6
Media+ Indole
NCIMB 42787
B
TRAP Assay
DPPH Activity (%)
0
20
40
60
80
U83836E
NCIMB 42787
C
TEAC Assay
Total Antioxidant Capacity Relative Trolox(mM)
0.0
0.5
1.0
1.5
2.0
Media
NCIMB 42787
D
U373 cells
Fluorescence intensity (%)
0
50
100
150
Media
NCIMB 42787
+TBHP
E
HMC3 cells
Fluorescence intensity (%)
0
50
100
150
Media
NCIMB 42787
+TBHP
F
SH-SY5Y cells
Fluorescence intensity (%)
0
50
100
150
**
Media
NCIMB 42787
+TBHP

Figure 26A

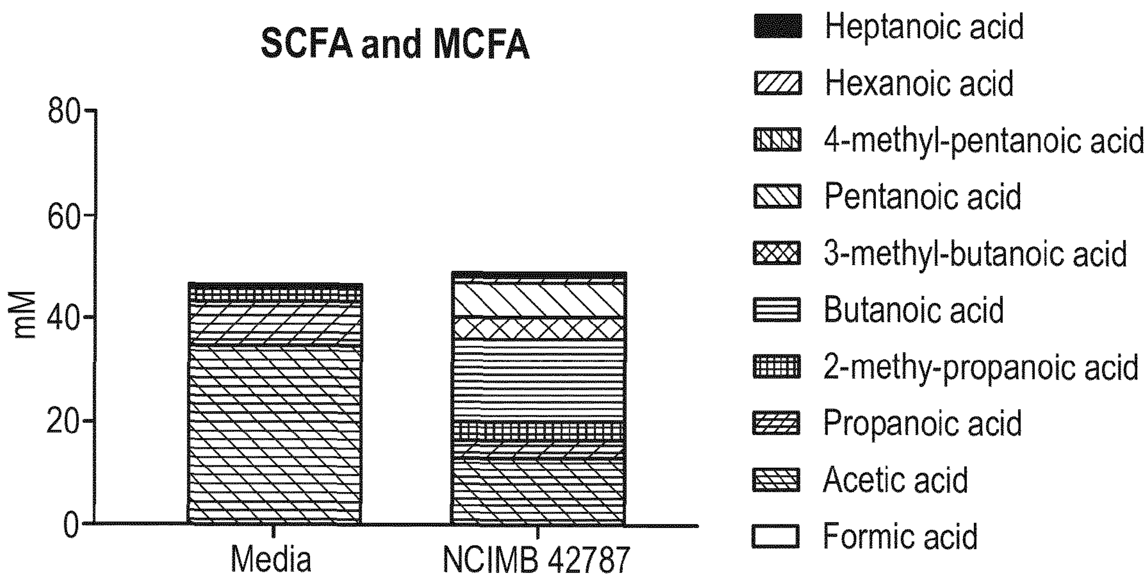


Figure 26B

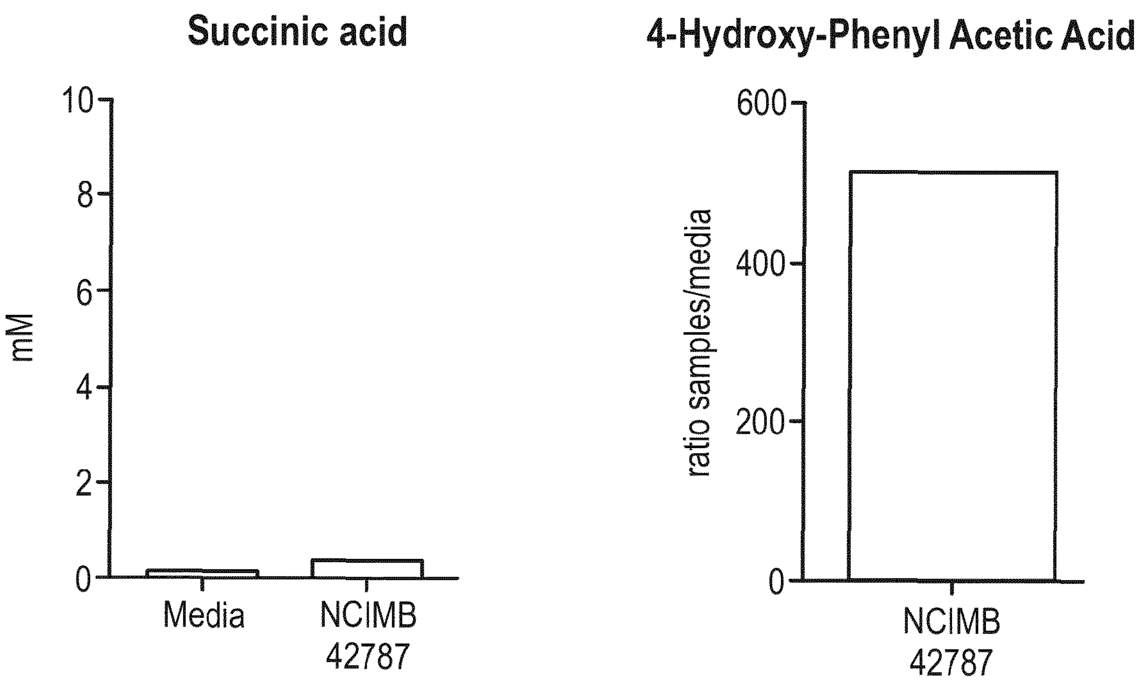

Figure 26C

**SCFA standards vs NCIMB 42787**

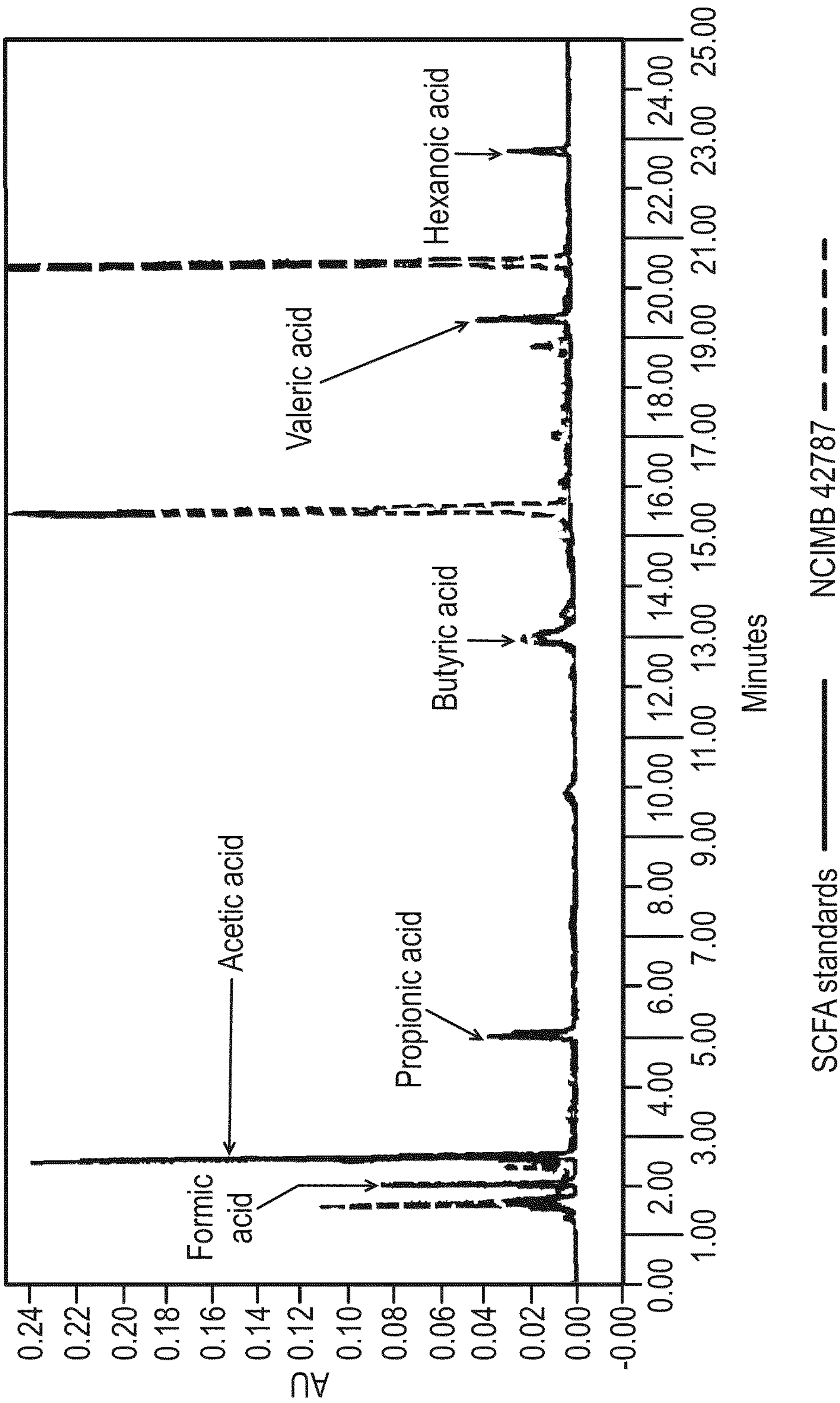

Figure 27

**U373 cells**

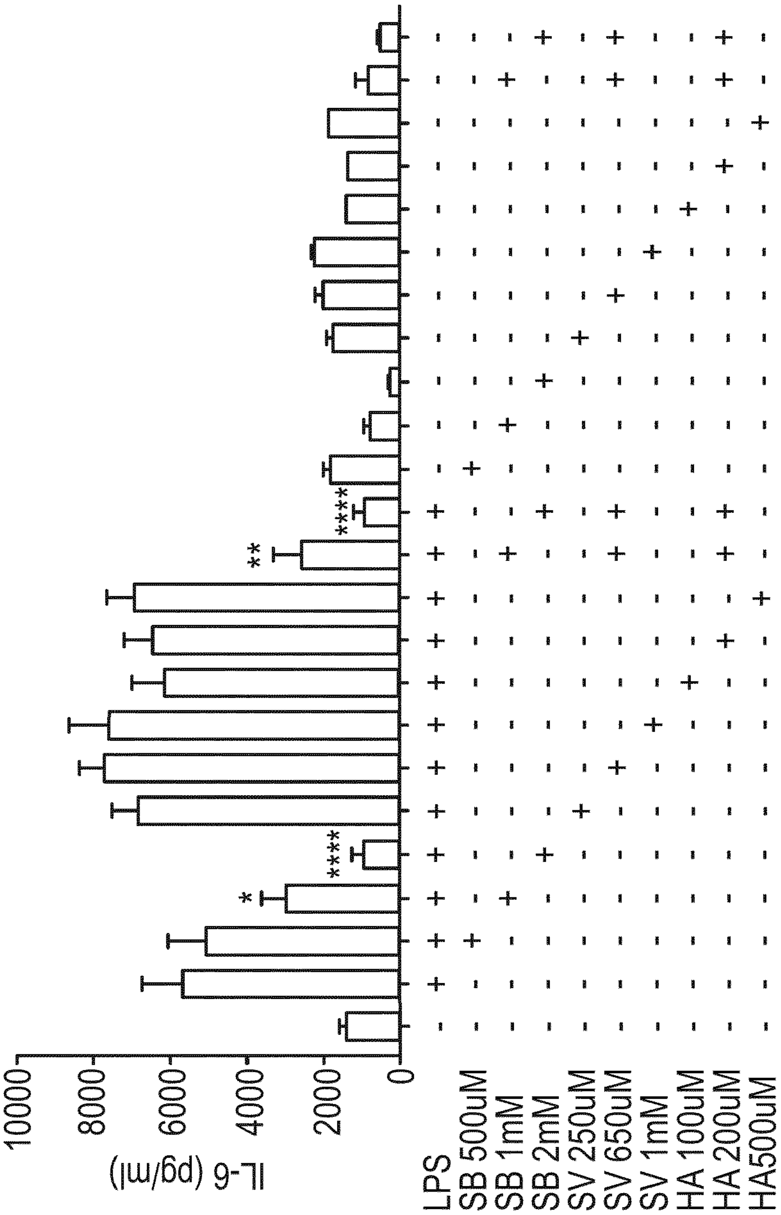

Figure 28A

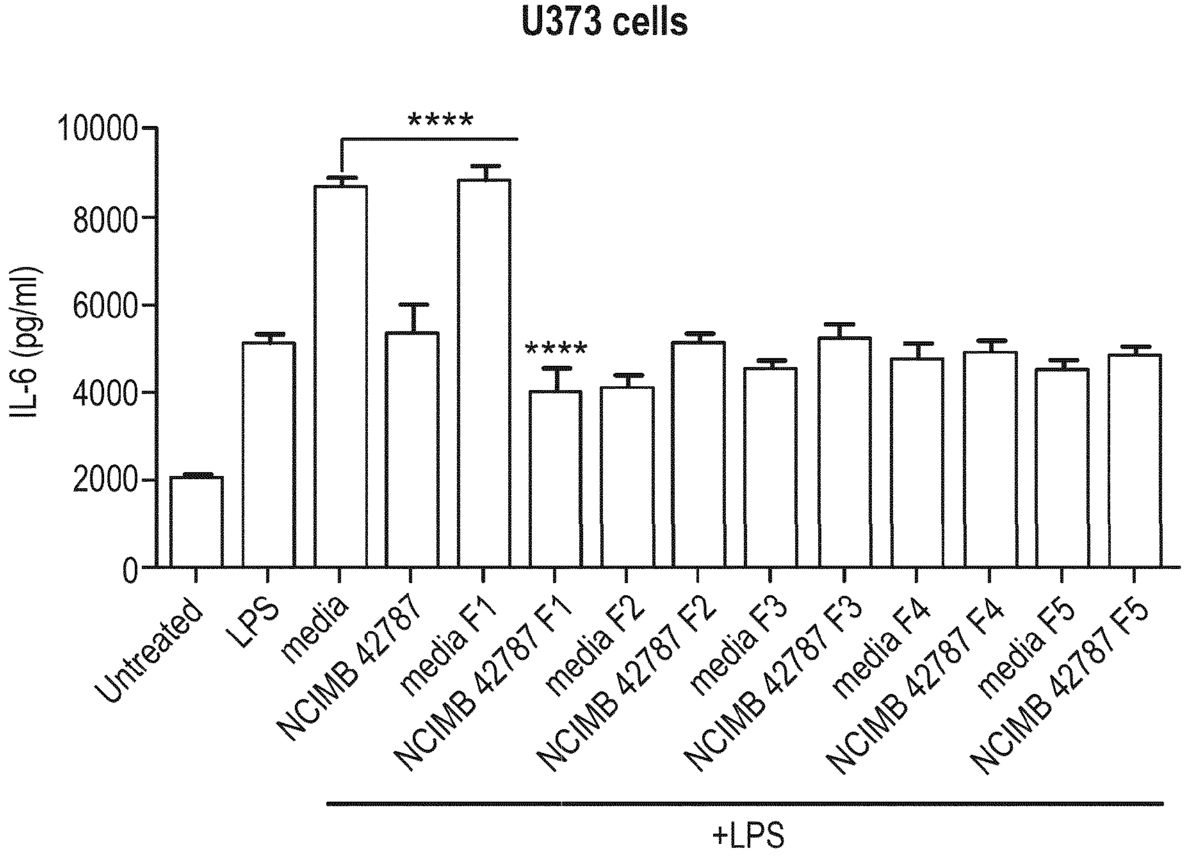


Figure 28B

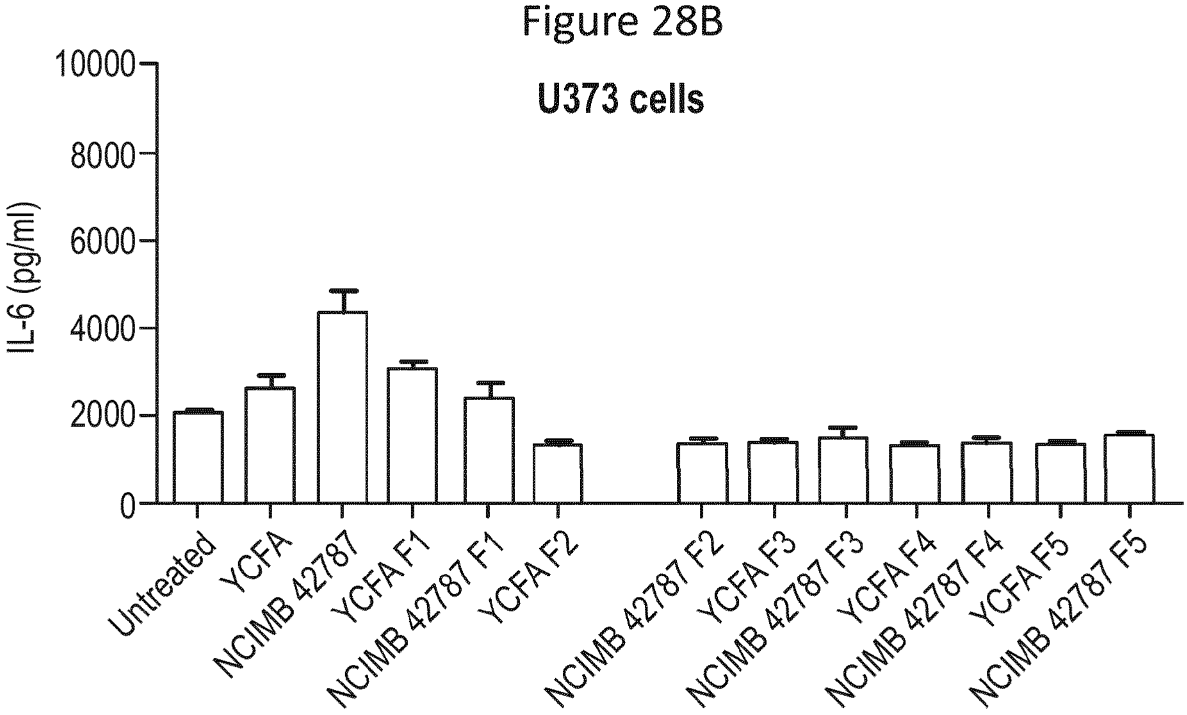

Figure 29

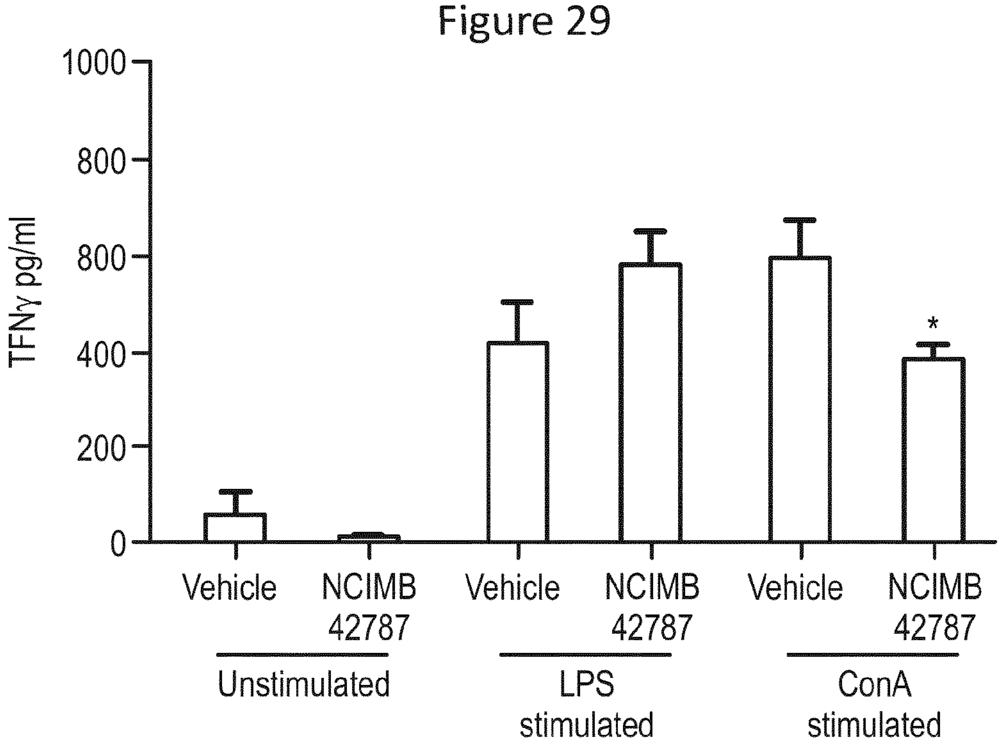


Figure 30

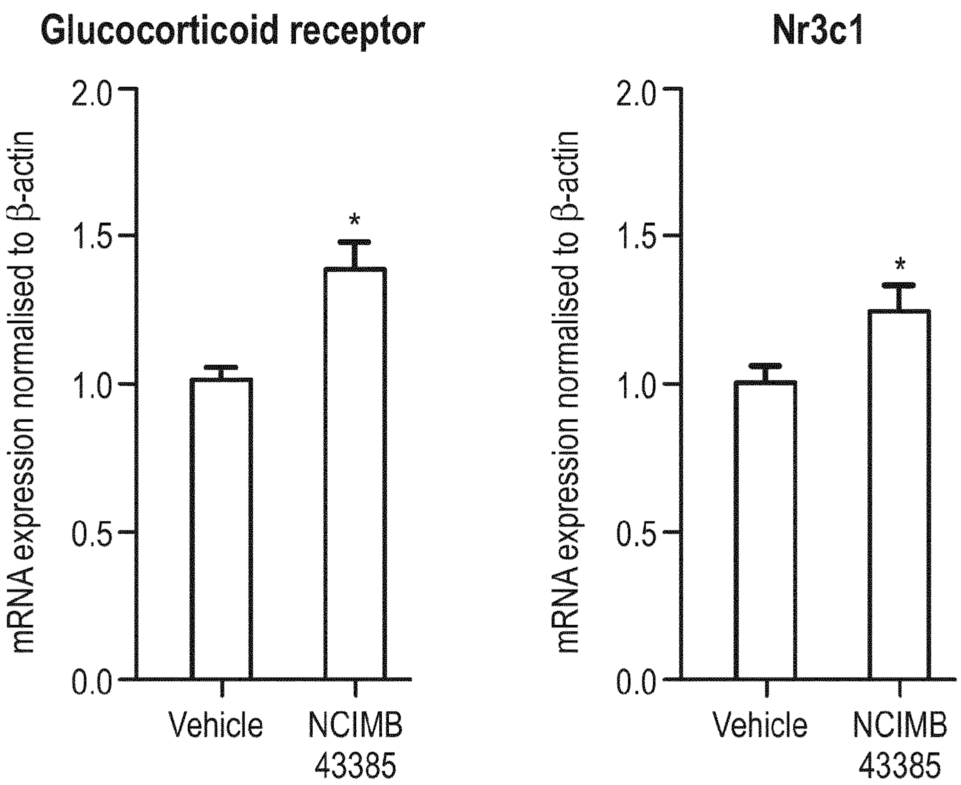

European Patent Office

# EUROPEAN SEARCH REPORT

Application Number
EP 22 16 2425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2004/120963 A1 (USHIDA KAZUNARI [JP] ET AL) 24 June 2004 (2004-06-24)<br>* claims 1-9; examples 3,4 *<br>* paragraphs [0013], [0014], [0016], [0019], [0022], [0023] * | 1-5, 11-14<br>1-14 | INV.<br>A61K35/74<br>A23L33/135<br>A61P1/00<br>A61P11/06<br>A61P3/10<br>A61P17/06<br>A61P19/02<br>A61P37/00<br>A61P35/00<br>A61P43/00 |
| X<br>Y | CN 104 415 060 A (BGI SHENZHEN CO LTD; SHENZHEN BGI AGRICULTURE AND CYCLE ECONOMIC TECHN) 18 March 2015 (2015-03-18)<br>* abstract *<br>* claims 1-10; examples 3,5 * | 1-5, 11-14<br>1-14 | |
| A | JP S55 57520 A (NISSHIN FLOUR MILLING CO) 28 April 1980 (1980-04-28)<br>* abstract * | 11-15 | |
| X<br>Y | WO 2004/085628 A1 (ROWETT RES INST [GB]; DUNCAN SYLVIA HELEN [GB]; FLINT HARRY JAMES [GB]) 7 October 2004 (2004-10-07)<br>* paragraph [0004]; claims 1-31 * | 1-5, 11-14<br>1-14 | TECHNICAL FIELDS SEARCHED (IPC) |
| A | WO 2017/091783 A2 (SERES THERAPEUTICS INC [US]) 1 June 2017 (2017-06-01)<br>* page 24, line 15 - page 25, line 10; claims 1-9 *<br>* example 7 * | 1-15 | A61K<br>A61P<br>A23L |
| A | NAYUDU NALLABELLI ET AL: "Biochemical and genome sequence analyses of Megasphaera sp. strain DISK18 from dental plaque of a healthy individual reveals commensal lifestyle",<br>SCIENTIFIC REPORTS,<br>vol. 6, no. 1,<br>21 September 2016 (2016-09-21),<br>XP055504215,<br>DOI: 10.1038/srep33665<br>* abstract *<br>-/-- | 1-15 | |

The present search report has been drawn up for all claims

1

| Place of search | Date of completion of the search | Examiner |
|---|---|---|
| Munich | 20 June 2022 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 16 2425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & DATABASE EMBL [Online]<br><br>7 January 2016 (2016-01-07), "Megasphaera sp. MTCC 12521 partial 16S rRNA gene, strain MTCC 12521, isolate DISK 18", retrieved from EBI accession no. EM_STD:LN998020<br>Database accession no. LN998020<br>* sequence *<br>----- | | |
| A | ROSHAN PADMANABHAN ET AL: "Non-contiguous finished genome sequence and description of Megasphaera massiliensis sp. nov.", STANDARDS IN GENOMIC SCIENCES, vol. 8, no. 3, 7 August 2013 (2013-08-07), pages 525-538, XP055504182, DOI: 10.4056/sigs.4077819<br>* abstract *<br>& DATABASE EMBL [Online]<br><br>2 October 2012 (2012-10-02), "Megasphaera massiliensis strain NP3 16S ribosomal RNA gene, partial sequence.", retrieved from EBI accession no. EM_STD:JX424772<br>Database accession no. JX424772<br>* sequence *<br>----- | 1-15 | TECHNICAL FIELDS SEARCHED (IPC) |
| A | US 2016/199424 A1 (BERRY DAVID [US] ET AL) 14 July 2016 (2016-07-14)<br>* paragraphs [0153], [0267], [0269], [0480], [0563], [845643800], [0831]; claims 1-19; sequence 1200 *<br>----- | 1-15 | |
| X | WO 2018/064165 A2 (UNIV TEXAS [US]) 5 April 2018 (2018-04-05) | 1-5, 11-14 | |
| Y | * claims 1-48 *<br>-----<br>-/-- | 1-14 | |

The present search report has been drawn up for all claims

1

| Place of search | Date of completion of the search | Examiner |
|---|---|---|
| Munich | 20 June 2022 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 16 2425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2017/172894 A2 (UNIV COLUMBIA [US]) 5 October 2017 (2017-10-05)<br>* page 35, line 18 - page 36, line 5; claims 1-16 * | 1-5, 11-14<br>1-14 | |
| Y | US 2018/008565 A1 (FEITELSON MARK A [US] ET AL) 11 January 2018 (2018-01-11)<br>* paragraphs [0006] - [0020]; claims 1-15 * | 1-14 | |
| Y | SIAVOSHIAN S ET AL: "Comparison of the effect of different short chain fatty acids on the growth and differentiation of human colonic carcinoma cell lines in vitro",<br>CELL BIOLOGY INTERNATIONAL, ACADEMIC PRESS, GB,<br>vol. 21, no. 5,<br>1 January 1997 (1997-01-01), pages 281-287, XP002376237,<br>ISSN: 1065-6995, DOI: 10.1006/CBIR.1997.0153<br>* abstract *<br>* Results * | 1-14 | TECHNICAL FIELDS SEARCHED (IPC) |
| Y | FERNÁNDEZ JAVIER ET AL: "Colon microbiota fermentation of dietary prebiotics towards short-chain fatty acids and their roles as anti-inflammatory and antitumour agents: A review",<br>JOURNAL OF FUNCTIONAL FOODS, ELSEVIER BV, NL,<br>vol. 25, 12 July 2016 (2016-07-12), pages 511-522, XP029665322,<br>ISSN: 1756-4646, DOI: 10.1016/J.JFF.2016.06.032<br>* abstract *<br>* 6. Conclusions *<br>-/-- | 1-14 | |

The present search report has been drawn up for all claims

1

| Place of search | Date of completion of the search | Examiner |
|---|---|---|
| Munich | 20 June 2022 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 16 2425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | YUILLE SAMANTHA ET AL: "Human gut bacteria as potent class I histone deacetylase inhibitors in vitro through production of butyric acid and valeric acid", PLOS ONE, vol. 13, no. 7, 27 July 2018 (2018-07-27), XP009508155, * the whole document * ----- | | |

TECHNICAL FIELDS SEARCHED (IPC)

The present search report has been drawn up for all claims

1

| Place of search | Date of completion of the search | Examiner |
|---|---|---|
| Munich | 20 June 2022 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 16 2425

This annex lists the patent family members relating to the patent documents cited in the above-mentioned European search report. The members are as contained in the European Patent Office EDP file on
The European Patent Office is in no way liable for these particulars which are merely given for the purpose of information.

20-06-2022

| Patent document cited in search report | Publication date | Patent family member(s) | Publication date |
|---|---|---|---|
| US 2004120963 A1 | 24-06-2004 | EP 1389464 A1 | 18-02-2004 |
| | | JP WO2002080947 A1 | 29-07-2004 |
| | | US 2004120963 A1 | 24-06-2004 |
| | | WO 02080947 A1 | 17-10-2002 |
| CN 104415060 A | 18-03-2015 | CN 104415060 A | 18-03-2015 |
| | | HK 1203398 A1 | 30-10-2015 |
| JP S5557520 A | 28-04-1980 | JP S5557520 A | 28-04-1980 |
| | | JP S6241215 B2 | 02-09-1987 |
| WO 2004085628 A1 | 07-10-2004 | AU 2004223657 A1 | 07-10-2004 |
| | | CA 2519204 A1 | 07-10-2004 |
| | | EP 1606394 A1 | 21-12-2005 |
| | | US 2007258953 A1 | 08-11-2007 |
| | | WO 2004085628 A1 | 07-10-2004 |
| WO 2017091783 A2 | 01-06-2017 | AU 2016359178 A1 | 14-06-2018 |
| | | BR 112018010430 A2 | 27-11-2018 |
| | | CA 3006187 A1 | 01-06-2017 |
| | | CN 109661236 A | 19-04-2019 |
| | | EP 3380108 A2 | 03-10-2018 |
| | | JP 2019501136 A | 17-01-2019 |
| | | JP 2022033885 A | 02-03-2022 |
| | | KR 20180083894 A | 23-07-2018 |
| | | RU 2018122664 A | 25-12-2019 |
| | | US 2019247447 A1 | 15-08-2019 |
| | | WO 2017091783 A2 | 01-06-2017 |
| US 2016199424 A1 | 14-07-2016 | AU 2015353425 A1 | 13-07-2017 |
| | | AU 2021204574 A1 | 29-07-2021 |
| | | CA 2973223 A1 | 02-06-2016 |
| | | CN 108135944 A | 08-06-2018 |
| | | EP 3223834 A2 | 04-10-2017 |
| | | EP 3747450 A1 | 09-12-2020 |
| | | HK 1244683 A1 | 17-08-2018 |
| | | MA 41020 A | 03-10-2017 |
| | | MA 52257 A | 21-04-2021 |
| | | US 2016143961 A1 | 26-05-2016 |
| | | US 2016143962 A1 | 26-05-2016 |
| | | US 2016193258 A1 | 07-07-2016 |
| | | US 2016199424 A1 | 14-07-2016 |
| | | US 2016235792 A1 | 18-08-2016 |
| | | US 2016271188 A1 | 22-09-2016 |
| | | US 2018015130 A1 | 18-01-2018 |
| | | US 2018071344 A1 | 15-03-2018 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 22 16 2425

This annex lists the patent family members relating to the patent documents cited in the above-mentioned European search report. The members are as contained in the European Patent Office EDP file on
The European Patent Office is in no way liable for these particulars which are merely given for the purpose of information.

20-06-2022

| Patent document cited in search report | Publication date | Patent family member(s) | Publication date |
|---|---|---|---|
| | | US 2018169153 A1 | 21-06-2018 |
| | | US 2019336543 A1 | 07-11-2019 |
| | | US 2020061129 A1 | 27-02-2020 |
| | | WO 2016086205 A2 | 02-06-2016 |
| | | WO 2016086206 A1 | 02-06-2016 |
| | | WO 2016086208 A1 | 02-06-2016 |
| | | WO 2016086209 A1 | 02-06-2016 |
| | | WO 2016086210 A1 | 02-06-2016 |
| WO 2018064165 A2 | 05-04-2018 | AU 2017335732 A1 | 04-04-2019 |
| | | BR 112019006041 A2 | 03-09-2019 |
| | | CA 3038076 A1 | 05-04-2018 |
| | | CN 110267651 A | 20-09-2019 |
| | | EP 3518946 A2 | 07-08-2019 |
| | | JP 2020500151 A | 09-01-2020 |
| | | KR 20190061042 A | 04-06-2019 |
| | | RU 2019108925 A | 29-10-2020 |
| | | US 2020129569 A1 | 30-04-2020 |
| | | WO 2018064165 A2 | 05-04-2018 |
| WO 2017172894 A2 | 05-10-2017 | US 2019125808 A1 | 02-05-2019 |
| | | WO 2017172894 A2 | 05-10-2017 |
| US 2018008565 A1 | 11-01-2018 | AU 2016209244 A1 | 17-08-2017 |
| | | AU 2019201799 A1 | 04-04-2019 |
| | | AU 2020239699 A1 | 15-10-2020 |
| | | CA 2974510 A1 | 28-07-2016 |
| | | CN 107405321 A | 28-11-2017 |
| | | EP 3247342 A1 | 29-11-2017 |
| | | JP 6783247 B2 | 11-11-2020 |
| | | JP 2018504457 A | 15-02-2018 |
| | | JP 2021020929 A | 18-02-2021 |
| | | KR 20170128247 A | 22-11-2017 |
| | | RU 2017127597 A | 25-02-2019 |
| | | SG 10201907660Y A | 30-10-2019 |
| | | SG 11201705953X A | 30-08-2017 |
| | | US 2018008565 A1 | 11-01-2018 |
| | | US 2018185307 A1 | 05-07-2018 |
| | | US 2019160031 A1 | 30-05-2019 |
| | | US 2022008369 A1 | 13-01-2022 |
| | | WO 2016118730 A1 | 28-07-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013050792 A
- WO 03046580 A
- WO 2013008039 A
- WO 2014167338 A


### Non-patent literature cited in the description


- **AHMED et al.** *Frontiers Cellular Neuroscience*
- **VOJINOV et al.** *Mol Med,* 2011, vol. 17 (5-6), 397-403
- **JOOSTEN et al.** *Mol Med,* 2011, vol. 17 (5-6), 391-396
- **REN et al.** *Inflamm Res,* 2016, vol. 65, 995-1008
- **TOVAR-CASTILLO et al.** *Int J Dermatol,* 2007, vol. 46, 239-46
- **BOVENSCHEN et al.** *J Invest Dermatol,* 2011, vol. 131, 1853-60
- **REILLY et al.** *Mol Med,* 2011, vol. 17 (5-6), 417-425
- **WANG et al.** *Immunol Cell Biol,* 1-8
- **PARK et al.** *J Clin Invest,* 2008, vol. 118, 2316-24
- **HAUMAITRE et al.** *Mol Cell Biol,* 2008, vol. 28, 6373-83
- **MOLSEY et al.** *J Biol Chem,* 2003, vol. 278, 19660-6
- **CHRISTENSEN et al.** *Mol Med,* 2011, vol. 17 (5-6), 370-390
- **JOHNSEN.** *J Chromatogr A,* 2017, vol. 1503, 57-64
- **SPOR et al.** *Nat Rev Microbiol.,* 2011, vol. 9 (4), 279-90
- **ECKBURG et al.** *Science,* 2005, vol. 308 (5728), 1635-8
- **MACPHERSON et al.** *Microbes Infect.,* 2001, vol. 3 (12), 1021-35
- **MACPHERSON et al.** *Cell Mol Life Sci.,* 2002, vol. 59 (12), 2088-96
- **MAZMANIAN et al.** *Cell,* 2005, vol. 122 (1), 107-18
- **FRANK et al.** *PNAS,* 2007, vol. 104 (34), 13780-5
- **SCANLAN et al.** *J Clin Microbiol.,* 2006, vol. 44 (11), 3980-8
- **KANG et al.** *Inflamm Bowel Dis.,* 2010, vol. 16 (12), 2034-42
- **MACHIELS et al.** *Gut,* 2013, vol. 63 (8), 1275-83
- **GOLDIN ; GORBACH.** *Clin Infect Dis.,* 2008, vol. 46 (2), S96-100
- **AZAD et al.** *BMJ,* 2013, vol. 347, f6471
- **TERRY ; MARGOLIS.** Handb Exp Pharmacol. 2017, vol. 239, 319-342
- **PADMANABHAN et al.** *Standards in Genomic Sciences,* 2013, vol. 8, 525-538
- **MASCO et al.** *Systematic and Applied Microbiology,* 2003, vol. 26, 557-563
- **SRUTKOVÁ et al.** *J. Microbiol. Methods,* 2011, vol. 87 (1), 10-6
- **LEONI et al.** *Mol. Med.,* 2005, vol. 11 (1-12), 1-15
- **GLAUBEN et al.** *Journal of Immunology,* 2006, vol. 176 (8), 5015-5022
- **WALMSLEY et al.** *Gut,* 1998, vol. 43, 29-32
- **GASCHE et al.** *Inflammatory Bowel Diseases,* vol. 6, 8-15
- **FAHY.** *Proc Am Thorac Soc,* 2009, vol. 6, 256-259
- **REDDY et al.** *J Clin. Invest.,* 2008, vol. 118, 2562-73
- **LENG et al.** *Exp. Hematol.,* 2006, vol. 34, 776-787
- **TAO et al.** *Nat. Med.,* 2007, vol. 13, 1299-1307
- **SONG et al.** *APMIS,* 2005, vol. 113, 264-268
- **ZIMMERMAN et al.** *Cancer Res,* 2007, vol. 67, 9074-54
- **ZHANG et al.** *Breast Cancer Res Treat,* 2005, vol. 94, 11-16
- **ABBAS ; GUPTA.** *Epigenetics,* 2008, vol. 3, 300-309
- **MINAMIYA et al.** *Lung Cancer,* 2011, vol. 74, 300-4
- **JUNG et al.** *J Cell Biochem,* 2012, vol. 113, 2167-2177
- **QUINT et al.** *Virchows Arch,* 2011, vol. 459, 129-139
- **MIYAMOTO-SHINOHARA et al.** *J. Gen. Appl. Microbiol.,* 2008, vol. 54, 9-24
- Cryopreservation and Freeze-Drying Protocols. Humana Press

- **LESLIE et al.** *Appl. Environ. Microbiol.,* 1995, vol. 61, 3592-3597
- **MITROPOULOU et al.** *J Nutr Metab.,* 2013, 716861
- **KAILASAPATHY et al.** *Curr Issues Intest Microbiol.,* 2002, vol. 3 (2), 39-48
- Handbook of Pharmaceutical Excipients. 1994
- Remington's Pharmaceutical Sciences. Mack Publishing Co, 1985
- **RONALD ATLAS.** Handbook of Microbiological Media. CRC Press, 2010
- **JENNIE C. HUNTER-CEVERA.** Maintaining Cultures for Biotechnology and Industry. Academic Press, 1996
- **STROBEL.** *Methods Mol Biol.,* 2009, vol. 581, 247-61
- **GENNARO.** Remington: The Science and Practice of Pharmacy. 2000
- Molecular Biology Techniques: An Intensive Laboratory Course. Academic Press, 1998
- Methods In Enzymology. Academic Press, Inc,
- Handbook of Experimental Immunology. Blackwell Scientific Publications, 1986, vol. I-IV
- **SAMBROOK et al.** Molecular Cloning: A Laboratory Manual. Cold Spring Harbor Laboratory Press, 2001
- Handbook of Surface and Colloidal Chemistry. CRC Press, 1997
- Current Protocols. Short protocols in molecular biology. 2002
- PCR (Introduction to Biotechniques Series. Springer Verlag, 1997
- Current Protocols in Molecular Biology. 1987
- **SMITH ; WATERMAN.** *Adv. Appl. Math.,* 1981, vol. 2, 482-489
- **WEST ; JOHNSTONE.** *J Clin Invest,* 2014, vol. 124, 30-39
- **GLAUBEN et al.** *J Immunol.,* 2006, vol. 176, 5015-5022
- **ANGIOLILLI et al.** *Ann Rheum Dis.,* 2017, vol. 76, 277-285
- **GONNEAUD et al.** *J Inflamm.,* 2014, vol. 11, 43
- **ALENGHAT et al.** *Nature,* 2013, vol. 504, 153-157
- **FELICE et al.** *Ailment Pharmacol Ther.,* 2015, vol. 41, 26-38
- **THANGARAJU et al.** *Cancer Res.,* 2009, vol. 67 (9), 2826-2832
- **SINGH et al.** *Immunity.,* 2014, vol. 40 (1), 128-39
- **LIVAK ; SCHMITTGEN.** *Methods,* 2001, vol. 25 (4), 402-8
- **JOHNSEN et al.** *Journal of Chromatography A,* 2017, vol. 1503, 57-64
- **VIZIN ; KOS.** *Radiol Oncol.,* 2015, vol. 49 (3), 217-226
- **SELVAN et al.** *AACR Annual Meeting,* 2008
- **BONNER et al.** *Clinical Cancer Research,* 2000, vol. 6, 597-601
- **ZHI et al.** *Oncotarget,* 2016, vol. 7 (40), 64798-64809
- **BAE et al.** *J Immunol.,* 01 July 2012, vol. 189 (1), 365-72
- **SASAKI-LMAMURA et al.** *Appl Environ Microbiol,* 2010, vol. 76 (13), 4260-4268
- **CHAI et al.** *Evid Based Complement Alternat Med,* 2012, 603678
- **DE BAERE et al.** *J Pharm Biomed Anal,* 2013, vol. 80, 107-115
- **SMART et al.** *Nat Protoc,* 2010, vol. 5 (10), 1709-1729
- **JOHNSEN et al.** *J Chromatogr A,* 2017, vol. 1503, 57-64